# EUROPEAN PATENT APPLICATION

(11) **EP 2 597 111 A1**
(43) Date of publication of application: **29.05.2013**
(21) Application number: 11809760.9
(22) Date of filing: 22.07.2011
(51) Int. Cl.: C08G 73/10

(54) **POLYIMIDE PRECURSOR, POLYIMIDE, AND MATERIALS TO BE USED IN PRODUCING SAME**

(30) Priority: 21.07.2011 JP 2011160371; 21.07.2011 JP 2011159931; 21.07.2011 JP 2011159919; 21.07.2011 JP 2011159910; 21.07.2011 JP 2011159902; 21.07.2011 JP 2011159898; 21.07.2011 JP 2011159850; 21.07.2011 JP 2011159837; 15.12.2010 JP 2010279547; 09.12.2010 JP 2010274277; 02.12.2010 JP 2010269053; 22.07.2010 JP 2010165368; 22.07.2010 JP 2010165367
(71) Applicant: Ube Industries, Ltd., Ube-shi Yamaguchi 755-8633 (JP)
(72) Inventor: TAKASAWA, Ryoichi, Ube-shi Yamaguchi 755-8633 (JP); OKA, Takuya, Ube-shi Yamaguchi 755-8633 (JP); KOHAMA, Yukinori, Ube-shi Yamaguchi 755-8633 (JP); NAKAGAWA, Miharu, Ube-shi Yamaguchi 755-8633 (JP); HISANO, Nobuharu, Ube-shi Yamaguchi 755-8633 (JP); KOHDA, Masafumi, Ichihara-shi Chiba 290-0045 (JP); NAKAYAMA, Takeshige, Ube-shi Yamaguchi 755-8633 (JP); NAKAYAMA, Tomonori, Ube-shi Yamaguchi 755-8633 (JP)
(74) Representative: Mai, Dörr, Besier
(86) International application number: PCT/JP2011/066765
(87) International publication number: WO 2012/011590

(57) **Abstract**

Disclosed is a novel co-polyimide precursor for producing a polyimide having high transparency. The co-polyimide precursor comprises a unit structure represented by general Formula (A1) and a unit structure represented by general Formula (A2): 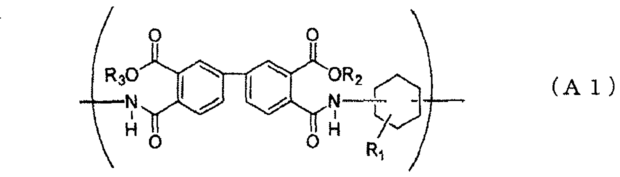 wherein, in general Formula (A1), R₁ represents a hydrogen atom or an alkyl group having 1 to 4 carbon atoms; and R₂ and R₃ each independently represent a hydrogen atom, an alkyl group having 1 to 6 carbon atoms, or an alkylsilyl group having 3 to 9 carbon atoms, 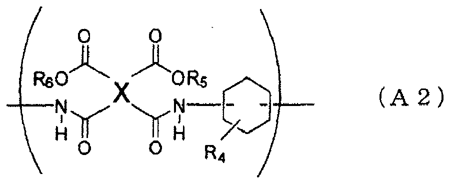 wherein, in general Formula (A2), R₄ represents a hydrogen atom or an alkyl group having 1 to 4 carbon atoms; R₅ and R₆ each independently represent a hydrogen, an alkyl group having 1 to 6 carbon atoms, or an alkylsilyl group having 3 to 9 carbon atoms; and X represents a tetravalent group other than those represented by Formulae (A3):

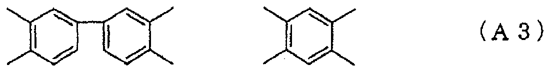

## Description

### TECHNICAL FIELD

The present invention relates to a polyimide having high transparency, high mechanical strength, and low coefficient of linear thermal expansion and relates to a polyimide precursor suitable for producing the polyimide.

### BACKGROUND ART

Recently, optical materials, such as optical fibers and optical waveguides in the optical communication field and liquid crystal alignment films and color filter protective films in the display device field, have been developed with the coming of an advanced information society. In particular, in the display device field, plastic substrates being lightweight and having excellent flexibility have been investigated as a replacement for glass substrates, and displays that can be bent or rolled up have been being actively developed. Thus, there is a demand for higher performance optical materials that can be used for such purposes.

In general, polyimides are essentially colored in yellowish brown by intramolecular conjugation or charge-transfer complex formation. As a countermeasure thereof, for example, a method of expressing transparency by inhibiting formation of charge-transfer complex by introducing fluorine, providing flexibility to the main chain, or introducing a bulky side chain is proposed (Non-Patent Document 1). In addition, methods of expressing transparency by using semi-alicyclic or wholly alicyclic polyimide resins that do not, in principle, form charge-transfer complexes are proposed (Japanese Patent Laid-Open No. 2002-348374 (Patent Document 1), Japanese Patent Laid-Open No. 2005-15629 (Patent Document 2), Japanese Patent Laid-Open No. 2002-161136 (Patent Document 3), and Non-Patent Document 2).

In particular, semi-alicyclic polyimides prepared using trans-1,4-diaminocyclohexanes as the diamine components and 3,3',4,4'-biphenyltetracarboxylic dianhydrides as the tetracarboxylic acid components are known to have excellent transparency, high heat resistance, and low coefficient of linear thermal expansion (Patent Document 3). Thus, the use of an alicyclic diamine as a monomer component is effective for preparing a transparent polyimide.
Unfortunately, the elongation at break of the film formed from the semi-alicyclic polyimide is 5 to 7% and is insufficient as base materials for, for example, flexible displays (Non-Patent Document 2). In addition, aliphatic diamines tend to form solvent-insoluble salts by a reaction with the carboxyl groups of low molecular weight amic acids generated in the initial stage of polymerization and thereby often cause a severe problem of preventing the polymerization from progressing. In order to avoid this problem, a method of solubilizing the salt formed in the initial stage of polymerization by heating the polymerization mixture at high temperature, for example, at 120°C, for a short period of time is known (Patent Document 3). In this method, however, the molecular weight of the polyimide precursor varies depending on the temperature history in the polymerization, and also the imidization is accelerated by the heat. Consequently, the polyimide precursor cannot be stably produced. Furthermore, since the resulting polyimide precursor solution needs to dissolve the salt at high temperature in the preparation step, increasing its concentration is impossible, and in addition, its handling property is poor, for example, it is difficult to control of the thickness of a polyimide film, and its storage stability is insufficient.

As described above, it is highly demanded that the polyimide precursor prepared using an alicyclic diamine can be stably produced under moderate conditions and also that the polyimide prepared from the polyimide precursor has excellent transparency, high heat resistance, and low coefficient of linear thermal expansion and also has bending resistance (toughness, i.e., sufficiently high elongation at break) required as a base material for, for example, a flexible display or touch panel.
Meanwhile, regarding transparency, in also the case of a semi-alicyclic polyimide prepared using trans-1,4-diaminocyclohexane, the optical transmission spectrum has absorption at about 400 nm. This demonstrates that the polyimide is colored not only due to the molecular structure, such as absorption by formation of a charge-transfer complex, but also due to the raw material of a polyimide precursor varnish.

2,3,3',4'-biphenyltetracarboxylic dianhydride is one of tetracarboxylic acid components as raw materials for polyimides. The purification of 2,3,3',4'-biphenyltetracarboxylic dianhydride has not been sufficiently investigated compared to other acid dianhydrides that are widely used as raw materials for polyimides, such as pyromellitic dianhydride and 3,3',4,4'-biphenyltetracarboxylic dianhydride.
Japanese Patent Laid-Open No. 2006-328040 (Patent Document 4) discloses a method of producing a powder of 2,3,3,4-biphenyltetracarboxylic dianhydride by heating to dehydrate 2,3,3',4'-biphenyltetracarboxylic acid under an inert gas atmosphere at 180 to 195°C for a sufficient time for completing dehydration.
Japanese Patent Laid-Open No. 2009-019014 (Patent Document 5) discloses a method of producing 2,3,3,4-biphenyltetracarboxylic dianhydride by stirring molten 2,3,3',4'-biphenyltetracarboxylic acid at a temperature of 200°C or more under an inert gas flow for thermal dehydration. The resulting 2,3,3,4-biphenyltetracarboxylic dianhydride is solidified by cooling and is pulverized with, for example, a pulverizer to give a powder of 2,3,3',4'-biphenyltetracarboxylic dianhydride.
Japanese Patent Laid-Open No. 2004-196687 (Patent Document 6) describes a method of purifying a biphenyltetracarboxylic anhydride prepared comprising hydrolyzing tetramethyl biphenyltetracarboxylate, dehydrating, adding an adsorbent in a solvent, filtering, and recrystallizing; and also describes that acetic anhydride is suitable as the solvent for the recrystallization. However, the patent document relates to a method of purifying 3,3',4,4'-biphenyltetracarboxylic dianhydride and does not describe 2,3,3',4'-biphenyltetracarboxylic dianhydride at all.

Regarding 3,3',4,4'-biphenyltetracarboxylic dianhydride, which is one of tetracarboxylic acid components as raw materials for polyimides, Japanese Patent Laid-Open No. 2005-314296 (Patent Document 7) describes the preparation of a thermal dehydration product of 3,3',4,4'-biphenyltetracarboxylic acid having reduced color by melting a thermal dehydration product of 3,3',4,4'-biphenyltetracarboxylic acid by heating, evaporating the molten material at a temperature of 307°C or more and 330°C or less, under reduced pressure while maintaining the oxygen concentration in the system to 10 ppm or less, and crystallizing the vapor through cooling.

Japanese Patent Laid-Open No. 2006-45198 (Patent Document 8) describes preparation of a thermal dehydration product of 3,3',4,4'-biphenyltetracarboxylic acid having reduced color by subjecting 3,3',4,4'-biphenyltetracarboxylic acid to cyclodehydration using a specific heater under a specific pressure condition by increasing the temperature at a specific temperature-increasing rate to a temperature range of 210 to 250°C at the highest and maintaining the temperature at 150 to 250°C for a specific period of time to prepare 3,3',4,4'-biphenyltetracarboxylic dianhydride and further subjecting the resulting 3,3',4,4'-biphenyltetracarboxylic dianhydride to sublimation purification under reduced pressure at a temperature of 250°C or more.

Japanese Patent Laid-Open No. 2004-196687 (Patent Document 6) describes a method of purifying a biphenyltetracarboxylic anhydride prepared comprising hydrolyzing 3,3',4,4'-tetramethyl biphenyltetracarboxylate, dehydrating, adding an adsorbent in a solvent, filtering, and recrystallizing; and also describes that acetic anhydride is suitable as the solvent for the recrystallization.
Japanese Patent Laid-Open No. 2004-196687 (Patent Document 6) describes a method of purifying a biphenyltetracarboxylic anhydride prepared by hydrolysis and dehydration of 3,3',4,4'-tetramethyl biphenyltetracarboxylate by filtration thereof in a solvent containing an adsorbent and recrystallization, and also describes that acetic anhydride is suitable as the solvent for the recrystallization.

As described in U.S. Patent Nos. 2606925 and 3636108 and Japanese Patent Laid-Open No. 2008-74754 (Patent Documents 9 to 11), methods of producing trans-1,4-diaminocyclohexane as a raw material of the diamine component of semi-alicyclic polyimide have been variously investigated for simplification of the process and an increase in yield. However, a trans-1,4-diaminocyclohexane powder reduced in coloring has not been investigated.

Non-Patent Document 2 either does not investigate any trans-1,4-diaminocyclohexane powder reduced in coloring and any polyimide reduced in coloring by using the trans-1,4-diaminocyclohexane powder as the diamine component.

Regarding 2,2',3,3'-biphenyltetracarboxylic dianhydride, which is one of tetracarboxylic acid components as raw materials of polyimides, Japanese Patent Laid-Open No. 2000-281616 (Patent Document 12) discloses a method of producing 2,2',3,3'-biphenyltetracarboxylic acid with a high yield by a simplified process and a polyimide resin prepared using the 2,2',3,3'-biphenyltetracarboxylic acid. Japanese Patent Laid-Open No. 2009-79009 (Patent Document 13) discloses a method of preparing 2,2',3,3'-biphenyltetracarboxylic dianhydride through dehydration of 2,2',3,3'-biphenyltetracarboxylic acid with acetic anhydride. These documents, however, merely describe 2,2',3,3'-biphenyltetracarboxylic acid and synthesis of 2,2',3,3'-biphenyltetracarboxylic dianhydride and do not describe or suggest any method of purifying 2,2',3,3'-biphenyltetracarboxylic dianhydride for reducing coloring.

### PRIOR ART REFERENCES

### PATENT DOCUMENTS

Patent Document 1: Japanese Patent Laid-Open No. 2002-34837
Patent Document 2: Japanese Patent Laid-Open No. 2005-15629
Patent Document 3: Japanese Patent Laid-Open No. 2002-161136
Patent Document 4: Japanese Patent Laid-Open No. 2006-328040
Patent Document 5: Japanese Patent Laid-Open No. 2009-019014
Patent Document 6: Japanese Patent Laid-Open No. 2004-196687
Patent Document 7: Japanese Patent Laid-Open No. 2005-314296
Patent Document 8: Japanese Patent Laid-Open No. 2006-45198
Patent Document 9: U.S. Patent No. 2606925
Patent Document 10: U.S. Patent No. 3636108
Patent Document 11: Japanese Patent Laid-Open No. 2008-74754
Patent Document 12: Japanese Patent Laid-Open No. 2000-281616
Patent Document 13: Japanese Patent Laid-Open No. 2009-79009

### NON-PATENT DOCUMENT:

Non-Patent Document 1: Polymer,47,2337(2006)
Non-Patent Document 2: M. Hasegawa, High Perform.Polym. 13, (2001) S93-S106

### SUMMARY OF THE INVENTION

### PROBLEM TO BE SOLVED BY THE INVENTION

The inventors of the present invention have conducted the investigation from the view point of a chemical structure and the investigation from the view point of purity of raw materials to obtain a polyimide having high transparency, and completed the present invention.
An object of an aspect of the present invention is to provide a co-polyimide precursor that can be produced stably under moderate conditions, and a co-polyimide having excellent transparency, high heat resistance, high glass transition temperature, and low coefficient of linear thermal expansion and also having bending resistance (toughness, i.e., sufficiently high elongation at break) at the same time.
An object of another aspect of the present invention is to provide a raw material for producing a polyimide having high transparency.
An object of each aspect of the present invention will be obvious from the following description.

### MEANS FOR SOLVING THE PROBLEM

Each aspect of the present invention is as follows.

### <The first aspect (PART A)>

A co-polyimide precursor comprising a unit structure represented by general Formula (A1) and a unit structure represented by general Formula (A2):

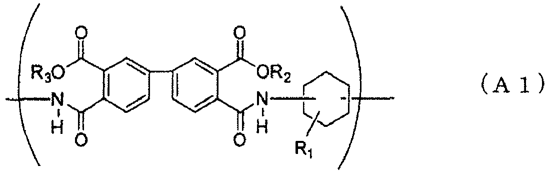

wherein, in general Formula (A1), R₁ represents a hydrogen atom or an alkyl group having 1 to 4 carbon atoms; and R₂ and R₃ each independently represent a hydrogen atom, an alkyl group having 1 to 6 carbon atoms, or an alkylsilyl group having 3 to 9 carbon atoms,

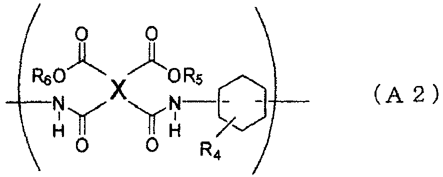

wherein, in general Formula (A2), R₄ represents a hydrogen atom or an alkyl group having 1 to 4 carbon atoms; R₅ and R₆ each independently represent a hydrogen, an alkyl group having 1 to 6 carbon atoms, or an alkylsilyl group having 3 to 9 carbon atoms; and X represents a tetravalent group other than those represented by Formulae (A3):

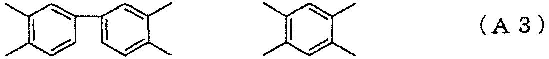

### <The second aspect (PART B)>

A polyimide precursor, comprising a unit structure represented by general Formula (B1):

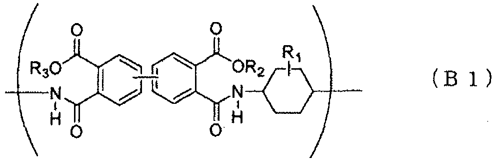

wherein, in general Formula (B1), R₁ represents a hydrogen atom or an alkyl group having 1 to 4 carbon atoms; R₂ and R₃ each represent a hydrogen atom or an alkylsilyl group having 3 to 9 carbon atoms, and at least one of R₂ and R₃ is an alkylsilyl group having 3 to 9 carbon atoms.

### <The third aspect (PART C)>

(Main aspect) A 2,3,3',4'-biphenyltetracarboxylic dianhydride powder, having a light transmittance of 85% or more at a wavelength of 400 nm and an optical path length of 1 cm as a 10% by mass solution in a 2 N aqueous sodium hydroxide solution.
(Another main aspect) A method of purifying a 2,3,3',4'-biphenyltetracarboxylic dianhydride powder, comprising mixing a solvent in which the solubility of 2,3,3',4'-biphenyltetracarboxylic dianhydride at 25°C is 1 g/100 g or more and a 2,3,3',4'-biphenyltetracarboxylic dianhydride powder in an uneven state where at least a part of the 2,3,3',4'-biphenyltetracarboxylic dianhydride powder is not dissolved; and separating and collecting the undissolved 2,3,3',4'-biphenyltetracarboxylic dianhydride powder from the mixture.

### <The fourth aspect (PART D)>

A method of purifying a 3,3',4,4'-biphenyltetracarboxylic dianhydride powder, comprising mixing a solvent in which the solubility of 3,3',4,4'-biphenyltetracarboxylic dianhydride at 25°C is 0.1 g/100 g or more and a 3,3',4,4'-biphenyltetracarboxylic dianhydride powder in an uneven state where at least a part of the 3,3',4,4'-biphenyltetracarboxylic dianhydride powder is not dissolved; and separating and collecting the undissolved 3,3',4,4'-biphenyltetracarboxylic dianhydride powder from the mixture.

### <The fifth aspect (PART E)>

A trans-1,4-diaminocyclohexane powder having a light transmittance of 90% or more at a wavelength of 400 nm and an optical path length of 1 cm as a 10% by mass solution in pure water.

### <The sixth aspect (PART F)>

A 2,2',3,3'-biphenyltetracarboxylic dianhydride powder having a light transmittance of 80% or more at a wavelength of 400 nm and an optical path length of 1 cm as a 10% by mass solution in a 2 N aqueous sodium hydroxide solution as a solvent.

### <The seventh aspect (PART G)>

(Main aspect) A polyimide prepared by a reaction between a diamine component and a tetracarboxylic acid component, wherein
the diamine component comprises an aromatic ring-free diamine (including a derivative thereof, the same applies to the following) having a light transmittance of 90% or more or an aromatic ring-containing diamine (including a derivative thereof, the same applies to the following) having a light transmittance of 80% or more (here, the transmittance of the diamine component is that measured at a wavelength of 400 nm and an optical path length of 1 cm as a 10% by mass solution in pure water or N, N-dimethylacetamide); and
the tetracarboxylic acid component comprises a tetracarboxylic acid (including a derivative thereof, the same applies to the following) having a light transmittance of 75% or more (here, the transmittance of the tetracarboxylic acid component is that measured at a wavelength of 400 nm and an optical path length of 1 cm as a 10% by mass solution in a 2 N aqueous sodium hydroxide solution).
(Another main aspect) A polyimide precursor, comprising an aromatic ring-free diamine in an amount of 50% by mol or more of the total moles of the diamine component used; the polyimide precursor having a light transmittance of 90% or more at a wavelength of 400 nm and an optical path length of 1 cm as a 10% by mass solution in a polar solvent.
(Another main aspect) A polyimide precursor, comprising an aromatic ring-containing diamine in an amount of 50% by mol or more of the total moles of the diamine component used; the polyimide precursor having a light transmittance of 50% or more at a wavelength of 400 nm and an optical path length of 1 cm as a 10% by mass solution in a polar solvent.

### <The eighth aspect (PART H)>

A method of producing a varnish, comprising at least an organic solvent and a polyimide precursor represented by general Formula (H1) or a polyimide represented by general Formula (H2):

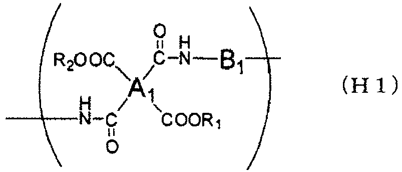

(in general Formula (H1), A₁ represents a tetravalent aliphatic or aromatic group; B₁ represents a divalent aliphatic or aromatic group; and R₁ and R₂ each independently represent a hydrogen atom, an alkyl group having 1 to 6 carbon atoms, or an alkylsilyl group having 3 to 9 carbon atoms),

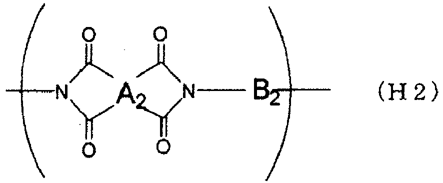

(in general Formula (H2), A₂ represents a tetravalent aliphatic or aromatic group; and B₂ represents a divalent aliphatic or aromatic group), wherein
the organic solvent to be contained in the varnish (hereinafter, referred to as the organic solvent used) has a light transmittance of 89% or more at 400 nm and an optical path length of 1 cm.

### EFFECT OF THE INVENTION

According to an aspect of the present invention, a co-polyimide precursor can be produced stably under moderate conditions, and a co-polyimide having excellent transparency, high heat resistance, high glass transition temperature, and low coefficient of linear thermal expansion and also having bending resistance (toughness, i.e., sufficiently high elongation at break) at the same time can be provided. In particular, the polyimide of the present invention can be suitably used for, for example, a transparent substrate of a display device such as a flexible display or touch panel or a solar cell substrate.
According to another aspect of the present invention, a raw material suitable for preparing a polyimide with high transparency can be provided.
The effects of each aspect of the present invention will be obvious from the following description.

### BRIEF DESCRIPTION OF THE DRAWINGS

Figure 1 shows the measurement result of dynamic viscoelasticity of the film prepared in Example A8.
Figure 2 shows the measurement result of dynamic viscoelasticity obtained in Example A9.
Figure 3 shows the measurement result of dynamic viscoelasticity of the film prepared in Example A14.
Figure 4 is a chart showing the GC analysis result of N-methyl-2-pyrrolidone (NMP) having a purity of 99.96%.
Figure 5 is a chart showing the GC analysis result of N,N-dimethylacetamide (DMAc) having a purity of 99.99%.
Figure 6 is a chart showing the GC analysis result of N-methyl-2-pyrrolidone (NMP) having a purity of 99.62%.
Figure 7 is a chart showing the GC analysis result of 1,3-dimethyl-2-imidazolidinone (DMI) having a purity of 99.30%.
Figure 8 is a graph showing a relationship between the purity (%) of solvents and the light transmittances (%) at 400 nm of polyimide films.
Figure 9 is a graph showing a relationship between the peak area (%) of impurities at long retention time and the light transmittances (%) at 400 nm of a polyimide films.
Figure 10 is a graph showing a relationship between the light transmittances (%) at 400 nm of solvents and the light transmittances (%) at 400 nm of polyimide films.
Figure 11 is a graph showing a relationship between the light transmittance (%) at 400 nm of a solvent after heating with refluxing and the light transmittance (%) at 400 nm of a polyimide film.

### EMBODIMENT FOR CARRYING OUT THE INVENTION

First to eighth Aspects of the present invention (hereinafter, simply referred to as the invention) will be described separately from Part A to Part H, respectively. The term "the present invention" in each Part generally indicates the invention described in the currently-referenced part, but may also indicate an invention described in another Part as long as there is no contradiction. However, if the invention described there contradicts the invention of another Part in view of the context or the gist of the invention described in the referenced Part, the term indicates only the invention described in the currently-referenced part. The inventions described in Parts A to H can be combined as long as there is a consistency.

### « PART A »

The object of the invention disclosed in Part A is to provide co-polyimide precursor that can be produced stably under moderate conditions, and a co-polyimide having excellent transparency, high heat resistance, high glass transition temperature, and low coefficient of linear thermal expansion and also having bending resistance (toughness, i.e., sufficiently high elongation at break) at the same time.

The invention disclosed in Part A relates to the following items.

1. A co-polyimide precursor, comprising a unit structure represented by general Formula (A1) and a unit structure represented by general Formula (A2):

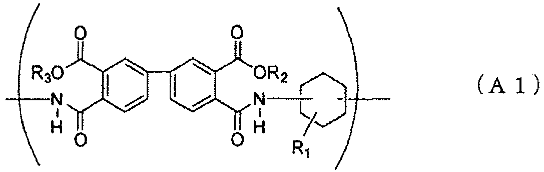

wherein, in general Formula (A1), R₁ represents a hydrogen atom or an alkyl group having 1 to 4 carbon atoms; and R₂ and R₃ each independently represent a hydrogen atom, an alkyl group having 1 to 6 carbon atoms, or an alkylsilyl group having 3 to 9 carbon atoms,

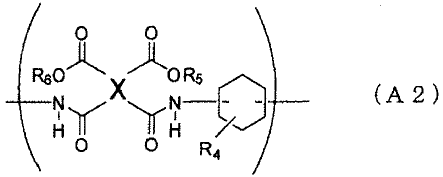

wherein, in general Formula (A2), R₄ represents a hydrogen atom or an alkyl group having 1 to 4 carbon atoms; R₅ and R₆ each independently represent a hydrogen, an alkyl group having 1 to 6 carbon atoms, or an alkylsilyl group having 3 to 9 carbon atoms; and X represents a tetravalent group other than those represented by Formulae (A3):

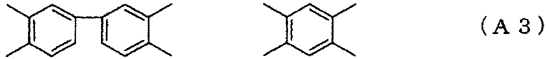

2. The co-polyimide precursor according to item 1, wherein the number ratio of the unit structures represented by general Formula (A1) to the unit structures represented by general Formula (A2) [the number of unit structures represented by general Formula (A1)/the number of unit structures represented by general Formula (A2)] is 50/50 to 99.5/0.5.

3. The co-polyimide precursor according to item 1 or 2, wherein X in general Formula (A2) is any one of tetravalent groups shown as Formulae (A4):

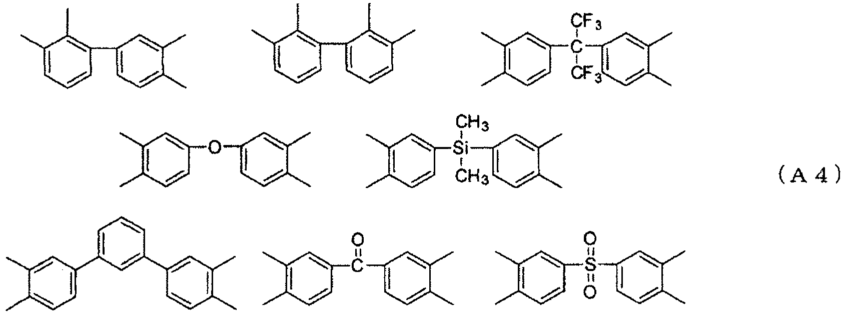

or a mixture thereof.

4. The co-polyimide precursor according to any one of items 1 to 3, having a logarithmic viscosity of 0.2 dL/g or more as a 0.5 g/dL solution in N,N-dimethylacetamide at 30°C.

5. A method of producing a co-polyimide precursor according to any one of items 1 to 4, comprising reacting a diamine component and a tetracarboxylic acid component in a solvent at temperature of 100°C or less.

6. The method of producing a co-polyimide precursor according to item 5, wherein the solvent used has a purity (a purity determined by GC analysis) of 99.8% or more.

7. A method of producing a solution composition of the co-polyimide precursor according to item 5 or 6, comprising reacting a tetracarboxylic acid component and a diamine component at a molar ratio such that the diamine component is excess to obtain a polyimide precursor; and further adding a carboxylic acid derivative in an amount approximately corresponding to the number of excess moles of the diamine to the resulting polyimide precursor such that the total molar proportion of the tetracarboxylic acid and the carboxylic acid derivative component is approximately equivalent to the molar proportion of the diamine component.

8. A co-polyimide having a unit structure represented by general Formula (A5) and a unit structure represented by general Formula (A6):

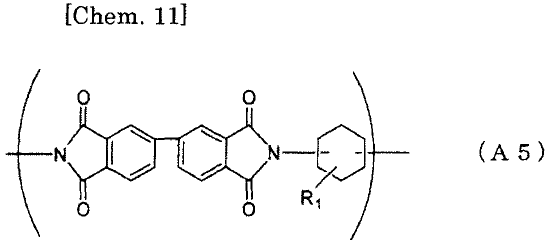

wherein, in general Formula (A5), R₁ represents a hydrogen atom or an alkyl group having 1 to 4 carbon atoms,

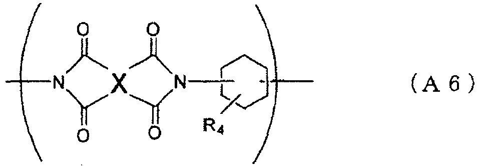

wherein, in general Formula (A6), R₄ represents a hydrogen atom or an alkyl group having 1 to 4 carbon atoms; and X represents a tetravalent group other than those represented by Formulae (A3).

9. The co-polyimide according to item 8, wherein the number ratio of the unit structures represented by general Formula (A5) to the unit structures represented by general Formula (A6) [the number of unit structures represented by general Formula (A5)/the number of unit structures represented by general Formula (A6)] is 50/50 to 99.5/0.5.

10. The co-polyimide according to item 8 or 9, wherein X in general Formula (A6) is any one of tetravalent groups shown as Formulae (A4) or a mixture thereof.

11. The co-polyimide according to any one of items 8 to 10, having toughness corresponding to an elongation at break at room temperature of 8% or more and transparency corresponding to a light transmittance at 400 nm of 50% or more when formed into a film having a thickness of 10 µm.

12. The co-polyimide according to any one of items 8 to 11, having an elastic modulus at room temperature of 3 GPa or more, toughness corresponding an elongation at break at room temperature of 10% or more, and transparency corresponding to a light transmittance at 400 nm of 75% or more when formed into a film having a thickness of 10 µm.

13. The co-polyimide according to any one of items 8 to 12, having an average coefficient of linear thermal expansion of 20 ppm/K or less at 50 to 200°C when formed into a film having a thickness of 10 µm.

14. The co-polyimide according to any one of items 8 to 13, wherein, in the dynamic viscoelastic measurement of a film having a thickness of 10 µm formed from the co-polyimide, as compared with a minimum storage elastic modulus observed at a temperature not lower than the glass transition temperature determined from the maximum point of tan δ, the co-polyimide has a maximum storage elastic modulus at a temperature not lower than the temperature at which the minimum storage elastic modulus is observed.

According to the invention disclosed in Part A, a co-polyimide precursor can be produced stably under moderate conditions, and a co-polyimide having excellent transparency, high heat resistance, high glass transition temperature, and low coefficient of linear thermal expansion and also having bending resistance (toughness, i.e., sufficiently high elongation at break) at the same time can be provided. In particular, the polyimide of the present invention can be suitably used for, for example, a transparent substrate of a display device such as a flexible display or touch panel or a solar cell substrate.

The invention disclosed in Part A will now be described in detail.
The co-polyimide precursor of the present invention disclosed in this Part is characterized in that it has a unit structure represented by general Formula (A1) and a unit structure represented by general Formula (A2).
Here, the number ratio of the unit structures represented by general Formula (A1) to the unit structures represented by general Formula (A2) [the number of unit structures represented by general Formula (A1)/the number of unit structures represented by general Formula (A2)] is not particularly limited, but the ratio of the unit structure represented by general Formula (A1) is preferably in the range of 40/60 or more, more preferably 50/50 or more, more preferably 80/20 or more, and most preferably 90/10 or more and preferably in the range of 99.5/0.5 or less and more preferably 98/2 or less. A too small proportion of the unit structure represented by general Formula (A1) may increase the coefficient of linear thermal expansion of the resulting co-polyimide. In contrast, a too high proportion may form a salt having low solubility during the production of the polyimide precursor to prevent the production under moderate conditions or may prevent the resulting co-polyimide from having toughness (sufficiently high elongation at break).

X in general Formula (A2) of the co-polyimide precursor of the present invention is not particularly limited as long as it is a tetravalent group other than those represented by Formula (A3) and is preferably any one of tetravalent groups represented by Formula (A4) or a mixture thereof.

Furthermore, the co-polyimide precursor of the present invention may contain a third unit structure within a range that exhibits the effects of the present invention, in addition to the unit structure (a first unit structure) represented by general Formula (A1) and the unit structure (a second unit structure) represented by general Formula (A2). The third unit structure preferably has a tetravalent aromatic or aliphatic group as X in the unit structure represented by general Formula (A2). Accordingly, the third unit structure is different from the unit structure (the first unit structure) represented by general Formula (A1), and X in the third unit structure is preferably selected so as to be different from the unit structure (the second unit structure) represented by general Formula (A2) having X being a tetravalent group represented by Formula (A4) or a mixture thereof. The tetravalent aromatic group represented by Formula (A7) provides high elastic modulus at high temperature and is therefore preferable as X in the third unit structure.

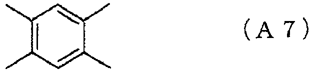

The number proportion of the third unit structures is not particularly limited, but is usually 20% or less, preferably 10% or less, and more preferably 5% or less based on the total number of the unit structures.

R₁ and R₄ in general Formulae (A1) and (A2) in the co-polyimide precursor of the present invention each independently represent a hydrogen atom or a linear or branched alkyl group having 1 to 4 carbon atoms, such as a methyl group, an ethyl group, a n-propyl group, an isopropyl group, a n-butyl group, an iso-butyl group, or a sec-butyl group. In order to provide low coefficient of linear thermal expansion to the resulting polyimide, R₁ and R₄ are each independently preferably a hydrogen atom or a methyl group, and R₁ and R₄ are more preferably hydrogen.

Although not particularly limited, in the co-polyimide precursor of the present invention, the substitution sites of the cyclohexane and the amino group in general Formulae (A1) and (A2) include 1,4-position substitution in a proportion of preferably 50% to 100%, more preferably 80% to 100%, more preferably 90% to 100%, and most preferably 100%. Furthermore, the isomeric structures of the 1,4-substituted cyclohexane preferably include 50 to 100%, more preferably 80 to 100%, more preferably 90 to 100%, and most preferably 100% of the trans-isomer. A reduction in content of the 1,4-substituted cyclohexane or the trans-configuration isomer prevents an increase in molecular weight of the polyimide precursor and may increase the coefficient of linear thermal expansion or the coloring of the resulting polyimide.

R₂, R₃, R₅, and R₆ in general Formulae (A1) and (A2) in the co-polyimide precursor of the present invention are hydrogen, alkyl groups having 1 to 6 carbon atoms such as, but not limited to, methyl, ethyl, n-propyl, isopropyl, n-butyl, iso-butyl, and sec-butyl groups, and alkylsilyl groups having 3 to 9 carbon atoms such as, but not limited to, trimethylsilyl, dimethylisopropylsilyl, tert-butyldimethylsilyl, and triisopropylsilyl groups. The trimethylsilyl group is preferred as the alkylsilyl group from the cost performance.

Furthermore, preferably, at least one of R₂ and R₃ in general Formula (A1) is an alkyl group having 1 to 6 carbon atoms or an alkylsilyl group having 3 to 9 carbon atoms; and at least one of R₅ and R₆ in general Formula (A2) is an alkyl group having 1 to 6 carbon atoms or an alkylsilyl group having 3 to 9 carbon atoms. When a part of R₂, R₃, R₅, and R₆ is an alkyl group or an alkylsilyl group, defects such as precipitation during the production of polyamic acid are improved, and a reduction in molecular weight occurring in the process of imidization can be prevented. As a result, the resulting co-polyimide has high toughness (elongation at break) and low coefficient of linear thermal expansion.

The co-polyimide precursor of the present invention may have any logarithmic viscosity without particular limitation, but the logarithmic viscosity at temperature: 30°C, concentration: 0.5 g/dL, solvent: N,N-dimethylacetamide solution is 0.2 dL/g or more and preferably 0.5 dL/g or more. A logarithmic viscosity of 0.2 dL/g or more provides a polyimide precursor with high molecular weight, which allows a polyimide film formed to have an increased mechanical strength. Furthermore, the logarithmic viscosity of the polyimide precursor of the present invention is not particularly limited, but is preferably 2.5 dL/g or less, more preferably 2.0 dL/g or less, and most preferably 1.5 dL/g or less. A low logarithmic viscosity decreases the viscosity of the polyimide precursor varnish, providing a good handling property during the step of forming a film.

The co-polyimide precursors of the present invention can be classified into 1) polyamic acid, 2) polyamic acid ester, and 3) polyamic acid silyl ester depending on the chemical structures of R₂, R₃, R₅, and R₆. The co-polyimide precursor in each group can be easily produced by the following process. The method of producing the polyimide precursor of the present invention is not limited to the following processes.

### 1) Polyamic acid

A polyimide precursor can be prepared by dissolving a diamine in an organic solvent, gradually adding a tetracarboxylic dianhydride to the resulting solution with stirring, and stirring the mixture in a temperature range of 0 to 120°C, preferably 5 to 80°C, for 1 to 72 hours. If the reaction temperature is 80°C or more, the molecular weight varies depending on the temperature history in the polymerization, and the imidization is accelerated by the heat. Accordingly, the polyimide precursor may not be stably produced.

### 2) Polyamic acid ester

A diester dicarboxylic acid chloride is prepared by reacting a tetracarboxylic dianhydride with an appropriate alcohol and reacting the resulting diester dicarboxylic acid with a chlorinating agent (e.g., thionyl chloride or oxalyl chloride). Subsequently, the diester dicarboxylic acid chloride and a diamine are stirred in a temperature range of -20 to 120°C, preferably -5 to 80°C, for 1 to 72 hours to give a polyimide precursor. If the reaction temperature is 80°C or more, the molecular weight varies depending on the temperature history in the polymerization, and the imidization is accelerated by the heat. Accordingly, the polyimide precursor may not be stably produced. The polyimide precursor can be also easily prepared by dehydration condensation of the diester dicarboxylic acid and the diamine using, for example, a phosphorus condensing agent or a carbodiimide condensing agent. Since the polyimide precursor prepared by this process is stable, for example, even purification by reprecipitation from a solvent such as water or alcohol can be employed.

### 3) Polyamic acid silyl ester

A polyimide precursor can be prepared by preparing a silylated diamine in advance by reacting a diamine and a silylating agent (the silylated diamine is optionally purified by, for example, distillation), dissolving the silylated diamine in a dehydrated solvent, gradually adding a tetracarboxylic dianhydride to the resulting solution with stirring, and stirring the mixture in a temperature range of 0 to 120°C, preferably 5 to 80°C, for 1 to 72 hours. If the reaction temperature is 80°C or more, the molecular weight varies depending on the temperature history in the polymerization, and the imidization is accelerated by the heat. Accordingly, the polyimide precursor may not be stably produced. Here, the use of a chlorine-free silylating agent as the silylating agent does not need purification of the resulting silylated diamine and is preferable. Examples of the chlorine-free silylating agent include N,O-bis(trimethylsilyl)trifluoroacetamide, N,O-bis(trimethylsilyl)acetamide, and hexamethyldisilazane. N,O-Bis(trimethylsilyl)acetamide and hexamethyldisilazane are preferable because they do not contain fluorine atoms and inexpensive. In order to facilitate the silylation of the diamine, an amine catalyst such as pyridine, piperidine, or triethylamine may be used. The catalyst can be also used as the polymerization catalyst of the polyimide precursor as it is.

All of the methods of production described above can be suitably performed in organic solvents. As a result, the co-polyimide precursor solution composition of the present invention can be readily prepared.

In each method of production, the molar ratio of the tetracarboxylic acid component to the diamine component can be appropriately determined depending on the purposed viscosity of the polyimide precursor and is preferably 0.90 to 1.10 and more preferably 0.95 to 1.05.

The tetracarboxylic acid component for the co-polyimide precursor of the present invention contains (i) 3,3', 4,4' - biphenyltetracarboxylic acids constituting a tetracarboxylic acid component in general general Formula (A1), and (ii) tetracarboxylic acid component other than 3,3', 4,4'-biphenyltetracarboxylic acids and pyromellitic acids and constituting a tetracarboxylic acid component in general general Formula (A2). There is no specific limitation for the tetracarboxylic acid component other than 3,3', 4,4' - biphenyltetracarboxylic acids and pyromellitic acids and any kind used for general polyimides may be used, but preferred is aromatic tetracarboxylic acids. The preferred examples of the tetracarboxylic acids include 2, 3, 3', 4'-biphenyltetracarboxylic acids, 2,2' ,3, 3' -biphenyltetracarboxylic acids, oxydiphthalic acids, 3,3', 4,4' - benzophenone tetracarboxylic acids, 3, 3 ', 4,4' - diphenylsulfone tetracarboxylic acids, m-terphenyl-3, 3 ', 4,4' - tetracarboxylic acids, 4,4'-(2,2 hexafluoroisopropylene)diphthalic acids, 2,2'-bis(3,4-dicarboxyphenyl) propanes, 1,4,5,8 - naphthalene tetracarboxylic acid, 2,3,6,7-naphthalene tetracarboxylic acid, (1,1':3',1"- terphenyl) -3, 3", 4, 4"-tetracarboxylic acid, 4,4'- (dimethylsiladiyl)diphthalic acids, 4,4' - (1,4-phenylenebis(oxy))diphthalic acids and the like. 2, 3, 3', 4'-biphenyltetracarboxylic acids, 2,2' ,3, 3' -biphenyltetracarboxylic acids, oxydiphthalic acids, 4,4'-(2,2 hexafluoroisopropylene)diphthalic acids and 4,4'- (dimethylsiladiyl)diphthalic acids are more preferred since they provide particularly high transparency. 2, 3, 3', 4'-biphenyltetracarboxylic acids, 2,2' ,3, 3' -biphenyltetracarboxylic acids and oxydiphthalic acids are particularly preferred since they provide low coefficient of thermal expansion; and 4,4'-(2,2 hexafluoroisopropylene)diphthalic acids and 4,4'-(dimethylsiladiyl)diphthalic acids are particularly preferred since they provide particularly high transparency.
Herein, the above tetracarboxylic acids include any of tetracarboxylic acid, tetracarboxylic anhydride, and derivative such as tetracarboxylic acid ester, and are used as a compound having preferred chemical structure for raw materials for the above production method.

As the diamine compound component, preferably used are diamines having a cyclohexane structure which may be optionally substituted constituting general Formulae (A1) and (A2). The examples thereof include, but not limited, preferably 1, 4-diaminocyclohexane, 1, 4-diamino-2-methylcyclohexane, 1,4-diamino-2-ethylcyclohexane, 1, 4-diamino-2-n-propylcyclohexane, 1, 4-diamino-2-isopropylcyclohexane, 1,4-diamino-2-n-butylcyclohexane, 1, 4-diamino-2-isobutylcyclohexane, 1, 4-diamino-2-sec-butylcyclohexane, 1, 4-diamino-2-tert-butylcyclohexane, 1,2-diaminocyclohexane. In particular, 1, 4-diaminocyclohexane is preferred because it provides a polyimide film having low coefficient of linear thermal expansion. Furthermore, 1, 4-steric configuration of the diamines having 1, 4-cyclohexane structure is not particularly limited, but it is preferably trans-configuration. Cis-configuration tends leading to a drawback such as coloring.

As a solvent used for the above-mentioned production method, those capable of dissolving the raw material monomers and the produced polyimide precursors may be used without any problem and the structure thereof is not limited. The examples preferably used include amide solvents such as N, N-dimethylformamide, N, N-dimethylacetamide and N-methylpyrrolidone; cyclic ester solvents such as γ-butyrolactone, γ-valerolactone, δ-valerolactone, γ-caprolactone, ε-caprolactone, and α-methyl-γ-butyrolactone; carbonate solvents such as ethylene carbonate and propylene carbonate; glycol-based solvents such as triethylene glycol; phenol-based solvents such as m-cresol, p-cresol, 3-chlorophenol and 4-chlorophenol; acetophenone, 1,3-dimethyl-2-imidazolidinone, sulfolane, and dimethylsulfoxide. Due to particularly excellent solubility, preference is give to aprotic solvents, such as N, N-dimethylformamide, N, N-dimethylacetamide, N-methyl-2-pyrrolidone, N-ethyl-2-pyrrolidone and dimethyl sulfoxide. In addition, other common organic solvents, for example, phenol, o-cresol, butyl acetate, ethyl acetate, isobutyl acetate, propyleneglycol methyl acetate, ethyl cellosolve, butyl cellosolve, 2-methylcellosolve acetate, ethylcellosolve acetate, butylcellosolve acetate, tetrahydrofuran, dimethoxyethane, diethoxyethane, dibutyl ether, diethylene glycol dimethyl ether, methyl isobutyl ketone, diisobutyl ketone, cyclopentanone, cyclohexanone, methyl ethyl ketone, acetone, butanol, ethanol, xylene, toluene, chlorobenzene, turpentine, mineral spirits, and petroleum naphtha-based solvents may be used. These solvents are preferably purified by distillation, dehydrating agent treatment, etc. for removing acidic components, alkaline components, metal components, and water and have a purity of 99.5% or more, preferably 99.7% or more, and more preferably 99.9% or more. High purity of the solvent provides high light transmittance of the produced polyimide film and therefore preferable. The solvents exemplified here is referred to as "organic solvent used in the method of production" in the other portions in this Part and in the other Part, and the preferred organic solvent is the same unless otherwise indicated.

The organic solvent (also may be referred to as an organic solvent or solvent) used in this Part is the organic solvent used in each step involved in the production of a polyimide precursor varnish. Examples of the organic solvent include the organic solvent used in the polymerization, the organic solvent used in the step of diluting the varnish to purposed concentration and viscosity, and the organic solvent used for preparing a dilution of, for example, an additive in advance.

In the method of production of the present invention, when the molar ratio of the tetracarboxylic acid component to the diamine component is an excess molar ratio of the diamine component, a carboxylic acid derivative can be optionally added in an amount approximately corresponding to the number of moles of the excess diamine such that the molar proportion of the tetracarboxylic acid component is approximately equivalent to the molar proportion of the diamine component. The carboxylic acid derivative optionally added here is tetracarboxylic acids that substantially do not increase the viscosity of the polyimide precursor solution (i.e., substantially does not participate in extension of molecular chain) or tricarboxylic acids and their anhydride or dicarboxylic acids and their anhydride functioning as a chain terminator.

The tetracarboxylic acid derivatives include 3,3',4,4'-biphenyltetracarboxylic acid, 2,3,3',4'-biphenyltetracarboxylic acid, 2,2',3,3'-biphenyltetracarboxylic acid, 1,2,3,4-butanetetracarboxylic acid, benzene-1,2,4,5-tetracarboxylic acid. The tricarboxylic acids include trimellitic acid and cyclohexane-1,2,4-tricarboxylic acid and acid anhydrides thereof. The dicarboxylic acids include phthalic acid, tetrahydrophthalic acid, cis-norbornene-endo-2,3-dicarboxylic acid, cyclohexanedicarboxylic acid, succinic acid, and maleic acid and acid anhydrides thereof. Use of these carboxylic acid derivatives may prevent the thermal coloring and thermal degradation during the heating. In particular, tetracarboxylic acid derivatives such as biphenyltetracarboxylic acids or carboxylic acid derivatives having a reactive functional group are preferred because they react during the imidization and improve a heat resistance.

The co-polyimide precursor solution composition of the present invention contains at least the co-polyimide precursor of the present invention and a solvent. The total amount of the tetracarboxylic acid component and the diamine component is 5% by mass or more, preferably 10% by mass or more, and more preferably 15% by mass or more based on the total amount of the solvent, the tetracarboxylic acid component, and the diamine component, and is usually 60% by mass or less and preferably 50% by mass or less. If the concentration is too low, it may be difficult to control the thickness of a film formed from the co-polyimide.

The solvent contained in the co-polyimide precursor solution composition of the present invention may be any solvent that can dissolve the polyimide precursor and is not particularly limited by the structure. Specific examples of the solvent include those exemplified as the "solvents used in the production" above. These solvents may be used in combination of two or more thereof. These solvents are preferably purified by distillation, dehydrating agent treatment, etc. for removing acidic components, alkaline components, metal components, and water and have a purity of 99.5% or more, preferably 99.7% or more, and more preferably 99.9% or more.

The polyimide precursor solution composition of the present invention may optionally contain a chemical imidization agent (an acid anhydride such as acetic anhydride or an amine compound such as pyridine or isoquinoline), an antioxidant, a filler, a dye, a pigment, a coupling agent such as silane coupling agent, a primer, a fire-retarding material, an antifoaming agent, a leveling agent, a rheology-controlling agent (flow assistant), a release agent, etc.

The co-polyimide of the present invention is characterized in that it has a unit structure represented by general Formula (A5) and a unit structure represented by general Formula (A6), and a preferred co-polyimide is prepared through a cyclodehydration reaction (imidization reaction) of the co-polyimide precursor of the present invention. Accordingly, the above-described factors (e.g., ratio of the unit structures and the third unit structure) regarding the co-polyimide precursor are applied to the resulting polyimide, i.e., co-polyimide of the present invention. The process of imidization is not particularly limited, and known thermal imidization or chemical imidization is suitably employed. Preferred examples of the form of the resulting polyimide include films, laminates of polyimide films and other base materials, coating films, powders, beads, molded products, foamed products, and varnishes.

The co-polyimide of the present invention preferably has, when formed into a film having a thickness of 10 µm, an elongation at break at room temperature of 8% or more in a tensile test and a light transmittance at 400 nm of 50% or more, and more preferably has an elastic modulus at room temperature of 3 GPa or more, an elongation at break of 10% or more, and a light transmittance at 400 nm of 75% or more, and thus has excellent transparency and toughness (sufficient elongation at break) that can endure bending.

Furthermore, the co-polyimide of the present invention has, but not limited to, an average coefficient of linear thermal expansion at 50 to 200°C of 20 ppm/K or less, more preferably 15 ppm/K or less, in the film face direction when formed into a film.

Furthermore, in the dynamic viscoelastic measurement of a film having a thickness of 10 µm formed from the co-polyimide of the present invention, as compared with a minimum storage elastic modulus observed at a temperature not lower than the glass transition temperature determined from the maximum point of tan δ, the co-polyimide of the present invention preferably has, but not limited to, a maximum storage elastic modulus at a temperature not lower than the temperature at which the minimum storage elastic modulus is observed. A co-polyimide having the maximum storage elastic modulus at a temperature not lower than the glass transition temperature can prevent a decrease of elastic modulus at high temperature and therefore can be formed into a polyimide film enduring the process at high temperature.

The thickness of a film formed from the co-polyimide of the present invention is determined depending on the purpose and is preferably about 1 to 250 µm and more preferably about 1 to 150 µm.

The polyimide of the present invention has excellent characteristics such as transparency, bending resistance, and high heat resistance, and further has a considerably low coefficient of linear thermal expansion and high solvent resistance. Therefore, the polyimide is suitably applied to a display transparent substrate, a touch panel transparent substrate, or a solar cell substrate.

An example of the method of producing a polyimide film/base material laminate or a polyimide film using the polyimide precursor of the present invention will now be described, but the method is not limited to the following method. The application examples of the polyimide precursor described here can also apply to the polyimide precursors disclosed in other Parts.

The polyimide precursor solution composition of the present invention is cast onto a base material such as a ceramic (glass, silicon, alumina), a metal (copper, aluminum, stainless steel), or a thermally stable plastic film (polyimide) and is dried in a temperature range of 20 to 180°C, preferably 20 to 150°C, with hot air or infrared radiation in vacuum, in an inert gas such as nitrogen, or in the air. Subsequently, the resulting polyimide precursor film is heated for imidization on the base material or in a state peeled from the base material and fixed at the ends at 200 to 500°C, more preferably about 250 to 450°C, with hot air or infrared radiation in vacuum, in an inert gas such as nitrogen, or in the air. Thus, a polyimide film/base material laminate or a polyimide film can be produced. The thermal imidization in vacuum or in an inert gas is desirable for preventing oxidative degradation of the resulting polyimide film. If the temperature for the thermal imidization is not too high, thermal imidization in the air is allowable. The thickness of the polyimide film (in the case of the polyimide film/base material laminate, the polyimide film layer) is preferably 1 to 250 µm, more preferably 1 to 150 µm, for the transportability in the subsequent steps.

The imidization of the polyimide precursor may be also performed by chemical treatment by, for example, immersing the polyimide precursor in a solution containing a cyclodehydrating agent such as acetic anhydride in the presence of a tertiary amine such as pyridine or triethylamine, instead of the thermal imidization by heat treatment as described above. In addition, a partially imidized polyimide precursor may be produced by stirring a polyimide precursor solution composition containing a cyclodehydrating agent in advance, casting the mixture onto a base material, and drying it. The partially imidized polyimide precursor can be formed into a polyimide film/base material laminate or a polyimide film by the heat treatment described above.

The thus-prepared polyimide film/base material laminate or polyimide film can be formed into a flexible conductive substrate by forming a conductive layer on one surface or both surfaces of the laminate or the film.

An example of the method of producing a laminate using the polyimide/substrate laminate will now be described. The method includes a step of producing a polyimide/substrate laminate by applying a polyimide precursor solution composition onto a ceramic substrate, a metal substrate, or a thermally stable plastic substrate and heating the composition in vacuum, nitrogen, or the air at 200 to 500°C for imidization; a step of producing a thin film/polyimide/substrate laminate by forming a ceramic thin film or metal thin film on the polyimide surface of the resulting laminate without peeling off the polyimide from the substrate; and a step of peeling off the polyimide from the substrate. Since the polyimide is subjected to the subsequent steps such as formation of the thin film by, for example, sputtering deposition in the state of polyimide/substrate laminate without peeling of the polyimide from the substrate, the method is economical and provides good transportability, size stability, and high dimensional accuracy in machining.

The flexible conductive substrate can be prepared by, for example, the following methods. That is, in a first method, a conductive laminate of (conductive layer)/(polyimide film)/(base material) is produced by forming a conductive layer of a conductive material (e.g., a metal, a metal oxide, a conductive organic material, or conductive carbon) on the polyimide film surface of the (polyimide film)/( base material) laminate without peeling the polyimide film from the substrate by, for example, sputtering deposition or printing. Subsequently, the (electrically conductive layer)/(polyimide film) laminate is peeled from the base material as necessary to provide a transparent and flexible conductive substrate composed of conductive layer/polyimide film laminate.
In a second method, the polyimide film is peeled off from the base material of a (polyimide film)/(base material) laminate to provide a polyimide film, and a conductive layer of a conductive material (e.g., a metal, a metal oxide, a conductive organic material, or conductive carbon) is formed on the polyimide film surface as in the first method to provide a transparent and flexible conductive substrate composed of (conductive layer)/(polyimide film) laminate.
In the first and the second methods, a gas-barrier layer against water vapor, oxygen, etc. or an inorganic layer such as a light controlling layer may be optionally formed by, for example, sputtering deposition or a gel-sol method before the formation of the conductive layer on the polyimide film surface.
Furthermore, a circuit is suitably formed on the conductive layer by a method such as photolithography, various printing methods, or ink-jetting.

The substrate of the present invention includes the circuit of the conductive layer on the surface of a polyimide film formed from the polyimide of the present invention, if necessary via a gas-barrier layer or an inorganic layer. The substrate is flexible and has excellent transparency, bending resistance, and heat resistance and also has a considerably low coefficient of linear thermal expansion and high solvent resistance. Therefore, a fine circuit can be readily formed. Accordingly, the substrate can be suitably used as a substrate for a display, touch panel, or solar cell.
That is, a flexible thin-film transistor is produced by further forming a transistor (inorganic transistor or organic transistor) on the substrate by a method such as deposition, various printing methods, or ink-jet method and is suitably used as a liquid crystal device for a display device, EL device, or photoelectric device. The application examples of the polyimide precursor described here can also apply to the polyimide precursors disclosed in other Parts.

### « PART B »

An object of the invention disclosed in Part B is to provide a polyimide precursor using an alicyclic diamine, where the polyimide precursor can be produced by a method suitable for actual industrial production and has a good handling property and storage stability. The polyimide prepared from such a polyimide precursor has high transparency, high glass transition temperature, low coefficient of linear thermal expansion and also has sufficiently high toughness. Accordingly, the polyimide can be suitably used in a plastic substrate as a replacement for the glass substrate of, in particular, a display device such as a liquid crystal display, an EL display, or electronic paper.

The invention disclosed in Part B relates to the following items.

1. A polyimide precursor, comprising a unit structure represented by general Formula (B1):

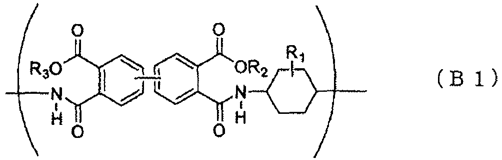

wherein, in general Formula (B1), R₁ represents a hydrogen atom or an alkyl group having 1 to 4 carbon atoms; R₂ and R₃ each represent a hydrogen atom or an alkylsilyl group having 3 to 9 carbon atoms, and at least one of R₂ and R₃ is an alkylsilyl group having 3 to 9 carbon atoms.

2. The polyimide precursor according to item 1, wherein general Formula (B1) is represented by general Formula (B2):

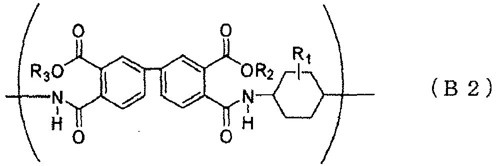

wherein, in general Formula (B2), R₁ represents a hydrogen atom or an alkyl group having 1 to 4 carbon atoms; R₂ and R₃ each represent a hydrogen atom or an alkylsilyl group having 3 to 9 carbon atoms, and at least one of R₂ and R₃ is an alkylsilyl group having 3 to 9 carbon atoms.

3. The polyimide precursor according to item 1 or 2, wherein the 1,4-cyclohexane structure in general Formula (B1) is a trans-isomer.

4. The polyimide precursor according to any one of items 1 to 3, having a logarithmic viscosity of 0.2 dL/g or more as a 0.5 g/dL solution in N,N-dimethylacetamide at 30°C.

5. A polyimide precursor solution composition, wherein the polyimide precursor according to any one of items 1 to 4 is uniformly dissolved in a solvent.

6. A polyimide prepared by imidization of the polyimide precursor according to any one of items 1 to 4.

7. The polyimide according to item 6, having a light transmittance at 400 nm of 50% or more and an elongation at break of 8% or more when formed into a film having a thickness of 10 µm.

8. The polyimide according to item 6, having an average coefficient of linear thermal expansion at 50 to 200°C of 19 ppm/K or less when formed into a film having a thickness of 10 µm.

9. A method of producing a polyimide precursor, comprising preparing a polyimide precursor containing a unit structure represented by general Formula (B1) at a polymerization temperature of 0 to 100°C.

10. A method of producing a polyimide precursor, comprising preparing a polyimide precursor containing a unit structure represented by general Formula (B1) by reacting at least a biphenyltetracarboxylic dianhydride and a diamine represented by general Formula (B3):

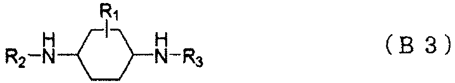

wherein, in general Formula (B3), R₁ represents a hydrogen atom or an alkyl group having 1 to 4 carbon atoms; R₂ and R₃ each represent a hydrogen atom or an alkylsilyl group having 3 to 9 carbon atoms, and at least one of R₂ and R₃ is an alkylsilyl group having 3 to 9 carbon atoms.

11. A method of producing a polyimide precursor, comprising preparing a polyimide precursor containing a unit structure represented by general Formula (B1) using a chlorine- and bromine-free silylating agent.

According to the invention disclosed in Part B, a polyimide precursor can be prepared using an alicyclic diamine by a method suitable for actual industrial production so as to have a good handling property and storage stability. The polyimide prepared from such a polyimide precursor has high transparency, high glass transition temperature, low coefficient of linear thermal expansion and also has sufficiently high toughness. Accordingly, the polyimide can be suitably used, in particular, in a plastic substrate as a replacement for the glass substrate of a display device such as a liquid crystal display, an EL display, or electronic paper.

The invention disclosed in Part B will now be described in detail.
The polyimide precursor, comprising a unit structure represented by general Formula (B1) of the present invention can be produced by, but not limited to, a process of reacting a diamine represented by general Formula (B3) silylated in advance and a tetracarboxylic dianhydride or a process of reacting a diamine, a tetracarboxylic dianhydride, and a silylating agent mixed at the same time. The former process can prevent salt formation in the initial stage of polymerization and is therefore preferable.

The diamine represented by general Formula (B3) can be prepared by, but not limited to, silylating a diamine represented by general Formula (B4) shown below with, for example, a silylating agent,

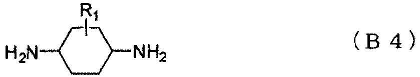

wherein, in general Formula (B4), R₁ represents a hydrogen atom or an alkyl group having 1 to 4 carbon atoms.

Examples of the diamine represented by general Formula (B4) include those having R₁ being a hydrogen atom or a linear or branched alkyl group having 1 to 4 carbon atoms such as a methyl group, an ethyl group, a n-propyl group, an isopropyl group, a n-butyl group, an iso-butyl group, or a sec-butyl group. Among these diamines, 1,4-diaminocyclohexane, 1,4-diamino-2-methylcyclohexane, 1,4-diamino-2-ethylcyclohexane, 1,4-diamino-2-n-propylcyclohexane, 1,4-diamino-2-isopropylcyclohexane, 1,4-diamino-2-n-butylcyclohexane, 1,4-diamino-2-isobutylcyclohexane, 1,4-diamino-2-sec-butylcyclohexane, and 1,4-diamino-2-tert-butylcyclohexane are preferable. In particular, 1,4-diaminocyclohexane can form a polyimide film having low coefficient of linear thermal expansion and is therefore more preferable.

Examples of the method of preparing the diamine represented by general Formula (B3) through silylation of a diamine represented by general Formula (B4) include, but are not limited to, 1) a process of reacting a diamine and a chlorine- and bromine-free silylating agent to give a mixture of a silylated diamine and a residual compound of the silylating agent; and 2) a process of reacting a diamine and a trialkylsilyl chloride and then purifying the reaction product by, for example, distillation to give a silylated diamine. The process 1) is preferable because it does not need any purification and allows shorter process.

In the process 1), a silylated diamine can be readily prepared by reacting a diamine and a chlorine- and bromine-free silylating agent in an inert gas atmosphere at 20 to 100°C for 10 minutes to 10 hours.

Preferred examples of the silylating agent used in the present invention include, but are not limited to, chlorine- and bromine-free silylating agents such as N,O-bis(trimethylsilyl)trifluoroacetamide, N,O-bis(trimethylsilyl)acetamide, and hexamethyldisilazane. The use of a chlorine- and bromine-free silylating agent does not leave chlorine and bromine compounds, for which burden on the environment is concerned, as residues even if purification is not performed and is therefore preferable. Furthermore, N,O-bis(trimethylsilyl)acetamide and hexamethyldisilazane do not contain fluorine atoms and are inexpensive and are therefore preferable. In order to facilitate the silylation, a catalyst such as pyridine, piperidine, or triethylamine may be used. The catalyst can be also used as the polymerization catalyst of the polyimide precursor as it is.

In the present invention, the silylation ratio of the silylated diamine represented by general Formula (B3) is not particularly limited as long as it is higher than the minimum silylation ratio required for preventing defects such as precipitation during the production of the polyimide precursor. The silylation ratio is the molar ratio of the silylated amine to the total amino groups of the diamine before the silylation and is 25 to 100% and preferably 50 to 100%. A low silylation ratio reduces the solubility during the reaction for preparing the polyimide precursor, resulting in a tendency of precipitation.

In the present invention, as the silylated diamine represented by general Formula (B3), preferred is diamines in which R₁ in general Formula (B3) represents a hydrogen atom or a linear or branched alkyl group having 1 to 4 carbon atoms such as a methyl group, an ethyl group, a n-propyl group, an isopropyl group, a n-butyl group, an iso-butyl group, or a sec-butyl group, preferably hydrogen atom or a methyl group and more preferably hydrogen atom in view of low coefficient of linear thermal expansion of the produced polyimide film. In addition, R₂ and R₃ are not limited as long as at least one of R₂ and R₃ is an alkylsilyl group having 3 to 9 carbon atoms, and are preferably trimethylsilyl, dimethylisopropylsilyl, tert-butyldimethylsilyl, and triisopropylsilyl groups. Trimethylsilyl group is preferred from the cost performance.

Although not particularly limited, the structures of the 1,4-substituted cyclohexane preferably include trans-isomer in proportion of 50 to 100%, preferably 60 to 100%, and more preferably 80 to 100%. If a proportion of trans-configuration isomer is lowered, a polyimide precursor having high molecular weight may not be obtained, and in addition, the coefficient of linear thermal expansion may become high.

As the biphenyltetracarboxylic dianhydride for producing a polyimide precursor of the present invention, there can be used any of structural isomers from 3, 3', 4,4' - biphenyltetracarboxylic dianhydride, 2, 3, 3',4' - biphenyltetracarboxylic dianhydride, and 2, 3, 2', 3' - biphenyltetracarboxylic dianhydride. Also, combination of these structural isomers may be used. Herein, the proportion of 3, 3', 4,4' - biphenyltetracarboxylic dianhydride is not limited as long as the required properties are not deteriorated, but it is 50 to 100%, preferably 80 to 100%, more preferably 90 to 100%, and most preferably 100% based on the total moles of biphenyltetracarboxylic dianhydrides. High content of 3, 3', 4,4' - biphenyltetracarboxylic dianhydride leads to lower coefficient of linear thermal expansion. Use of 2, 3, 3',4' - biphenyltetracarboxylic dianhydride or 2, 3, 2', 3'-biphenyltetracarboxylic dianhydride for biphenyltetracarboxylic dianhydride for producing a polyimide precursor of the present invention improves the solubility of the polyimide precursor leading to easiness of the production and increases the elongation at break and transparency of the produced polyimide.

In the tetracarboxylic dianhydride for producing the polyimide precursor of the present invention, a tetracarboxylic dianhydride other than the above biphenyltetracarboxylic dianhydride may be used in an amount of 50 % or less, preferably 20 % or less, more preferably 10 % or less based on the total moles of tetracarboxylic dianhydrides. Use of the tetracarboxylic dianhydride other than biphenyltetracarboxylic dianhydrides improves the solubility of the polyimide precursor leading to easiness of the production. The tetracarboxylic dianhydride other than biphenyltetracarboxylic dianhydrides is not particularly limited and may be any tetracarboxylic dianhydride generally employed for a polyimide, but an aromatic tetracarboxylic dianhydride is preferred. The examples of such tetracarboxylic dianhydride include pyromellitic dianhydride, oxydiphthalic dianhydride, 3,3', 4,4' - benzophenone tetracarboxylic dianhydride, 3, 3 ', 4,4' - diphenylsulfone tetracarboxylic dianhydride, m-terphenyl-3, 3 ', 4,4' - tetracarboxylic dianhydride, 4,4'-(2,2 hexafluoroisopropylene)diphthalic dianhydride, 2,2'-bis(3,4-dicarboxyphenyl)propane dianhydride, 1,4,5,8 - naphthalenetetracarboxylic dianhydride, 2,3,6,7 - naphthalenetetracarboxylic dianhydride, (1,1':3',1"- terphenyl) -3, 3", 4, 4" - tetracarboxylic dianhydride, 4,4'- (dimethyl-siladiyl)diphthalic dianhydride, 4,4' - (1,4 - phenylene bis(oxy)) diphthalic dianhydride and the like, and more preferably 2, 2', 3, 3' - biphenyltetracarboxylic dianhydride and 2, 2', 3, 3' - biphenyltetracarboxylic dianhydride.

The method of producing a polyimide precursor of the present invention is not particularly limited. Preferably, a silylated diamine is dissolved in a dehydrated solvent under an atmosphere of an inert gas such as nitrogen, and a tetracarboxylic dianhydride is added to the solution with stirring. The reaction temperature is 0 to 100°C, preferably 20 to 80°C, and most preferably 40 to 80°C. A reaction temperature of 100°C or less does not cause imidization and therefore allows stable production of the polyimide precursor and also can reduce the manufacturing cost and is preferable. The end point of the reaction is the time at which the viscosity of the polyimide precursor becomes constant. The reaction time varies depending on the types of the tetracarboxylic anhydride and the diamine and the temperature, but is usually 3 to 12 hours.

The polyimide precursor produced by this method has high solubility, unlike known polyimide precursors (polyamic acids), and therefore the salt of the polyimide precursor and the diamine hardly precipitates. Thus, the method is suitable for actual industrial production. The molecular weight of the polyimide precursor can be controlled by performing polymerization while adjusting the molar ratio of the tetracarboxylic dianhydride to the diamine and confirming the molecular weight by measuring the viscosity or GPC, and stable production is possible. The polyimide precursor of the present invention has high solubility and can therefore produce a polyimide precursor solution (composition) with a relatively high concentration.

The method of producing the polyimide precursor of the present invention preferably uses an organic solvent. Specific examples of the organic solvent include those exemplified as the "organic solvent used in the method of production" in Part A.

In the present invention, the concentration of monomer components composed of the tetracarboxylic dianhydride and the diamine in the finally prepared polyimide precursor solution (composition) is not particularly limited, but is 5% by weight or more, preferably 10% by weight or more, and most preferably 15 to 50% by weight based on the total amount of the monomer components and the solvent. Higher concentration of the monomer components allows formation of a thick polyimide film.

The molar ratio of the tetracarboxylic dianhydride to the diamine used (tetracarboxylic dianhydride/diamine) can be appropriately determined depending on the target viscosity of the polyimide precursor and is preferably 0.90 to 1.10 and more preferably 0.95 to 1.05.

In the method of producing the polyimide precursor of the present invention, when the total moles of diamines with respect to the total moles of tetracarboxylic dianhydrides is excess, a tetraacid derivative or an acid anhydride derivative can be added to the polyimide precursor solution. Examples of the tetraacid derivative include 1,2,3,4-butanetetracarboxylic acid, benzene-1,2,4,5-tetracarboxylic acid, and biphenyltetracarboxylic acid. Examples of the acid anhydride include phthalic anhydride, tetrahydrophthalic anhydride, cis-norbornene-endo-2,3-dicarboxylic anhydride, cyclohexanedicarboxylic anhydride, succinic anhydride, and maleic anhydride. The use of a tetraacid derivative or an acid anhydride can further prevent thermal coloring and thermal degradation during the heating.

The logarithmic viscosity of the polyimide precursor of the present invention is not particularly limited and is preferably 0.2 dL/g or more, more preferably 0.5 dL/g or more, as a 0.5 g/dL solution in N,N-dimethylacetamide at 30°C. When the logarithmic viscosity is 0.2 dL/g or less, the polyimide precursor has a low molecular weight to reduce the mechanical strength of the resulting polyimide film. The logarithmic viscosity is also preferably 2.5 dL/g or less and more preferably 2.0 dL/g or less. When the logarithmic viscosity is 2.0 dL/g or less, the polyimide precursor solution composition has a low viscosity to provide a good handling property during the polyimide film production.

The polyimide precursor solution composition (varnish) of the present invention is mainly composed of a polyimide precursor and a solvent. The concentration of monomer components composed of the tetracarboxylic dianhydride and the diamine is 10% by weight or more, more preferably 15 to 50% by weight, based on the total amount of the monomer components and the solvent. If the monomer concentration is 10% by weight or less, it is difficult to control the thickness of the prepared polyimide film. The polyimide precursor of the present invention has high solubility and can thereby provide a polyimide precursor solution composition with a relatively high concentration.

The solvent contained in the polyimide precursor composition of the present invention may be any solvent that can dissolve the polyimide precursor and is not particularly limited by the structure. Specific examples of the solvent include those exemplified as the "organic solvent used in the method of production" in Part A.

The polyimide precursor solution composition of the present invention may optionally contain a generally-used chemical imidization agent (an acid anhydride such as acetic anhydride or an amine compound such as pyridine or isoquinoline), an antioxidant, a filler, a dye, an inorganic pigment, a silane coupling agent, a fire-retarding material, an antifoaming agent, a leveling agent, a rheology-controlling agent (flow assistant), a release agent, etc.

The polyimide of the present invention can be produced through a cyclization reaction (imidization reaction) of the polyimide precursor of the present invention. The imidization may be performed by any method, and known thermal imidization or chemical imidization can be employed. Examples of the usable form of the polyimide include films, metal/polyimide film laminates, ceramic/polyimide film laminates, plastic film/polyimide laminates, powders, molded products, and varnishes.

The polyimide of the present invention has excellent transparency having a light transmittance at 400 nm of, preferably 50% or more, more preferably 75% or more, and most preferably 80% or more when formed into a film having a thickness of 10 µm.

Furthermore, the polyimide of the present invention has a considerably low coefficient of linear thermal expansion at 50 to 200°C of 50 ppm/K or less, more preferably -5 to 19 ppm/K, and most preferably 0 to 15 ppm/K on average when formed into a film.

The thickness of a film formed from the polyimide of the present invention depends on the purpose and is preferably about 1 to 250 µm and more preferably about 1 to 150 µm.

The polyimide of the present invention has excellent characteristics such as transparency, bending resistance, and high heat resistance, and further has a considerably low coefficient of linear thermal expansion and high solvent resistance. Therefore, the polyimide can be suitably applied to a display transparent substrate, a touch panel transparent substrate, or a solar cell substrate.

The polyimide precursor of the present invention can be used for producing a (polyimide film)/(base material) laminate or a polyimide film. Examples of the method of production are as those described in Part A, and the (polyimide film)/(base material) laminate or the polyimide film can be produced as in Part A, and also a flexible conductive substrate can be produced as in Part A.

### « PART C »

The invention disclosed in Part C relates to a method of purifying a 2,3,3',4'-biphenyltetracarboxylic dianhydride powder having reduced color, the powder, and a polyimide prepared using the powder. Here, the 2,3,3',4'-biphenyltetracarboxylic dianhydride powder is a powder mainly composed of 2,3,3',4'-biphenyltetracarboxylic dianhydride and is suitably used as a chemical raw material substantially consisting of 2,3,3',4'-biphenyltetracarboxylic dianhydride.

As described in Background Art, the production methods described in Japanese Patent Laid-Open Nos. 2006-328040 and 2009-019014 are each for producing a powder of 2,3,3',4'-biphenyltetracarboxylic dianhydride with high purity that allows production of polyamic acid having high logarithmic viscosity. Though these methods achieve the purpose, any investigation for reducing coloring of the 2,3,3',4'-biphenyltetracarboxylic dianhydride powder has not been performed. Thus, there has still been a room for improvement in coloring.
As described in Japanese Patent Laid-Open No. 2009-019014 (paragraphs 0032 and 0033), the 2,3,3',4'-biphenyltetracarboxylic dianhydride powder behaves absolutely differently from a 3,3',4,4'-biphenyltetracarboxylic dianhydride powder. That is, the 2,3,3',4'-biphenyltetracarboxylic dianhydride powder has low crystallinity to readily generate an amorphous portion as well as a crystalline portion. The amorphous portion is believed to cause quality deterioration, and the 2,3,3',4'-biphenyltetracarboxylic dianhydride powder is obviously different in hue and contained components such as moisture content, in addition to the difference between a crystalline tendency and an amorphous tendency.

The invention disclosed in Part C was made as a result of various investigations for reducing the coloring of the 2,3,3',4'-biphenyltetracarboxylic dianhydride powder having such specific properties.
That is, the purpose of the invention disclosed in Part C is to provide a purifying method to readily obtain a 2,3,3',4'-biphenyltetracarboxylic dianhydride powder having reduced color by a simple procedure, a 2,3,3',4'-biphenyltetracarboxylic dianhydride powder having reduced color, and a polyimide having an increased light transmittance that can be suitably used as a high-performance optical material.

The invention disclosed in Part C relates to the following items.
1. A method of purifying a 2,3,3',4'-biphenyltetracarboxylic dianhydride powder, comprising mixing a solvent in which the solubility of 2,3,3',4'-biphenyltetracarboxylic dianhydride at 25°C is 1 g/100 g or more and a 2,3,3',4'-biphenyltetracarboxylic dianhydride powder in an uneven state where at least a part of the 2,3,3',4'-biphenyltetracarboxylic dianhydride powder is not dissolved; and separating and collecting the undissolved 2,3,3',4'-biphenyltetracarboxylic dianhydride powder from the mixture.

2. The method of purification according to item 1, wherein the solubility of 2,3,3',4'-biphenyltetracarboxylic dianhydride in the solvent at 25°C is 1 g/100 g to 30 g/100 g.

3. The method of purification according to item 1 or 2, wherein the solvent is acetone.

4. A 2,3,3',4'-biphenyltetracarboxylic dianhydride powder, having a light transmittance of 85% or more at a wavelength of 400 nm and an optical path length of 1 cm as a 10% by mass solution in a 2 N aqueous sodium hydroxide solution.

5. The 2,3,3',4'-biphenyltetracarboxylic dianhydride powder according to item 4, wherein the light transmittance at a wavelength of 400 nm and an optical path length of 1 cm is 90% or more.

6. A polyimide produced from the 2,3,3',4'-biphenyltetracarboxylic dianhydride powder according to item 4 or 5, having improved light transmittance when formed into a film.

7. The polyimide according to item 6, having a light transmittance of 70% or more at 400 nm when formed into a film having a thickness of 10 µm.

The invention disclosed in Part C can provide a method of readily purifying a 2,3,3',4'-biphenyltetracarboxylic dianhydride powder having reduced color by a simple procedure, a 2,3,3',4'-biphenyltetracarboxylic dianhydride powder having reduced color, and a polyimide having an increased light transmittance that can be suitably used as a high-performance optical material.
The 2,3,3',4'-biphenyltetracarboxylic dianhydride powder prepared by the invention disclosed in Part C can provide an end product having higher transparency, in particular, a polyimide by using it in place of the 2,3,3',4'-biphenyltetracarboxylic dianhydride powder of the conventional technology.
The 2,3,3',4'-biphenyltetracarboxylic dianhydride powder prepared by the invention disclosed in Part C can be also used in the production of the polyimide precursors described in Parts A and B.

The invention disclosed in Part C will now be described in detail.
The method of purifying a 2,3,3',4'-biphenyltetracarboxylic dianhydride powder of the present invention disclosed in Part C is characterized by mixing a solvent in which the solubility of 2,3,3',4'-biphenyltetracarboxylic dianhydride at 25°C is 1 g/100 g or more and a 2,3,3',4'-biphenyltetracarboxylic dianhydride powder in an uneven state where at least a part of the 2,3,3',4'-biphenyltetracarboxylic dianhydride powder is not dissolved; and subsequently separating and collecting the undissolved 2,3,3',4'-biphenyltetracarboxylic dianhydride powder from the mixture.

The solvent used in the present invention is a solvent in which the solubility of 2,3,3',4'-biphenyltetracarboxylic dianhydride at 25°C is 1 g/100 g or more, preferably 3 g/100 g or more, and most preferably 7 g/100 g or more and preferably 100 g/100 g or less, more preferably 50 g/100 g or less, more preferably 30 g/100 g or less, and most preferably 20 g/100 g or less. A too low solubility makes it difficult to readily provide a 2,3,3',4'-biphenyltetracarboxylic dianhydride powder having reduced color. A too high solubility causes excess dissolution of the raw material to reduce the yield and is therefore uneconomic. The solvent is not necessarily a single one and a mixture of a plurality of solvents may be used, as long as the solubility of the powder in the mixture is 1 g/100 g or more.

The examples of the solvent used in the present invention include, but not limited to, aliphatic hydrocarbons such as n-hexane, cyclohexane, heptane and octane; aromatic hydrocarbons such as benzene, toluene and xylene; alcohols such as methanol, ethanol, butanol, isopropyl alcohol, n-propyl alcohol, butanol, tert-butanol, butanediol, ethyl hexanol, and benzyl alcohol; ketones such as acetone, methyl ethyl ketone, methyl isobutyl ketone, diisobutyl ketone and cyclohexanone; esters such as ethyl acetate, methyl acetate, butyl acetate, methoxybutyl acetate, cellosolve acetate, amyl acetate, n-propyl acetate, isopropyl acetate, methyl lactate, ethyl lactate, butyl lactate, γ-valerolactone, δ-valerolactone, γ-caprolactone, ε - caprolactone and α-methyl-γ-butyrolactone; ethers such as dimethyl ether, ethyl methyl ether, diethyl ether, furan, dibenzofuran, oxetane, tetrahydrofuran, tetrahydropyran, methyl cellosolve, cellosolve, butyl cellosolve, dioxane, methyl tertiary butyl ether, butyl carbitol, ethylene glycol, diethylene glycol, triethylene glycol, propylene glycol, diethyleneglycol monomethyl ether, triethylene glycol monomethyl ether, propylene glycol monomethyl ether, 3-methoxy-3-methyl-1-butanol, ethyleneglycol monomethyl ether acetate, propyleneglycol monomethyl ether acetate, diethyleneglycol monomethyl ether acetate, diethyleneglycol monoethyl ether acetate; nitriles such as acetonitrile, propionitrile and butyronitrile; amides such as N-methyl-2-pyrrolidone, N, N-dimethylformamide and N, N-dimethylacetamide; sulfones such as dimethyl sulfoxide; carbonates such as dimethyl carbonate and diethyl carbonate; phenols such as m-cresol, p-cresol, 3 - chlorophenol and 4-chlorophenol; and others, for examples, acetophenone, 1,3-dimethyl-2-imidazolidinone, sulfolane and water. A solvent in which the solubility of 2,3,3',4'-biphenyltetracarboxylic dianhydride is less than 1 g/100 g may be used in combination with a solvent in which the solubility thereof is 1 g/100 g or more such that the solubility in the resulting mixture is 1 g/100 g or more. When alcohols or water is used, they may react with an acid anhydride to cause a ring-opening reaction. Accordingly, it is preferable to conduct a heat treatment after purification. The heat treatment after purification can be avoided by using a high-purity solvent not containing water and alcohols.

Among these solvents, particularly preferred are acetone, methyl ethyl ketone, methyl isobutyl ketone, ethyl acetate, butyl acetate and tetrahydrofuran due to high purification efficiency and easiness of handling.

The solubility of 2,3,3',4'-biphenyltetracarboxylic dianhydride at 25°C is the amount (g) of 2,3,3',4'-biphenyltetracarboxylic dianhydride dissolved in 100 g of the solvent of interest at 25°C.
In the present invention, the solubility is measured by the following method.
That is, 10 g of a 2,3,3',4'-biphenyltetracarboxylic dianhydride powder having a purity of 99% or more and 20 g of a solvent of interest are mixed and are stirred at 25°C for 3 hours to give a mixture (confirm in advance this stirring condition provides a saturated state, and the amount of the powder is increased to twice, three times, ... when the saturation is not achieved). The 2,3,3',4'-biphenyltetracarboxylic dianhydride powder not dissolved in this mixture is removed by filtration with a filter paper 5A manufactured by Advantec, Inc. to yield a saturated solution of 2,3,3',4'-biphenyltetracarboxylic dianhydride as the filtrate. 5g of the saturated solution of 2,3,3',4'-biphenyltetracarboxylic dianhydride is weighed in a petri dish and is heated at 80°C for 1 hour and then at 200°C for 1 hour to remove the solvent. The mass of the 2,3,3',4'-biphenyltetracarboxylic dianhydride in the petri dish after the heating is measured, and the solubility at 25°C is calculated based on the mass value.

In the method of purification of the present invention, a solvent in which the solubility of 2,3,3',4'-biphenyltetracarboxylic dianhydride at 25°C is 1 g/100 g or more and a 2,3,3',4'-biphenyltetracarboxylic dianhydride powder are mixed in an uneven state where at least a part of the 2,3,3',4'-biphenyltetracarboxylic dianhydride powder is not dissolved. That is, the mixture prepared here is in an uneven mixture state where a part of the 2,3,3',4'-biphenyltetracarboxylic dianhydride powder is dissolved while the residual powder being undissolved, prepared by mixing the solvent and the 2,3,3',4'-biphenyltetracarboxylic dianhydride powder in an excess amount than the solubility. Accordingly, the mixture ratio between the solvent and the 2,3,3',4'-biphenyltetracarboxylic dianhydride powder is determined such that the amount of the 2,3,3',4'-biphenyltetracarboxylic dianhydride powder is higher than the solubility at the temperature (preferably 25°C) of the mixture. The amount of the powder is preferably about 2 to 100 times, more preferably about 2 to 50 times, and most preferably about 5 to 20 times the solubility. A too small amount of the powder increases the proportion of the powder dissolved and not collected and is therefore uneconomic. A too large amount of the powder may make the purification effect insufficient.

The temperature for handling the mixture is not particularly limited and is preferably about room temperature (about 0 to 50°C) because of its simplicity and economic efficiency. Low temperature or high temperature makes the process complicated and is uneconomic. When the solvent is water or contains water or has a functional group readily reacting with acid anhydrides, the mixture is preferably handled at a lower temperature for preventing the water or the functional group from reacting with acid anhydrides.

The 2,3,3',4'-biphenyltetracarboxylic dianhydride powder used here may be any known powder without limitation. For example, the powder may be produced by the method described in Patent Document 1 or 2 or may be produced by another known method. In order to be suitably used as a chemical raw material immediately after purification, preferred is the powder having a purity of 98% by mass or more, preferably 99% by mass or more, and more preferably 99.5% by mass or more. Although the particle diameter or particle shape is not particularly limited, powder with a particle diameter of 5 mm or less and preferably 1 mm or less is suitable. The degree of crystallinity is not particularly limited.

In the present invention, the mixture prepared by mixing a 2,3,3',4'-biphenyltetracarboxylic dianhydride powder with a solvent in an uneven state where a part of the 2,3,3',4'-biphenyltetracarboxylic dianhydride powder is dissolved while the residual powder being undissolved is preferably stirred with a mixer. The stirring time is not particularly limited as long as a sufficient purification effect is obtained. In the present invention, the solution portion of the mixture is not necessarily in a saturated state as long as coloring is reduced by dissolution of a part of the powder. The stirring time is usually about 0.5 to 6 hours.

After sufficient stirring of the mixture, the undissolved 2,3,3',4'-biphenyltetracarboxylic dianhydride powder in the mixture is separated and collected from the solvent. In this separation, the coloring-causing materials are separated together with the solvent, and thereby a 2,3,3',4'-biphenyltetracarboxylic dianhydride powder having reduced color can be suitably collected. In general, the separation step can be suitably performed by filtration. The separated 2,3,3',4'-biphenyltetracarboxylic dianhydride powder contains the solvent. Accordingly, if necessary, the powder is sufficiently dried by, for example, heating, air blow, or reduced pressure in an inert atmosphere. When the solvent is water or a water-containing solvent, a (significantly small) part of anhydride rings may be converted into dicarboxylic acid groups by hydrolysis during the purification step. In such a case, the solvent is preferably dried at high temperature (100°C or more, preferably 150°C or more) that readily causes dehydration, performing drying and dehydration simultaneously.

As shown in Comparative Example C3 below, it is difficult to prepare a 2,3,3',4'-biphenyltetracarboxylic dianhydride powder having reduced color by recrystallization. However, the method of purification of the present invention can readily prepare a less-colored 2,3,3',4'-biphenyltetracarboxylic dianhydride powder. The reason is assumed due to specific conditions that the 2,3,3',4'-biphenyltetracarboxylic dianhydride powder forms a special structure consisting of a crystalline portion and an amorphous portion and that the amorphous portion contains a larger amount of the coloring-causing materials, the coloring-causing materials are present on the crystal surfaces in a larger amount, and also the coloring-causing materials are readily dissolved in the solvent.

The 2,3,3',4'-biphenyltetracarboxylic dianhydride powder of the present invention is characterized by having reduced color and having high transparency having a light transmittance, at a wavelength of 400 nm, of 85% or more, preferably 90% or more, as a 10% by mass solution in a 2 N aqueous sodium hydroxide solution. The 2,3,3',4'-biphenyltetracarboxylic dianhydride having such a light transmittance can provide a polyimide having high transparency and is therefore significantly suitable as a tetracarboxylic acid component of a polyimide for high-performance optical material.

The polyimide of the present invention is characterized by being prepared using, as a tetracarboxylic acid component, 2,3,3',4'-biphenyltetracarboxylic dianhydride powder having a light transmittance, at a wavelength of 400 nm, of 85% or more, preferably 90% or more, as a 10% by mass solution in a 2 N aqueous sodium hydroxide solution and having an increased light transmittance when formed into a film. A film having a thickness of 10 µm preferably has a light transmittance at 400 nm of 70% or more.
The polyimide of the present invention can be suitably prepared using the 2,3,3',4'-biphenyltetracarboxylic dianhydride powder satisfying the above-mentioned requirements as at least a part of the tetracarboxylic acid component. The tetracarboxylic acid component may further contain a tetracarboxylic acid component, in addition to the 2,3,3',4'-biphenyltetracarboxylic dianhydride satisfying the above-mentioned requirements. Preferred examples of the optional tetracarboxylic acid component include, but are not limited to, pyromellitic dianhydride, 3,3',4,4'-biphenyltetracarboxylic dianhydride, benzophenonetetracarboxylic dianhydride, and oxydiphthalic dianhydride.

The diamine component is not particularly limited. The diamine component of the polyimide may be any known diamine component and is preferably selected from the group consisting of aliphatic diamines, diamines having alicyclic structures, and aromatic diamines having substituent(s) of any of halogen groups, carbonyl groups and sulfonyl groups (i.e., aromatic diamines having any of halogen groups, carbonyl groups, and sulfonyl groups as substituents) for increasing the transparency of the polyimide. The diamines here are diamines and diamine derivatives, such as diamine and diisocyanate, which are usually used as raw materials of polyimides. The diamine derivative may be one prepared by reacting a diamine with a silylating agent (such as an amide-based silylating agent) for increasing the reactivity or the solubility of the reaction product.

The examples of aliphatic diamines include linear or branched aliphatic amines and derivatives thereof such as diaminobutane, diaminopentane, diaminohexane, diaminoheptane, diaminooctane, diaminononane, diaminodecane, diaminoundecane and diaminododecane.

The examples of diamines having alicyclic structures include diamines having alicyclic structures and derivatives thereof such as 1,4-diaminocyclohexane, 1,3-diaminocyclohexane, 1,2-diaminocyclohexane, 3-methyl-1, 4-diaminocyclohexane, 3-methyl-, 3-aminomethyl-, 5,5-dimethylcyclohexylamine, 1,3-bisaminomethyl cyclohexane, bis (4,4'-aminocyclohexyl) methane, bis(3,3'-methyl-4, 4'-aminocyclohexyl) methane, bis(aminomethyl)norbornane, bis(aminomethyl)-tricyclo[5,2,1,0]decane, isophorone diamine and 1,3-diaminoadamantane.

The examples of aromatic diamines having substituent(s) of any of halogen groups, carbonyl groups and sulfonyl groups include aromatic diamines having halogen groups such as 3,5-diaminobenzotrifluoride, 2-(trifluoromethyl)-1,4-phenylenediamine, 5-(trifluoromethyl)-1,3-phenylenediamine, 1,3-diamino-2,4,5,6-tetrafluorobenzene, 2,2-bis[4-(4-aminophenoxy)phenyl]-hexafluoropropane, 2,2-bis(3-aminophenyl)-1,1,1,3 3,3 -hexafluoropropane, 2,2'-bis -(4-aminophenyl)-hexafluoropropane, 4,4-bis(trifluoromethoxy)benzidine, 3,3'-diamino-5, 5'-trifluoromethylbiphenyl, 3,3'-diamino-6, 6'-trifluoromethylbiphenyl, 3,3'-bis(trifluoromethyl)benzidine, 2,2-bis[4-(4-aminophenoxy) phenyl]hexafluoropropane, 4,4'-trifluoromethyl-2, 2'-diaminobiphenyl, 2,2'-bis(trifluoromethyl)-4,4'-diaminobiphenyl, 3,3-dichloro-4, 4'-diaminobiphenyl, 2,2',5,5'-dichloro-4, 4'-diaminobiphenyl, 4,4'-methylene- bis(2-chloroaniline) and derivatives thereof; aromatic diamines having carbonyl groups such as 4,4'-diamino benzophenone, 3,3'-diamino benzophenone, 4-aminophenyl-4 - aminobenzoate, bis (4-aminophenyl) terephthalate ester, bis-(4-aminophenyl) biphenyl-4, 4'-dicarboxylate, 1,4-bis(4-aminobenzoyloxy) benzene, 1,3-bis(4-aminobenzoyloxy)benzene, 4,4'-diamino benzanilide, N, N-bis(4-aminophenyl)terephthalamide, N, N'-p-phenylenebis(p-aminobenzamide) and N, N'-m-phenylenebis (p-aminobenzamide) and derivatives thereof; aromatic diamines having sulfonyl groups such as 3,3'-diaminodiphenyl sulfone, 3,4'-diaminodiphenyl sulfone, 4,4'-diaminodiphenyl sulfone, 3,3'-diamino-4,4'-dihydroxydiphenyl sulfone, o-tolidine sulfone, bis[4-(4-aminophenoxy)phenyl]sulfone, bis[4-(3-aminophenoxy)phenyl]sulfone and derivatives thereof.

Among these diamines, preference is given to 1,4-diaminocyclohexane, bis(4,4'-aminocyclohexyl)methane, 2,2'-bis(trifluoromethyl)-4,4'-diaminobiphenyl, 4,4'-diaminodiphenyl sulfone and derivatives thereof because produced polyimides therefrom are excellent in transparency and heat resistance, and particularly preferred is trans-1,4-diaminocyclohexane and derivatives thereof because produced polyimides therefrom are excellent in low coefficient of linear thermal expansion.

The polyimide of the present invention can be suitably prepared by a known method. The polyimide can be suitably prepared through a reaction of a tetracarboxylic acid component and a diamine component in a solvent at a relatively low temperature to generate a polyimide precursor, a polyamic acid, and subjecting the polyimide precursor to thermal imidization or chemical imidization with acetic anhydride and the like. Alternatively, the polyimide can be suitably prepared by reacting a tetracarboxylic acid component and a diamine component in a solvent at a relatively high temperature to directly generate the polyimide. In the fields of electronic parts and display devices, the polyimide can be suitably used, in particular, in a film form.

### << PART D >>

The invention disclosed in Part D relates to a method of purifying a 3,3',4,4'-biphenyltetracarboxylic dianhydride powder having reduced color and a polyimide prepared using the powder. Here, the 3,3',4,4'-biphenyltetracarboxylic dianhydride powder is a powder mainly composed of 3,3',4,4'-biphenyltetracarboxylic dianhydride and is suitably used as a chemical raw material substantially consisting of 3,3',4,4'-biphenyltetracarboxylic dianhydride.

As described in Background Art, the methods of production described in Japanese Patent Laid-Open Nos. 2005-314296 and 2006-45198 can prepare 3,3',4,4'-biphenyltetracarboxylic dianhydride having reduced color. However, these methods need huge equipment such as an apparatus for heating to melt and evaporating a material at high temperature in a specific oxygen concentration under reduced pressure or a specific heating apparatus having a specific structure for dehydration and thus have disadvantages in facilities cost. The methods also have a disadvantage of requiring complicated operations under strict operation conditions and therefore need improvement.
The method of production described in Japanese Patent Laid-Open No. 2004-196687 can prepare 3,3',4,4'-biphenyltetracarboxylic dianhydride containing reduced amount of alkali metals. However, the effect of reducing coloring by recrystallization from acetic anhydride is insufficient.

The invention disclosed in Part D was made as a result of various investigations for a method of purification that can readily prepare a 3,3',4,4'-biphenyltetracarboxylic dianhydride powder having reduced color with a simple operation under moderate conditions without requiring huge facilities.
That is, the purpose of the invention disclosed in Part D is to provide a method of readily purifying a 3,3',4,4'-biphenyltetracarboxylic dianhydride powder having reduced color by a simple operation under moderate condition without requiring huge facilities and to provide a polyimide having excellent transparency prepared using the 3,3',4,4'-biphenyltetracarboxylic dianhydride powder having reduced color prepared by this method.

The invention disclosed in Part D relates to the following items. 1. A method of purifying a 3,3',4,4'-biphenyltetracarboxylic dianhydride powder, comprising mixing a solvent in which the solubility of 3,3',4,4'-biphenyltetracarboxylic dianhydride at 25°C is 0.1 g/100 g or more and a 3,3',4,4'-biphenyltetracarboxylic dianhydride powder in an uneven state where at least a part of the 3,3',4,4'-biphenyltetracarboxylic dianhydride powder is not dissolved; and separating and collecting the undissolved 3,3',4,4'-biphenyltetracarboxylic dianhydride powder from the mixture.

2. The method of purification according to item 1, wherein the solubility of 3,3',4,4'-biphenyltetracarboxylic dianhydride in the solvent at 25°C is 1 g/100 g or more.

3. The method of purification according to item 1 or 2, wherein the solvent is N,N-dimethylformamide, N,N-dimethylacetamide, N-methyl-2-pyrrolidone, or N-ethyl-2-pyrrolidone.

4. The method of purification according to any one of items 1 to 3, wherein the separated and collected 3,3',4,4'-biphenyltetracarboxylic dianhydride powder has a light transmittance at a wavelength of 400 nm and an optical path length of 1 cm of 75% or more as a 10% by mass solution in a 2 N aqueous sodium hydroxide solution.

5. The method of purification according to any one of items 1 to 4, further including sublimation of the separated and collected 3,3',4,4'-biphenyltetracarboxylic dianhydride powder.

6. A polyimide having a light transmittance of 80% or more at 400 nm when formed into a film having a thickness of 10 µm in which the polyimide is formed from a tetracarboxylic acid component comprising the separated and collected 3,3',4,4'-biphenyltetracarboxylic dianhydride powder by the method of purification according to any one of items 1 to 5 and a diamine component comprising a diamine selected from the group consisting of aliphatic diamines, diamines having alicyclic structures, and aromatic diamines having substituent(s) of any of halogen groups, carbonyl groups and sulfonyl groups.

7. The polyimide according to item 6 which is used as an optical material.

8. A method of producing a polyimide comprising polymerizing and imidizing a tetracarboxylic acid component comprising the separated and collected 3,3',4,4'-biphenyltetracarboxylic dianhydride powder in the method of purification according to any one of items 1 to 5 and a diamine component comprising a diamine selected from the group consisting of aliphatic diamines, diamines having alicyclic structures, and aromatic diamines having substituent(s) of any of halogen groups, carbonyl groups and sulfonyl groups.

The invention disclosed in Part D can provide a method of readily purifying a 3,3',4,4'-biphenyltetracarboxylic dianhydride powder having reduced color by a simple operation under moderate conditions without requiring huge facilities. The use of the 3,3',4,4'-biphenyltetracarboxylic dianhydride powder having reduced color prepared by the method of purification of the present invention can provide a polyimide that can be suitably used as a high-performance optical material having excellent transparency, in particular, as a transparent base material of a display device such as a flexible display or touch panel.
The 3,3',4,4'-biphenyltetracarboxylic dianhydride powder prepared by the invention disclosed in Part D can provide an end product having higher transparency, in particular, a polyimide by using it in place of the 3,3',4,4'-biphenyltetracarboxylic dianhydride powder of the conventional technology.
The 3,3',4,4'-biphenyltetracarboxylic dianhydride powder prepared by the invention disclosed in Part D can be also preferably used in the production of the polyimide precursors described in Parts A and B.

The invention disclosed in Part D will now be described in detail.
In the description below, 3,3',4,4'-biphenyltetracarboxylic dianhydride may be abbreviated as s-BPDA, and a 3,3',4,4'-biphenyltetracarboxylic dianhydride powder may be abbreviated as s-BPDA powder.
The method of purifying a 3,3',4,4'-biphenyltetracarboxylic dianhydride powder of the present invention disclosed in Part D is characterized by mixing a solvent in which the solubility of s-BPDA at 25°C is 0.1 g/100 g or more and s-BPDA powder as a raw material in an uneven state where at least a part of the s-BPDA powder is not dissolved; and then separating and collecting the undissolved s-BPDA powder from the mixture.

Here, a solvent in which the solubility of s-BPDA at 25°C is 0.1 g/100 g or more means that 0.1 g or more of s-BPDA is dissolved in 100 g of the solvent of interest at 25°C.
In the present invention, the solubility of s-BPDA was measured by the following method.
That is, 5.0 g of s-BPDA powder having a purity of 99% or more and 50.0 g of a solvent of interest are mixed and are stirred at 25°C for 3 hours to give a mixture (confirm in advance this stirring condition provides a saturated state, and the amount of the powder is increased to twice, three times, ... when the saturation is not achieved). The s-BPDA powder not dissolved in this mixture is removed by filtration with a filter paper 5A manufactured by Advantec, Inc. to yield a saturated solution of s-BPDA as the filtrate. 5g of the saturated solution of s-BPDA is weighed in a petri dish and is heated at 80°C for 1 hour and then at 200°C for 1 hour to remove the solvent. The mass of the s-BPDA in the petri dish after the heating is measured, and the solubility at 25°C is calculated based on the mass value.

The solubility of s-BPDA at 25°C in the solvent that is suitably used in the method of purification of the present invention is 0.1 g/100 g or more, preferably 1.0 g/100 g or more, and more preferably 2.0 g/100 g or more and preferably 100.0 g/100 g or less, and more preferably 30.0 g/100 g or less. A low solubility makes it difficult to provide s-BPDA powder having reduced color. High solubility allows preparation of s-BPDA powder having reduced color, but causes excess dissolution of the raw material to reduce the yield and is therefore uneconomic. The solvent is not necessarily a single one and a mixture of a plurality of solvents may be used, as long as the solubility of the powder in the mixture is 0.1 g/100 g or more.

The solvent used in the present invention is not particularly limited, and examples of the solvent include those exemplified in Part C as solvents used for purifying 2,3,3',4'-biphenyltetracarboxylic dianhydride. In particular, dimethyl sulfoxide, N,N-dimethylformamide, N,N-dimethylacetamide, N-methyl-2-pyrrolidone, and N-ethyl-2-pyrrolidone are preferred. A solvent in which the solubility of s-BPDA is less than 0.1 g/100 g may be used in combination with a solvent in which the solubility thereof is 0.1 g/100 g or more such that the solubility in the resulting mixture is 0.1 g/100 g or more. When alcohols or water is used, they may react with an acid anhydride to cause a ring-opening reaction. Accordingly, it is preferable to conduct heat treatment after purification. The heat treatment after purification can be avoided by using a high-purity solvent not containing water and alcohols.

In the present invention, s-BPDA powder and a solvent having an appropriate solubility are mixed in an uneven state where at least a part of the s-BPDA powder is not dissolved. Thus, coloring-causing materials in the s-BPDA powder are selectively dissolved in the solvent, and the undissolved s-BPDA powder having reduced color is separated and collected, and thus s-BPDA powder having reduced color is easily obtained at a high yield.

That is, the resulting mixture here is prepared by mixing a solvent and s-BPDA powder in an excess amount than the solubility and is in an uneven mixture state where a part of the powder is dissolved while the residual powder is undissolved. Accordingly, the mixture ratio between the solvent and the s-BPDA powder is determined such that the amount of the s-BPDA powder is higher than the solubility at the temperature (preferably 25°C) of the mixture. The amount of the powder is preferably about 2 to 5000 times, more preferably about 5 to 2000 times, more preferably about 5 to 200 times, and most preferably 5 to 100 times the solubility. A too small amount of the powder increases the proportion of the powder dissolved and not collected and is therefore uneconomic. A too large amount of the powder may make the purification effect insufficient.

In the method of purification of the present invention, the temperature of mixing a solvent and s-BPDA powder is preferably a relatively low temperature, lower than the boiling point of the solvent. Specifically, the temperature is 150°C or less, preferably 100°C or less, more preferably less than 70°C, and most preferably 0 to 50°C. In particular, it is preferable to perform purification at a room temperature of 0 to 50°C without heating. High temperature by heating or reflux may cause coloring by a reaction, decomposition, or oxidative degradation of the solvent. In addition, high temperature may cause coloring of the s-BPDA powder itself by oxidation and the like.

The mixture is preferably stirred with a mixer. The stirring time is not particularly limited as long as a sufficient purification effect is obtained. In the present invention, the solution portion of the mixture is not necessarily in a saturated state as long as coloring is reduced by dissolution of a part of the powder in the solvent. The stirring time is usually about 0.5 to 6 hours.

The s-BPDA powder used as the raw material in the method of purification of the present invention is not particularly limited, and a known powder can be suitably used. For example, the powder may be produced by the method described in Patent Document 1 or 2 or may be produced by another known method. In order to be suitably used as a chemical raw material immediately after purification, preferred is the powder having a purity of 98% by mass or more, preferably 99% by mass or more, and more preferably 99.5% by mass or more. The powder may also have any particle diameter and any particle shape and usually has a particle diameter of 5 mm or less and preferably 1 mm or less. The crystallizability (degree of crystallinity) of the powder is not particularly limited.

In the method of purification of the present invention, after sufficient stirring of the mixture, the undissolved s-BPDA powder in the mixture is preferably separated from the solvent and is collected. The coloring-causing materials are separated together with the solvent, and thereby s-BPDA powder having reduced color can be suitably collected. The undissolved s-BPDA powder can be suitably separated and collected from the mixture by a known method such as atmospheric pressure filtration, pressure filtration, suction filtration, or centrifugal filtration. The separation step is preferably performed at approximately the same temperature as that during mixing and stirring the mixture. If the temperature of the separation step is lower than that during mixing and stirring the mixture, the coloring-causing materials dissolved once in the solvent may precipitate again to color the s-BPDA powder.
The solvent adheres to the separated and collected s-BPDA powder and remains therein. Accordingly, the collected s-BPDA powder is preferably sufficiently dried by a known method such as hot-air drying, heat drying, or vacuum drying preferably in an inert atmosphere. In the purification step, a part of acid anhydrides may cause a ring-opening reaction. In such a case, cyclization is preferably performed in the drying step by heating and the like.

In the method of purification of the present invention, it is preferable to further sublimate the s-BPDA that has been prepared by mixing a solvent in which the solubility of s-BPDA at 25°C is 0.1 g/100 g or more and s-BPDA powder in an uneven state where at least a part of the s-BPDA powder is not dissolved and subsequently separating and collecting the undissolved powder from the mixture.
The sublimation is not required to be performed under specific conditions and can be suitably performed under known conditions. As disclosed in Japanese Patent Laid-Open Nos. 2005-314296 and 2006-45198, the s-BPDA powder may be melted by heating and evaporated under reduced pressure at high temperature of 250°C or more, and the vapor may be cooled for crystallization. Alternatively, s-BPDA crystals being further less colored can be suitably prepared by sublimating the s-BPDA powder at a relatively low temperature of about 100 to 250°C without melting by heating. Even if the s-BPDA crystals are aggregated, a powder can be readily formed by pulverization.

According to the method of purification of the present invention, a 3,3',4,4'-biphenyltetracarboxylic dianhydride powder having reduced color can be readily prepared without requiring huge facilities by a simple operation under moderate conditions. The resulting s-BPDA powder has a light transmittance, at a wavelength of 400 nm, of higher than 75%, preferably 80% or more, as a 10% by mass solution in a 2 N aqueous sodium hydroxide solution.

A polyimide that can be suitably used as a high-performance optical material having excellent transparency can be readily prepared using the s-BPDA powder prepared by the method of purification of the present invention.

Accordingly, the present invention relates to the following polyimide and a method of producing a polyimide. That is, the polyimide of the present invention has a light transmittance of 70% or more at 400 nm when formed into a film having a thickness of 10 µm in which the polyimide is formed from a tetracarboxylic acid component comprising the 3,3',4,4'-biphenyltetracarboxylic dianhydride powder separated and collected by the method of purification of the present invention and a diamine component comprising a diamine selected from the group consisting of aliphatic diamines, diamines having alicyclic structures, and aromatic diamines having substituent(s) of any of halogen groups, carbonyl groups and sulfonyl groups.

In the polyimide of the present invention, the tetracarboxylic acid component may include a tetracarboxylic acid component other than the s-BPDA powder of the present invention in an amount of 50 % or less, preferably 25 % or less, more preferably 10 % or less based on the total 3, 3', 4' of tetracarboxylic acid component. Use of the tetracarboxylic acid component other than the s-BPDA powder of the present invention may improve the solubility of the polyimide precursor leading to easiness of the production.
The tetracarboxylic acid component other than the s-BPDA powder of the present invention is not particularly limited and may be any tetracarboxylic acid component generally employed as a raw material for a polyimide, but an aromatic tetracarboxylic dianhydride is preferred. The examples of such tetracarboxylic dianhydride include 2, 2', 3, 3'-biphenyltetracarboxylic dianhydride, 2, 3, 3', 4'- biphenyltetracarboxylic dianhydride, pyromellitic dianhydride, oxydiphthalic dianhydride, 3,3', 4,4' - benzophenone tetracarboxylic dianhydride, 3, 3 ', 4,4' - diphenylsulfone tetracarboxylic dianhydride, m-terphenyl-3, 3 ', 4,4'-tetracarboxylic dianhydride, 4,4'-(2,2 hexafluoroisopropylene)diphthalic dianhydride, 2,2'-bis(3,4-dicarboxyphenyl)propane dianhydride, 1,4,5,8 - naphthalenetetracarboxylic dianhydride, 2,3,6,7 - naphthalene tetracarboxylic dianhydride, (1,1':3',1"- terphenyl) -3, 3", 4, 4"-tetracarboxylic dianhydride, 4,4'- (dimethyl-siladiyl)diphthalic dianhydride, 4,4' - (1,4 - phenylenebis(oxy))diphthalic dianhydride and the like, and more preferably 2, 2', 3, 3' - biphenyltetracarboxylic dianhydride and 2, 3, 3', 4'-biphenyltetracarboxylic dianhydride.

For a diamine component, the diamines explained in Part C can be used.

As same as the description in Part C, among these diamines, preference is given to 1,4-diaminocyclohexane, bis(4,4'-aminocyclohexyl)methane, 2,2'-bis(trifluoromethyl)-4,4'-diaminobiphenyl, 4,4'-diaminodiphenyl sulfone and derivatives thereof because produced polyimides therefrom are excellent in transparency and heat resistance, and particularly preferred is trans-1,4-diaminocyclohexane and derivatives thereof because produced polyimides therefrom are excellent in low coefficient of linear thermal expansion.

The polyimide of the present invention is characterized in that it has a light transmittance of 80% or more at 400 nm when formed into a film having a thickness of 10 µm. Accordingly, it is advantageously used for an optical material.

The method of producing a polyimide is characterized in that it comprises polymerizing and imidizing a tetracarboxylic acid component comprising the separated and collected 3,3',4,4'-biphenyltetracarboxylic dianhydride powder in the method of purification according to the present invention and a diamine component comprising a diamine selected from the group consisting of aliphatic diamines, diamines having alicyclic structures, and aromatic diamines having substituent(s) of any of halogen groups, carbonyl groups and sulfonyl groups.
The method and condition of the polymerization and imidization is not particularly limited and a method and a condition generally employed for conventional methods of producing a polyimide may be used, but products will be more easily produced by the method through the polyimide precursor as described below.

### 1) Polyamic acid

A polyimide precursor is prepared by dissolving a diamine in an organic solvent, gradually adding a tetracarboxylic dianhydride to the resulting solution with stirring, and stirring the mixture in a temperature range of 0 to 100°C for 1 to 72 hours.

### 2) Polyamic acid silyl ester

A silylated diamine is prepared in advance by reacting a diamine and a silylating agent. The silylated diamine is optionally purified by, for example, distillation. The silylated diamine is dissolved in a dehydrated solvent, and a tetracarboxylic dianhydride is gradually added thereto with stirring, followed by stirring in a temperature range of 0 to 100°C for 1 to 72 hours to prepare a polyimide precursor. The use of a chlorine-free silylating agent does not require the purification of silylated diamine and is therefore preferable. Examples of the silylating agent not containing chlorine atoms include N,O-bis(trimethylsilyl)trifluoroacetamide, N,O-bis(trimethylsilyl)acetamide, and hexamethyldisilazane. Furthermore, N,O-bis(trimethylsilyl)acetamide and hexamethyldisilazane are preferable because they do not contain fluorine atoms and inexpensive. In order to facilitate the silylation of the diamine, an amine catalyst such as pyridine, piperidine, or triethylamine may be used. The catalyst can be also used as the polymerization catalyst of the polyimide precursor as it is.

Each of the methods of production described above can be suitably performed in an organic solvent, and as a result, a polyimide precursor solution composition can be readily prepared.

In these methods of production, the molar ratio of the tetracarboxylic acid component to the diamine component can be appropriately determined based on the viscosity of a target polyimide precursor and is preferably 0.90 to 1.10 and more preferably 0.95 to 1.05.

Specifically, the organic solvent used in the method of production is preferably an aprotic solvent such as N,N-dimethylformamide, N,N-dimethylacetamide, N-methyl-2-pyrrolidone, or dimethyl sulfoxide, but the structure is not particularly limited because any solvent may be used without problem as long as the solvent can dissolve the raw material monomers and the generated polyimide precursor. Examples of the usable organic solvent include those exemplified as the "organic solvent used in the method of production" in Part A.

In the present invention, the polyimide precursor solution composition may optionally contain a chemical imidization agent (an acid anhydride such as acetic anhydride or an amine compound such as pyridine or isoquinoline), an antioxidant, a filler, a dye, an inorganic pigment, a silane coupling agent, a fire-retarding material, an antifoaming agent, a leveling agent, a rheology-controlling agent (flow assistant), a release agent, etc.

The polyimide of the present invention can be produced through a cyclodehydration reaction (imidization reaction) of the polyimide precursor. The process of imidization is not particularly limited, and a known thermal imidization or chemical imidization can be suitably employed. Preferred examples of the form of the resulting polyimide include films, polyimide laminates, powders, beads, molded products, foamed products, and varnishes.

The polyimide precursor can be used for producing a polyimide/substrate laminate or a polyimide film. Examples of the method of production are as described in Part A, and the polyimide/base material laminate or the polyimide film can be produced as in Part A, and also a flexible conductive substrate can be produced as in Part A.

The polyimide film or the polyimide/substrate laminate can be suitably used, after a ceramic thin film, a metal thin film or the like is formed on the polyimide surface, as a substrate such as a transparent base material for displays, a transparent base material for touch panels, or a transparent substrate for solar cells for which transparency as a material is required.

### « PART E »

The invention disclosed in Part E relates to a trans-1,4-diaminocyclohexane powder having reduced color and a polyimide prepared using it as the diamine component. Here, the trans-1,4-diaminocyclohexane powder is a powder mainly composed of trans-1,4-diaminocyclohexane and is suitably used as a chemical raw material substantially consisting of trans-1,4-diaminocyclohexane.

The invention disclosed in Part E was made as a result of various investigations for reducing the coloring of the trans-1,4-diaminocyclohexane powder, with the aim of developing its use in a high-performance optical material for which polyimides have not been sufficiently investigated.
That is, it is an object of the invention disclosed in Part E to propose a trans-1,4-diaminocyclohexane powder having reduced color and a polyimide having reduced color prepared using it as a diamine component.

The invention disclosed in Part E relates to the following items.
1. A trans-1,4-diaminocyclohexane powder having a light transmittance of 90% or more at a wavelength of 400 nm and an optical path length of 1 cm as a 10% by mass solution in pure water.
2. The trans-1,4-diaminocyclohexane powder according to item 1, wherein the light transmittance at a wavelength of 400 nm and an optical path length of 1 cm is 95% or more.
3. A polyimide prepared using the trans-1,4-diaminocyclohexane powder according to item 1 or 2 as the diamine component and having a light transmittance of 80% or more at 400 nm when formed into a film having a thickness of 10 µm.
4. A polyimide according to item 3 to be used as an optical material.

The invention disclosed in Part E can propose a trans-1,4-diaminocyclohexane powder reduced in coloring and a polyimide reduced in coloring prepared using it as the diamine component. The polyimide prepared using the trans-1,4-diaminocyclohexane powder having reduced color of the present invention has a light transmittance of 80% or more at 400 nm and can be suitably used as an optical material.
The trans-1,4-diaminocyclohexane powder prepared by the invention disclosed in Part E can provide an end product having higher transparency, in particular, a polyimide by using it in place of the trans-1,4-diaminocyclohexane powder of the conventional technology.
The trans-1,4-diaminocyclohexane powder prepared by the invention disclosed in Part E can be also preferably used in the production of the polyimide precursors described in Parts A and B.

The invention disclosed in Part E will now be described in detail.
The trans-1,4-diaminocyclohexane powder (hereinafter, trans-1,4-diaminocyclohexane may be abbreviated as t-DACH, and the trans-1,4-diaminocyclohexane powder may be abbreviated as the t-DACH powder) of the invention disclosed in Part E has a light transmittance, at a wavelength of 400 nm, of 90% or more and more preferably 95% or more as a 10% by mass solution in pure water. If the light transmittance is less than 90%, the powder looks light yellow and cannot achieve the purpose of the present invention.

In the present invention, method of synthesis of crude t-DACH as a raw material may be any method and preference is given to a method of hydrogenating the nitro group and the benzene ring of p-nitroaniline to reduce into 1,4-diaminocyclohexane (U.S. Patent No. 2606925 (Patent Document 9)) or a method of hydrogenating p-phenylenediamine (U.S. Patent No. 3636108 and Japanese Patent Laid-Open No. 2008-74754 (Patent Documents 10 and 11)). In general, a (crude) t-DACH powder having a purity of 95% or more and preferably 99% or more, which can be used in the conventional production of polyimide, can be prepared by the above-mentioned production methods.

The t-DACH powder having reduced color of the present invention can be suitably prepared by (1) purification method of subliming the (crude) t-DACH powder or (2) purification method of treating it with an adsorbent. These purification methods may be performed alone or may be repeated or may be performed in combination. The (crude) t-DACH used in such purification preferably has a purity of 90% or more and more preferably 95% or more. A purity of less than 90% may not sufficiently remove the coloring in the purification process.

(1) The purification method by sublimation is not particularly limited, but a t-DACH powder (crystal) having reduced color is prepared by heating raw material t-DACH in an inert gas at atmospheric or reduced pressure for sublimation, allowing the sublimate to adhere to a cooled wall surface, and optionally pulverizing the resulting powder. As for the sublimation conditions, pressure employed is atmospheric pressure or less than atmospheric pressure, preferably 50 Torr or less, and more preferably 1 Torr or less, and temperature under reduced pressure is 20 to 150°C and preferably 50 to 100°C, and temperature under atmospheric pressure is 120 to 200°C and preferably 150 to 180°C.

(2) The purification method by treatment with an adsorbent can be performed, for example, by dissolving the (crude) t-DACH powder in a solvent and bringing the solution into contact with the adsorbent or by heating the (crude) t-DACH powder and bringing the fused powder into contact with the adsorbent. As the adsorbent, for example, activated carbon, graphite carbon black, activated clay, diatomaceous earth, activated alumina, silica gel, a molecular sieve, a carbon molecular sieve, a synthetic adsorbent, a basic anion exchange resin, or a chelate resin can be suitably used. The amount of the adsorbent used is 0.001 to 0.5 times, preferably 0.005 to 0.1 times, based on the mass of t-DACH. The conditions are not particularly limited and are preferably a temperature of 150°C or less and preferably 100°C or less, a treatment time of 5 min to 2 hours and preferably 30 min to 1 hour, and under an inert gas atmosphere.

In the method of bringing a solution of a (crude) t-DACH powder dissolved in a solvent into contact with an adsorbent, the solvent may be distilled away when the t-DACH powder is collected from the solution after the treatment with the adsorbent, but the t-DACH is preferably precipitated and recrystallized. Any solvent that can dissolve the t-DACH can be used without limitation, and examples thereof include aliphatic hydrocarbon solvents, aromatic hydrocarbon solvents, alcohol solvents, ketone solvents, ester solvents, ether solvents, nitrile solvents, amide solvents, sulfone solvents, carbonate solvents, phenol solvents, and water. In particular, aliphatic hydrocarbon solvents such as n-hexane, cyclohexane and n-heptane are suitable for the subsequent recrystallization and are therefore preferable.

A polyimide having reduced color of the present invention can be suitably prepared using a trans-1,4-diaminocyclohexane powder having reduced color having a light transmittance, at 400 nm, of 90% or more, preferably 95% or more, as the diamine component.

As the diamine component, a diamine other than t-DACH may be used together with the t-DACH. The diamine component other than t-DACH is not particularly limited and may be any diamine that is generally used for polyimides. In order to increase the transparency of polyimide, the diamines (excluding t-DACH) described in Part C can be suitably used. These diamines can be optionally used as a diamine component in addition to t-DACH.

The diamine component used for the polyimide of the present invention may be suitably used as a diamine derivative obtained by reaction with a silylating agent (such as an amide-based silylating agent) for increasing the reactivity or the solubility of the reaction product.

The tetracarboxylic acid component for the polyimide of the present invention is not particularly limited and may be any tetracarboxylic acid component generally employed as a raw material for a polyimide, but an aromatic tetracarboxylic dianhydride and alicyclic tetracarboxylic dianhydride are preferred.
The examples of the aromatic tetracarboxylic dianhydride include 3, 3', 4, 4' - biphenyltetracarboxylic dianhydride, 2, 2', 3, 3' - biphenyltetracarboxylic dianhydride, 2, 3', 3, 4' - biphenyltetracarboxylic dianhydride, pyromellitic dianhydride, oxydiphthalic dianhydride, 3,3', 4,4' - benzophenone tetracarboxylic dianhydride, 3, 3', 4,4' - diphenylsulfone tetracarboxylic dianhydride, m-terphenyl-3, 3', 4,4' - tetracarboxylic dianhydride, 4,4'-(2,2 hexafluoroisopropylene)diphthalic dianhydride, 2,2'-bis(3,4-dicarboxyphenyl) propane dianhydride, 1,4,5,8 - naphthalene tetracarboxylic dianhydride, 2,3,6,7 - naphthalenetetracarboxylic dianhydride, (1,1':3',1"-terphenyl)-3, 3", 4, 4" - tetracarboxylic dianhydride, 4,4'- (dimethylsiladiyl)diphthalic dianhydride, 4,4' - (1,4-phenylenebis(oxy))diphthalic dianhydride and the like. The examples of the alicyclic tetracarboxylic dianhydride include bicyclo[2.2.2]oct-7-ene-2,3,5,6-tetracarboxylic dianhydride, bicyclo[2.2.2]octane-2,3,5,6-tetracarboxylic dianhydride, 5-(dioxotetrahydrofuryl-3-methyl)-3-cyclohexene-1,2-dicarboxylic anhydride, 4-(2,5-dioxotetrahydrofuran-3-yl)-tetralin-1,2-dicarboxylic anhydride, tetrahydrofuran-2,3,4,5-tetracarboxylic dianhydride, bicyclo-3,3',4,4'-tetracarboxylic dianhydride, 3c-carboxymethylcyclopentane-1r,2c,4c-tricarboxylic-1,4,2,3-dianhydride, 1,2,4,5-cyclohexanetetracarboxylic dianhydride, 1,2,3,4-cyclobutanetetracarboxylic dianhydride, 1,2,3,4-cyclopentanetetracarboxylic dianhydride, and the like. In particular, biphenyltetracarboxylic dianhydrides are preferable because they give a polyimide excellent in mechanical properties and heat resistance.

The polyimide of the present invention can be suitably prepared by polymerization imidization of a tetracarboxylic acid component and a trans-1,4-diaminocyclohexane powder having reduced color and having a light transmittance at 400 nm of 90% or more and preferably 95% or more.
The method and conditions for the polymerization imidization are not particularly limited, and the method and conditions for polymerization imidization employed in the conventional method of producing polyimides can be suitably employed, but the polyimide can be readily produced by the method of producing a polyimide precursor described in Part D, i.e., a method through 1) a polyamic acid or 2) a polyamic acid silyl ester.

Each of the methods of production described above can be suitably performed in an organic solvent, and as a result, a polyimide precursor solution composition can be readily prepared.

In these methods of production, the molar ratio of the tetracarboxylic acid component to the diamine component can be appropriately determined based on the viscosity of a target polyimide precursor and is preferably 0.90 to 1.10 and more preferably 0.95 to 1.05.

Specifically, the organic solvent used in the method of production is preferably an aprotic solvent such as N,N-dimethylformamide, N,N-dimethylacetamide, N-methyl-2-pyrrolidone, or dimethyl sulfoxide, but the structure is not particularly limited because any solvent may be used without problem as long as the solvent can dissolve the raw material monomers and the generated polyimide precursor. Examples of the usable organic solvent include those exemplified as the "organic solvent used in the method of production" in Part A.

In the present invention, the polyimide precursor solution composition may optionally contain a chemical imidization agent (an acid anhydride such as acetic anhydride or an amine compound such as pyridine or isoquinoline), an antioxidant, a filler, a dye, an inorganic pigment, a silane coupling agent, a fire-retarding material, an antifoaming agent, a leveling agent, a rheology-controlling agent (flow assistant), a release agent, etc.

The polyimide of the present invention can be produced through a cyclodehydration reaction (imidization reaction) of the polyimide precursor. The process of imidization is not particularly limited, and a known thermal imidization or chemical imidization can be suitably employed. Preferred examples of the form of the resulting polyimide include films, polyimide laminates, powders, beads, molded products, foamed products, and varnishes.

The polyimide precursor can be used for producing a polyimide/substrate laminate or a polyimide film. Examples of the method of production are as described in Part A, and the polyimide/base material laminate or the polyimide film can be produced as in Part A, and also a flexible conductive substrate can be produced as in Part A.

After forming, for example, a ceramic thin film, a metal thin film or the like on the surface of polyimide, the polyimide film or the polyimide/substrate laminate can be suitably used as an optical material such as a transparent base material for displays, a transparent base material for touch panels, or a transparent substrate for solar cells for which transparency as a material is required.

### << PART F >>

The invention disclosed in Part F relates to a method of purifying a 2,2',3,3'-biphenyltetracarboxylic dianhydride powder having reduced color, the powder, and a polyimide prepared using the powder. Here, the 2,2',3,3'-biphenyltetracarboxylic dianhydride powder is a powder mainly composed of 2,2',3,3'-biphenyltetracarboxylic dianhydride and is preferably used as a chemical raw material substantially consisting of 2,2',3,3'-biphenyltetracarboxylic dianhydride.

As described in Background Art, Japanese Patent Laid-Open No. 2000-281616 discloses a method of producing 2,2',3,3'-biphenyltetracarboxylic acid, but does not describe any production of 2,2',3,3'-biphenyltetracarboxylic dianhydride. In addition, it is described that a polyimide resin prepared from 2,2',3,3'-biphenyltetracarboxylic dianhydride and 4,4'-oxydianiline is less colored compared to known polyimide resins. This effect is caused by the difference between the molecular structure of known polyimides and the molecular structure derived from 2,2',3,3'-biphenyltetracarboxylic dianhydride, and any effect by a reduction in coloring of 2,2',3,3'-biphenyltetracarboxylic dianhydride is not described at all.
Japanese Patent Laid-Open No. 2009-79009 describes a method of preparing 2,2',3,3'-biphenyltetracarboxylic dianhydride by acetic anhydride or heating, but does not describe any method of purifying 2,2',3,3'-biphenyltetracarboxylic dianhydride and coloring thereof.

The invention disclosed in Part F was made as a result of various investigations for reducing the coloring of the 2,2',3,3'-biphenyltetracarboxylic dianhydride powder, with the aim of developing its use in a high-performance optical material which exceeds the conventional application of polyimides.
That is, the purpose of the invention disclosed in Part F is to provide a method of readily purifying a 2,2',3,3'-biphenyltetracarboxylic dianhydride powder having reduced color by a simple procedure, a 2,2',3,3'-biphenyltetracarboxylic dianhydride powder having reduced color and a polyimide having an increased light transmittance using it.

The invention disclosed in Part F relates to the following items.

1. A 2,2',3,3'-biphenyltetracarboxylic dianhydride powder having a light transmittance of 80% or more at a wavelength of 400 nm and an optical path length of 1 cm as a 10% by mass solution in a 2 N aqueous sodium hydroxide solution as a solvent.

2. The 2,2',3,3'-biphenyltetracarboxylic dianhydride powder according to item 1, wherein the light transmittance at a wavelength of 400 nm and an optical path length of 1 cm is 90% or more.

3. A method of purifying a 2,2',3,3'-biphenyltetracarboxylic dianhydride powder by mixing a solvent and a 2,2',3,3'-biphenyltetracarboxylic dianhydride powder in an uneven state where at least a part of the 2,2',3,3'-biphenyltetracarboxylic dianhydride powder is not dissolved and subsequently separating and collecting the undissolved 2,2',3,3'-biphenyltetracarboxylic dianhydride powder from the mixture.

4. The method of purifying a 2,2',3,3'-biphenyltetracarboxylic dianhydride powder according to item 3, wherein the solvent comprises at least any one of alcohol solvents, ketone solvents, ester solvents, ether solvents, nitrile solvents, amide solvents, sulfone solvents, carbonate solvents, phenol solvents, and water.

5. The method of purifying a 2,2',3,3'-biphenyltetracarboxylic dianhydride powder according to item 3 or 4, wherein the solvent used is one in which the solubility of 2,2',3,3'-biphenyltetracarboxylic dianhydride at 25°C is 0.5 g/100 g or more.

6. The method of purifying a 2,2',3,3'-biphenyltetracarboxylic dianhydride powder according to item 3 or 4, wherein the solvent used is one in which the solubility of 2,2',3,3'-biphenyltetracarboxylic dianhydride at 25°C is from 3 g/100 g to 20 g/100 g.

7. The method of purifying a 2,2',3,3'-biphenyltetracarboxylic dianhydride powder according to any one of items 3 to 6, wherein the solvent is dimethyl sulfoxide, N,N-dimethylformamide, N,N-dimethylacetamide, N-methyl-2-pyrrolidone, or N-ethyl-2-pyrrolidone.

8. The method of purifying a 2,2',3,3'-biphenyltetracarboxylic dianhydride powder comprising recrystallizing a powder containing 2,2',3,3'-biphenyltetracarboxylic dianhydride from a solution containing an acid anhydride.

9. A method of purifying a 2,2',3,3'-biphenyltetracarboxylic dianhydride powder comprising heating a powder containing 2,2',3,3'-biphenyltetracarboxylic dianhydride at 150 to 350°C under a reduced pressure of 50 Torr or less for sublimation.

10. A polyimide produced using the 2,2',3,3'-biphenyltetracarboxylic dianhydride powder according to item 1 or 2, having an improved light transmittance when formed into a film.

11. The polyimide according to item 10 having a light transmittance of 80% or more at 400 nm when formed into a film having a thickness of 10 µm.

The invention disclosed in Part F can provide a method of readily purifying a 2,2',3,3'-biphenyltetracarboxylic dianhydride powder having reduced color by a simple operation, a 2,2',3,3'-biphenyltetracarboxylic dianhydride powder having reduced color, and a polyimide having increased light transmittance prepared using the 2,2',3,3'-biphenyltetracarboxylic dianhydride powder.
The 2,2',3,3'-biphenyltetracarboxylic dianhydride powder of the invention disclosed in Part F can provide an end product having higher transparency, in particular, a polyimide by using it in place of the 2,2',3,3'-biphenyltetracarboxylic dianhydride powder of the conventional technology.
The 2,2',3,3'-biphenyltetracarboxylic dianhydride powder prepared by the invention disclosed in Part F can be also preferably used in the production of the polyimide precursors described in Parts A and B.

The 2,2',3,3'-biphenyltetracarboxylic dianhydride powder (hereinafter, 2,2',3,3'-biphenyltetracarboxylic dianhydride may be abbreviated as i-BPDA, and the 2,2',3,3'-biphenyltetracarboxylic dianhydride powder is abbreviated as the i-BPDA powder) of the invention disclosed in Part F is characterized by having a light transmittance, at a wavelength of 400 nm and an optical path length of 1 cm, of 80% or more as a 10% by mass solution in a 2 N aqueous sodium hydroxide solution of 1 cm. If the light transmittance is less than 80%, the powder looks light yellow and cannot achieve the purpose of the present invention. The light transmittance is preferably 90% or more.

The i-BPDA may be synthesized by any method by preparing 2,2',3,3'-biphenyltetracarboxylic acid as an intermediate and dehydrating it.
The 2,2',3,3'-biphenyltetracarboxylic acid is suitably synthesized by a) a method of production described in Journal of Chemical Society, 1914, vol. 105, p. 2471, a so-called Ullmann reaction, through a coupling reaction by heating to high temperature in the presence of a copper powder, b) a method of production described in Patent Document 1 using a dialkylbenzenemononitro compound as a raw material and sequentially performing reduction, benzidine rearrangement, deamination, and oxidation, or c) a method of production described in Patent Document 2 using 2-dimethyl-3-chlorobenzene as a raw material and sequentially performing coupling and oxidation.

Dehydration of 2,2',3,3'-biphenyltetracarboxylic acid to synthesize i-BPDA can be suitably performed by any known method, for example, dehydration by addition of an acid anhydride such as acetic anhydride, dehydration through overheating by addition of a solvent that forms azeotrope with water, or dehydration by heating under an inert gas or reduced pressure. Such a method can generally provide i-BPDA powder having a purity of 90% or more, preferably 95% or more, which can be used in known production of polyimide.

In order to reduce coloring, the method of producing i-BPDA powder of the present invention preferably includes any one of the following purification steps:
(1) purification method of mixing a solvent and i-BPDA powder in an uneven state where at least a part of the i-BPDA powder is not dissolved and subsequently separating and collecting the undissolved i-BPDA powder from the mixture;
(2) purification method of recrystallizing from a solution containing an acid anhydride; and
(3) purification method of subliming by heating under reduced pressure. These purification steps may be repeated multiple times or may be employed in combination. The purity of the i-BPDA before purification is 90% or more, preferably 95% or more, and most preferably 98% or more. If the purity is less than 90%, the coloring may not be sufficiently removed by these purification steps.

In the purification method (1) of the present invention, a solvent in which the solubility of i-BPDA at 25°C is 0.5 g/100 g or more is mixed with the i-BPDA powder in an uneven state where at least a part of the i-BPDA powder is not dissolved, and then the undissolved i-BPDA powder is separated and collected from the mixture. Here, the solvent in which the solubility of i-BPDA at 25°C is 0.5 g/100 g or more means that 100 g of the solvent can dissolve 0.5 g or more of i-BPDA at 25°C. The solubility of i-BPDA of the present invention can be determined by the method described in Example below.

The solubility of i-BPDA at 25°C in the solvent used in the method (1) of purification of the present invention is 0.5 g/100 g or more, preferably 3 g/100 g to 20 g/100 g. The use of a solvent having appropriate solubility and appropriate setting of the treatment temperature allow easy removal of deteriorated materials derived from i-BPDA and a slight amount of impurities and easy preparation of i-BPDA powder having reduced color with a high yield. The solvent is not necessarily a single one. A mixture of a plurality of solvents may be used, as long as the solubility of the powder in the mixture is 0.5 g/100 g or more.

The examples of the preferred solvent include, but not limited to, alcohols such as methanol, ethanol, butanol, isopropyl alcohol, n-propyl alcohol, butanol, tert-butanol, butanediol, ethyl hexanol, and benzyl alcohol; ketones such as acetone, methyl ethyl ketone, methyl isobutyl ketone, diisobutyl ketone and cyclohexanone; esters such as ethyl acetate, methyl acetate, butyl acetate, methoxybutyl acetate, cellosolve acetate, amyl acetate, n-propyl acetate, isopropyl acetate, methyl lactate, ethyl lactate, butyl lactate, γ-valerolactone, δ-valerolactone, γ-caprolactone, ε-caprolactone and α-methyl-γ-butyrolactone; ethers such as dimethyl ether, ethyl methyl ether, diethyl ether, furan, dibenzofuran, oxetane, tetrahydrofuran, tetrahydropyran, methyl cellosolve, cellosolve, butyl cellosolve, dioxane, methyl tertiary butyl ether, butyl carbitol, ethylene glycol, diethylene glycol, triethylene glycol, propylene glycol, diethylene glycol monomethyl ether, triethylene glycol monomethyl ether, propylene glycol monomethyl ether, 3-methoxy-3-methyl-1-butanol, ethyleneglycol monomethyl ether acetate, propylene glycol monomethyl ether acetate, diethylene glycol monomethyl ether acetate, diethylene glycol monoethyl ether acetate; nitriles such as acetonitrile, propionitrile and butyronitrile; amides such as N-methyl-2-pyrrolidone, N, N-dimethylformamide and N, N-dimethylacetamide; sulfones such as dimethyl sulfoxide; carbonates such as dimethyl carbonate and diethyl carbonate; phenols such as m-cresol, p-cresol, 3 - chlorophenol and 4-chlorophenol; and others, for examples, acetophenone, 1,3-dimethyl-2-imidazolidinone, sulfolane and water. In particular, preference is given to dimethyl sulfoxide, N, N-dimethylformamide, N, N-dimethylacetamide, N-methyl-2-pyrrolidone and N-ethyl-2-pyrrolidone. These solvents are preferably high-purity solvents not containing impurities, metal component and water. When alcohols or water is used, an acid anhydride may cause a ring-opening reaction. Accordingly, it is preferable to conduct heat treatment for ring-closure in a subsequent operation.

In the purification method (1) of the present invention, the temperature of mixing a solvent and i-BPDA powder should be lower than the boiling point of the solvent and is 150°C or less, preferably 100°C or less, and more preferably 0 to 50°C. A treatment at a temperature near the boiling point of the solvent may cause coloring by the reaction, decomposition, or oxidative degradation of the solvent.

In the purification method (1) of the present invention, the undissolved i-BPDA powder can be suitably separated and collected from the mixture by a known method such as atmospheric pressure filtration, pressure filtration, filtration under reduced pressure, or centrifugal filtration. In the solvent extraction at ambient temperature or higher, heating is preferably performed for preventing precipitation. In addition, a decrease in temperature during extraction before the completion of filtration may cause precipitation of impurities dissolved in the solvent and is therefore not preferable.

In the purification method (1) of the present invention, the separated and collected i-BPDA powder is preferably dried. The drying can be suitably performed by a known method such as hot-air drying, heat drying under an inert gas flow, or vacuum drying. A part of acid anhydrides may cause a ring-opening reaction during the solvent extraction. Accordingly, cyclization is preferably performed in the drying step by, for example, heating.

In the purification method (2) of purification of the present invention, a purification step of recrystallizing a powder containing 90% or more of i-BPDA from a solution containing an acid anhydride can be suitably used. The solution containing an acid anhydride used here is preferably a solution containing an aliphatic acid anhydride such as acetic anhydride or propionic anhydride in a molar amount of twice or more of the amount of 2,2',3,3'-biphenyltetracarboxylic acid. The same solvents as those in the method (1) of purification are preferably used. The filtration and drying of the purified product is suitably performed by the methods described above.

In the purification method (3) of the present invention, i-BPDA can be suitably purified by sublimation at a temperature of 350°C or less and a reduced pressure of 50 Torr or less. The sublimation conditions are preferably a temperature of 350°C or less and a reduced pressure of 50 Torr or less, preferably a temperature of 150 to 300°C or less and a reduced pressure of 5 Torr or less. A temperature of 350°C or more may decompose and color i-BPDA, whereas a temperature of 150°C or less decreases the production efficiency. A reduced pressure of 50 Torr or more may oxidize or color i-BPDA. In addition, the production methods described in Japanese Patent Laid-Open Nos. 2005-314296 and 2006-45198 may be performed sequentially.

The polyimide of the present invention is prepared by a reaction between i-BPDA having a light transmittance of 80% or more at 400 nm and an optical path length of 1 cm and a diamine component. The polyimide has higher light transmittance than that of a polyimide prepared by a reaction of i-BPDA having a light transmittance of less than 80% at 400 nm and a diamine component. The polyimide preferably has a light transmittance, at 400 nm, of 70% or more and more preferably 80% or more when formed into a film having a thickness of 10 µm.

The polyimide of the present invention may further include another tetracarboxylic dianhydride in addition to i-BPDA in an amount of 90% or less, preferably 50% or less, based on the total moles of the tetracarboxylic dianhydrides. The use of a tetracarboxylic dianhydride other than i-BPDA increases the solubility of the polyimide precursor, resulting in easiness of the production. The tetracarboxylic dianhydride other than i-BPDA is not particularly limited and may be any tetracarboxylic dianhydride generally employed for a polyimide, but an aromatic tetracarboxylic dianhydride is preferred. The examples of such tetracarboxylic dianhydride include 3, 3', 4, 4' - biphenyltetracarboxylic dianhydride, 2, 3', 3, 4' - biphenyltetracarboxylic dianhydride, pyromellitic dianhydride, oxydiphthalic dianhydride, 3,3', 4,4' - benzophenone tetracarboxylic dianhydride, 3, 3 ', 4,4' - diphenylsulfone tetracarboxylic dianhydride, m-terphenyl-3, 3 ', 4,4' - tetracarboxylic dianhydride, 4,4'-(2,2 hexafluoroisopropylene)diphthalic dianhydride, 2,2'-bis(3,4-dicarboxyphenyl)propane dianhydride, 1,4,5,8 - naphthalenetetracarboxylic dianhydride, 2,3,6,7 - naphthalenetetracarboxylic dianhydride, (1,1':3',1"- terphenyl) -3, 3", 4, 4" - tetracarboxylic dianhydride, 4,4'- (dimethylsiladiyl)diphthalic dianhydride, 4,4' - (1,4 - phenylenebis(oxy))diphthalic dianhydride and the like, and more preferably 2, 2', 3, 3' - biphenyltetracarboxylic dianhydride and 2, 2', 3, 3' - biphenyltetracarboxylic dianhydride.

The diamine component used for preparation of the polyimide of the present invention is not particularly limited and may be diamines described in Part C.

As described in Part C, among these diamines, 1,4-diaminocyclohexane, bis(4,4'-aminocyclohexyl)methane, 2,2'-bis(trifluoromethyl)-4,4'-diaminobiphenyl, 4,4'-diaminodiphenylsulfone, and derivatives thereof provide excellent transparency and heat resistance to the resulting polyimides and are therefore more preferable; and trans-1,4-diaminocyclohexane further has low coefficient of linear thermal expansion and is therefore most preferable.

The diamine component may be suitably used as a diamine derivative obtained by reaction with a silylating agent (such as an amide-based silylating agent) for increasing the reactivity or the solubility of the reaction product.

The polyimide precursor can be readily produced by, but not particularly limited to, the method of producing a polyimide precursor described in Part D, i.e., a method through 1) a polyamic acid or 2) a polyamic acid silyl ester.

In addition, each of the methods of production can be suitably performed in an organic solvent, and as a result, a polyimide precursor solution composition can be readily prepared.

In these methods of production, the molar ratio of the tetracarboxylic acid component to the diamine component can be appropriately determined based on the viscosity of a target polyimide precursor and is preferably 0.90 to 1.10 and more preferably 0.95 to 1.05.

Specifically, the organic solvent used in the method of production is preferably an aprotic solvent such as N,N-dimethylformamide, N,N-dimethylacetamide, N-methyl-2-pyrrolidone, or dimethyl sulfoxide, but the structure is not particularly limited because any solvent may be used without problem as long as the solvent can dissolve the raw material monomers and the generated polyimide precursor. Examples of the usable organic solvent include those exemplified as the "organic solvent used in the method of production" in Part A.

The polyimide precursor solution of the present invention may optionally contain a chemical imidization agent (an acid anhydride such as acetic anhydride or an amine compound such as pyridine or isoquinoline), an antioxidant, a filler, a dye, an inorganic pigment, a silane coupling agent, a fire-retarding material, an antifoaming agent, a leveling agent, a rheology-controlling agent (flow assistant), a release agent, etc.

The polyimide of the present invention can be produced through a cyclodehydration reaction (imidization reaction) of the polyimide precursor of the present invention. The process of imidization is not particularly limited, and a known thermal imidization or chemical imidization can be suitably employed. Preferred examples of the form of the resulting polyimide include films, polyimide laminates, powders, beads, molded products, foamed products, and varnishes.

The polyimide of the present invention has, but is not limited to, an average coefficient of linear thermal expansion at 50 to 200°C of 50 ppm/K or less, preferably 30 ppm/K or less, and more preferably 20 ppm/K or less when formed into a film.

The thickness of a film formed from the polyimide of the present invention is determined depending on the purpose and is preferably about 1 to 200 µm and more preferably about 1 to 100 µm.

The polyimide of the present invention is suitable for, but not particularly limited to, an optical material because it has excellent properties of transparency and toughness. For examples it used as a substrate such as a transparent base material for displays, a transparent base material for touch panels, or a transparent substrate for solar cells.

The polyimide precursor can be used for producing a polyimide/substrate laminate and a polyimide film. Examples of the method of production are as those described in Part A, and the polyimide film/base material laminate or the polyimide film can be produced as in Part A, and also a flexible conductive substrate can be produced as in Part A.

### « PART G »

The invention disclosed in Part G relates to a polyimide having high transparency, high mechanical strength, and low coefficient of linear thermal expansion and relates to a polyimide precursor thereof.

The purpose of the invention disclosed in Part G is to provide a polyimide having excellent transparency, high mechanical strength, and low coefficient of linear thermal expansion suitable for a transparent base material for a flexible display, solar cell, or touch panel and to provide a polyimide precursor of the polyimide. The transparency has been extremely improved compared with a known polyimide by strictly controlling the transmittance of the diamine and the tetracarboxylic dianhydride.

The invention disclosed in Part G relates to the following items.
1. A polyimide prepared by a reaction between a diamine component and a tetracarboxylic acid component, wherein
   the diamine component comprises an aromatic ring-free diamine (including a derivative thereof, the same applies to the following) having a light transmittance of 90% or more or an aromatic ring-containing diamine (including a derivative thereof, the same applies to the following) having a light transmittance of 80% or more (here, the transmittance of the diamine component is that measured at a wavelength of 400 nm and an optical path length of 1 cm as a 10% by mass solution in pure water or N, N-dimethylacetamide); and
   the tetracarboxylic acid component comprises a tetracarboxylic acid (including a derivative thereof, the same applies to the following) having a light transmittance of 75% or more (here, the transmittance of the tetracarboxylic acid component is that measured at a wavelength of 400 nm and an optical path length of 1 cm as a 10% by mass solution in a 2 N aqueous sodium hydroxide solution).

2. The polyimide according to item 1, wherein the diamine has a light transmittance of 95% or more at a wavelength of 400 nm and an optical path length of 1 cm, and the tetracarboxylic acid has a light transmittance of 80% or more.

3. The polyimide according to item 1 or 2, wherein at least one of the tetracarboxylic acid and the diamine is an aromatic compound.

4. The polyimide according to item 1 or 2, wherein the tetracarboxylic acid is an aromatic tetracarboxylic acid compound, and the diamine is an aliphatic diamine compound.

5. The polyimide according to any one of items 1 to 4, wherein the tetracarboxylic acid is biphenyltetracarboxylic acid.

6. The polyimide according to any one of items 1 to 4, wherein the diamine is trans-1,4-diaminocyclohexane.

7. The polyimide according to any one of items 1 to 6, having a light transmittance of 80% or more at 400 nm when formed into a film having a thickness of 10 µm.

8. The polyimide according to any one of items 1 to 7, which is used as an optical material.

9. A polyimide precursor, comprising an aromatic ring-free diamine in an amount of 50% by mol or more of the total moles of the diamine component used; the polyimide precursor having a light transmittance of 90% or more at a wavelength of 400 nm and an optical path length of 1 cm as a 10% by mass solution in a polar solvent.

10. A polyimide precursor, comprising an aromatic ring-containing diamine in an amount of 50% by mol or more of the total moles of the diamine component used; the polyimide precursor having a light transmittance of 50% or more at a wavelength of 400 nm and an optical path length of 1 cm as a 10% by mass solution in a polar solvent.

11. The polyimide precursor according to item 9 or 10, having a logarithmic viscosity of 0.2 dL/g or more as a 0.5 g/dL solution in N,N-dimethylacetamide at 30°C.

12. The polyimide precursor according to item 9 or 11 comprising a unit structure represented by general Formula (G1):

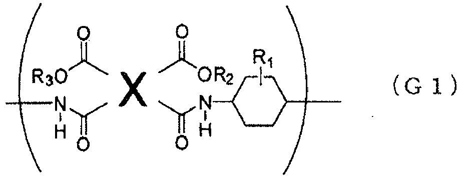

wherein, in general Formula (G1), X represents a tetravalent organic group; R₁ represents a hydrogen atom or an alkyl group having 1 to 4 carbon atoms; and R₂ and R₃ each represent a hydrogen atom, an alkyl group having 1 to 6 carbon atoms, or an alkylsilyl group having 3 to 9 carbon atoms.

13. A polyimide precursor solution composition comprising the polyimide precursor according to any one of items 9 to 12 evenly dissolved in a solvent.

14. A polyimide prepared by imidization of the polyimide precursor according to any one of items 9 to 12.

According to the invention disclosed in Part G, it is possible to provide a polyimide having excellent transparency, high mechanical strength, and low coefficient of linear thermal expansion suitable for a transparent base material for a flexible display, solar cell, or touch panel and to provide a polyimide precursor of the polyimide.

The polyimide disclosed in Part G is prepared by a reaction between a diamine component and a tetracarboxylic acid component, wherein
the diamine component contains an aromatic ring-free diamine (including a derivative thereof, as described above) having a light transmittance of 90% or more and preferably 95% or more or an aromatic ring-containing (including a derivative thereof, as described above) diamine having a light transmittance of 70% or more and preferably 80% or more (here, the light transmittance is that measured at a wavelength of 400 nm and an optical path length of 1 cm as a 10% by mass solution in pure water or N, N-dimethylacetamide); and
the tetracarboxylic acid component contains a tetracarboxylic acid (including a derivative thereof, as described above) having a light transmittance of 80% or more, preferably 85% or more, and most preferably 90% or more (here, the light transmittance is that measured at a wavelength of 400 nm and an optical path length of 1 cm as a 10% by mass solution in a 2 N aqueous sodium hydroxide solution). When the diamine constituting the diamine component and the tetracarboxylic acid constituting the tetracarboxylic acid component each have light transmittance in the above-mentioned ranges, the resulting polyimide is reduced in coloring and is therefore advantageous. In addition, preferably 80% or more, more preferably 90% or more, more preferably 95% or more, and most preferably 100% of the (one or more) diamines constituting the diamine component satisfy the above-mentioned light transmittance. Similarly, preferably 80% or more, more preferably 90% or more, more preferably 95% or more, and most preferably 100% of the (one or more) tetracarboxylic acids constituting the tetracarboxylic acid component satisfy the above-mentioned light transmittance.

While not particularly limited, in the polyimide of the present invention, at least one of the tetracarboxylic acid component and the diamine component is preferably an aromatic compound because the resulting polyimide has high heat resistance. Furthermore, it is more preferable that the tetracarboxylic acid component essentially consists of aromatic tetracarboxylic acids and the diamine component essentially consists of aliphatic diamines because it improves transparency and achieves low coefficient of linear thermal expansion.

The tetracarboxylic acid component used for the polyimide of the present invention is not particularly limited and may be any tetracarboxylic acid component generally employed as a raw material for a polyimide, but an aromatic tetracarboxylic dianhydride and alicyclic tetracarboxylic dianhydride are preferred. The examples of the aromatic tetracarboxylic dianhydride and the alicyclic tetracarboxylic dianhydride include those exemplified in Part E.

In particular, preference is given to 3, 3', 4, 4' - biphenyltetracarboxylic dianhydride, 2, 2', 3, 3' - biphenyltetracarboxylic dianhydride, 2, 3', 3, 4' - biphenyltetracarboxylic dianhydride, pyromellitic dianhydride, 4,4'-(2,2 hexafluoroisopropylene)diphthalic dianhydride and 4,4'-(dimethylsiladiyl)diphthalic dianhydride because they give a polyimide excellent in mechanical properties and heat resistance. Particularly preference is given to 3, 3', 4, 4' - biphenyltetracarboxylic dianhydride, 2, 2', 3, 3' - biphenyltetracarboxylic dianhydride, 2, 3', 3, 4' - biphenyltetracarboxylic dianhydride because they give a polyimide having low coefficient of linear thermal expansion.

The tetracarboxylic acid used in the present invention is preferably purified for reducing coloring. The purification may be performed by any known method without particular limitation, and preferred are the following methods:
(1) purification method of mixing a solvent and a tetracarboxylic acid (e.g., tetracarboxylic dianhydride) powder in an uneven state where at least a part of the tetracarboxylic acid powder is not dissolved and subsequently separating and collecting the undissolved tetracarboxylic acid powder from the mixture;
(2) purification method of recrystallizing from a solution containing an acid anhydride; and
(3) purification method of subliming by heating under reduced pressure. These purification steps may be repeated multiple times or may be employed in combination.

The diamine component is not particularly limited and may be any diamine that is generally used for polyimides. Preferred examples of the diamine include those described in Part C for increasing the transparency of polyimides.

Preference is given to 1,4-Diaminocyclohexane, bis(4,4'-aminocyclohexane)methane, 2,2'-bis(trifluoromethyl)-4,4'-diaminobiphenyl, and 4,4'-diaminodiphenylsulfone because polyimides using these have excellent transparency and heat resistance; and particular preference is given to trans-1,4-diaminocyclohexane because the resulting polyimide has low coefficient of linear thermal expansion.

The diamines used in the present invention are preferably purified for reducing coloring. The purification may be performed by any known method without particular limitation and can be suitably purified by the following methods:
(1) purification method by sublimation,
(2) purification method by treatment with an adsorbent, or
(3) purification method by recrystallization.
   These purification steps may be repeated multiple times or may be employed in combination.

The diamine component may be suitably used as a diamine derivative that is a compound obtained by reaction with a silylating agent (such as an amide-based silylating agent) for increasing the reactivity or the solubility of the reaction product.

When the polyimide precursor of the present invention contains an aromatic ring-free diamine in an amount of 50% by mol or more of the total moles of the diamine component used, the light transmittance of the polyimide precursor at a wavelength of 400 nm and an optical path length of 1 cm is 90% or more, preferably 95% or more, as a 10% by mass solution in a polar solvent. Alternatively, when the polyimide precursor contains an aromatic ring-containing diamine in an amount of 50% by mol or more of the total moles of the diamine component used, the light transmittance at a wavelength of 400 nm and an optical path length of 1 cm is 50% or more, preferably 55% or more, as a 10% by mass solution in a polar solvent.
The solvent used for the measurement is not particularly limited as long as it dissolves the polyimide precursor, and the examples thereof include amide solvents such as N, N-dimethylformamide, N, N-dimethylacetamide, N-methyl-2-pyrrolidone and N-ethyl-2-pyrrolidone; cyclic ester solvents such as γ-butyrolactone, γ-valerolactone, δ-valerolactone, γ-caprolactone, ε-caprolactone, and α-methyl-γ-butyrolactone; carbonate solvents such as ethylene carbonate and propylene carbonate; glycol-based solvents such as triethylene glycol; phenol-based solvents such as m-cresol, p-cresol, 3-chlorophenol and 4-chlorophenol; acetophenone, 1,3-dimethyl-2-imidazolidinone, sulfolane, and dimethylsulfoxide. In addition, other common organic solvents, for example, phenol, o-cresol, butyl acetate, ethyl acetate, isobutyl acetate, propylene glycol methyl acetate, ethyl cellosolve, butyl cellosolve, 2-methylcellosolve acetate, ethyl cellosolve acetate, butyl cellosolve acetate, tetrahydrofuran, dimethoxyethane, diethoxyethane, dibutyl ether, diethylene glycol dimethyl ether, methyl isobutyl ketone, diisobutyl ketone, cyclopentanone, cyclohexanone, methyl ethyl ketone, acetone, butanol, and ethanol may be used. These solvents may be used in combination of two or more.

The polyimide precursor of the present invention can be readily produced by, but not limited to, the method of producing a polyimide precursor described in Part D, i.e., a method through 1) a polyamic acid or 2) a polyamic acid silyl ester, or a method through 3) a polyamic acid ester shown below.

### 3) Polyamic acid ester

A diester dicarboxylic acid chloride is prepared by reacting a tetracarboxylic dianhydride with an appropriate alcohol and then reacting the resulting diester dicarboxylic acid with a chlorinating agent (e.g., thionyl chloride or oxalyl chloride). A polyimide precursor can be prepared by reacting the diester dicarboxylic acid chloride with a diamine. Alternatively, the polyimide precursor can be readily prepared by dehydration condensation of a diester dicarboxylic acid and a diamine using, for example, a phosphorus condensing agent or a carbodiimide condensing agent. Furthermore, since the polyimide precursor is stable, for example, even purification by reprecipitation from a solvent such as water or alcohol can be performed.

Each of the above-mentioned methods of production (the above methods 1) to 3)) can be suitably performed in an organic solvent, and as a result, a polyimide precursor solution composition can be readily prepared.

In these methods of production, the molar ratio of the tetracarboxylic acid component to the diamine component can be appropriately determined based on the viscosity of a target polyimide precursor and is preferably 0.90 to 1.10 and more preferably 0.95 to 1.05.

Specifically, the organic solvent used in the method of production is preferably an aprotic solvent such as N,N-dimethylformamide, N,N-dimethylacetamide, N-methyl-2-pyrrolidone, or dimethyl sulfoxide, but the structure is not particularly limited because any solvent may be used without problem as long as the solvent can dissolve the raw material monomers and the polyimide precursor produced. Examples of the usable organic solvent include those exemplified as the "organic solvent used in the method of production" in Part A.

The logarithmic viscosity of the polyimide precursor of the present invention is not particularly limited and is preferably 0.2 dL/g or more, more preferably 0.5 dL/g or more, as a 0.5 g/dL solution in N,N-dimethylacetamide at 30°C. When the logarithmic viscosity is 0.2 dL/g or more, the polyimide precursor has high molecular weight to increase the mechanical strength of the resulting polyimide film. The logarithmic viscosity is also preferably 2.5 dL/g or less and more preferably 2.0 dL/g or less. When the logarithmic viscosity is small, the polyimide precursor solution composition has a low viscosity to provide a good handling property during the polyimide film production.

The polyimide precursor of the present invention comprises, but not limited to, preferably a unit structure represented by general Formula (G1):

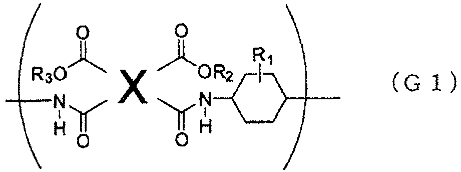

wherein, in general Formula (G1), X represents a tetravalent organic group; R₁ represents a hydrogen atom or an alkyl group having 1 to 4 carbon atoms; and R₂ and R₃ each represent a hydrogen atom, an alkyl group having 1 to 6 carbon atoms, or an alkylsilyl group having 3 to 9 carbon atoms.
X is preferably a tetravalent organic group selected from Formula (G2) below and particularly preferably a tetravalent biphenyl isomer.

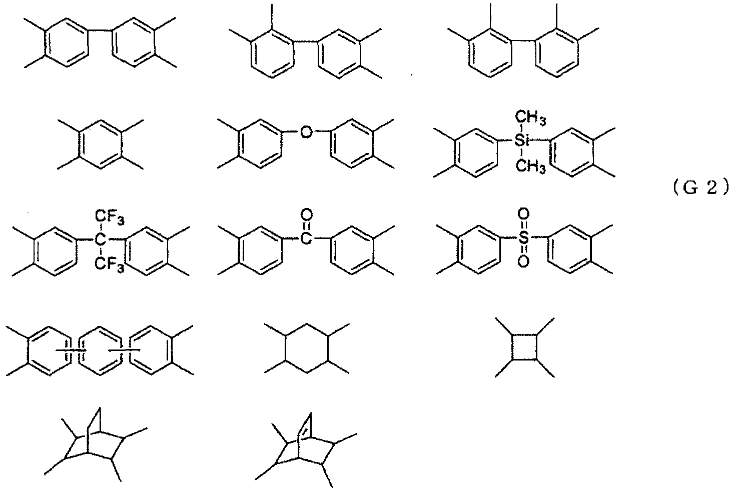

The polyimide of the present invention can be produced through a cyclodehydration reaction (imidization reaction) of the polyimide precursor. The process of imidization is not particularly limited, and a known thermal imidization or chemical imidization can be suitably employed. Preferred examples of the form of the resulting polyimide include films, polyimide laminates, coating films, powders, beads, molded products, foamed products, and varnishes.

The polyimide of the present invention has, but not limited to, a light transmittance of 80% or more, preferably 85% or more, and more preferably 90% or more, at 400 nm when formed into a film having a thickness of 10 µm

The polyimide of the present invention has, but is not limited to, an average coefficient of linear thermal expansion at 50 to 200°C of 50 ppm/K or less, preferably 30 ppm/K or less, and more preferably 20 ppm/K or less when formed into a film.

The thickness of a film formed from the polyimide of the present invention is determined depending on the purpose and is preferably about 1 to 200 µm and more preferably about 1 to 100 µm.

The polyimide of the present invention is suitable for, but not particularly limited to, an optical material because it has excellent properties of transparency and toughness. For examples it used as a substrate such as a transparent base material for displays, a transparent base material for touch panels, or a transparent substrate for solar cells.

The polyimide precursor can be used for producing a polyimide/substrate laminate and a polyimide film. Examples of the method of production are as those described in Part A, and the polyimide film/base material laminate or the polyimide film can be produced as in Part A, and also a flexible conductive substrate can be produced as in Part A.

### << PART H >>

The invention disclosed in Part H relates to a polyimide precursor varnish that can provide a polyimide having high transparency most suitable as an optical material having high heat resistance and relates to a method of producing the polyimide varnish. Specifically, the invention is achieved by strictly controlling the purity of the organic solvent used.

As described in Background Art, in also the case of a semi-alicyclic polyimide prepared using trans-1,4-diaminocyclohexane, the optical transmission spectrum has an absorption at about 400 nm. This demonstrates that the polyimide is colored not only due to the molecular structure, such as CT absorption, but also due to the raw material of a polyimide precursor varnish. Since the polyimide precursor and the polyimide have poor solubility, a nitrogen-containing solvent is usually used. Since the nitrogen-containing solvent tends to be colored at high temperature, coloring derived from the solvent is suspected. However, how to suppress this phenomenon has not been investigated in known technology.

The purpose of the invention disclosed in Part H is to provide a polyimide precursor varnish that can prepare a polyimide having high transparency most suitable as a transparent base material for a flexible display, solar cell, or touch panel and to provide a method of producing a polyimide varnish.

The invention disclosed in Part H relates to the following items.

1. A method of producing a varnish, comprising at least an organic solvent and a polyimide precursor represented by general Formula (H1) or a polyimide represented by general Formula (H2):

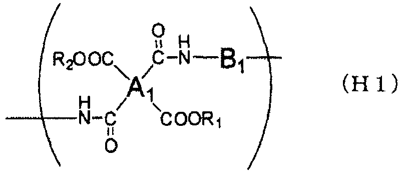

(in general Formula (H1), A₁ represents a tetravalent aliphatic or aromatic group; B₁ represents a divalent aliphatic or aromatic group; and R₁ and R₂ each independently represent a hydrogen atom, an alkyl group having 1 to 6 carbon atoms, or an alkylsilyl group having 3 to 9 carbon atoms),

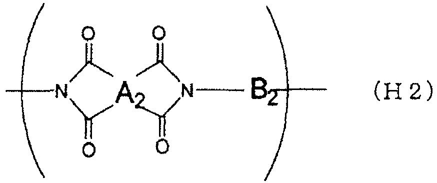

(in general Formula (H2), A₂ represents a tetravalent aliphatic or aromatic group; and B₂ represents a divalent aliphatic or aromatic group), wherein
the organic solvent to be contained in the varnish (hereinafter, referred to as the organic solvent used) has a light transmittance of 89% or more at 400 nm and an optical path length of 1 cm.

2. The method of producing a varnish according to item 1, wherein the organic solvent used has a light transmittance of 20% or more at 400 nm and an optical path length of 1 cm after heating with refluxing in nitrogen for 3 hours.

3. The method of producing a varnish according to item 1 or 2, wherein the organic solvent used has a purity of 99.8% or more as measured by gas chromatography.

4. The method of producing a varnish according to any one of items 1 to 3, wherein in the organic solvent used, amount of impurities of which peak appears on the longer time side with respect to the main component peak retention time in gas chromatography is less than 0.2% in total.

5. The method of producing a varnish according to any one of items 1 to 4, wherein the organic solvent used has a purity of 99.9% or more.

6. The method of producing a varnish according to any one of items 1 to 5, wherein amount of components non-volatile at 250°C in the organic solvent used is 0.1% or less.

7. The method of producing a varnish according to any one of items of 1 to 6, wherein a metal content in the organic solvent used is 10 ppm or less.

8. The method of producing a varnish according to any one of items 1 to 7, wherein the organic solvent used is a nitrogen-containing compound.

9. The method of producing a varnish according to item 8, wherein the organic solvent used is selected from the group consisting of N,N-dimethylacetamide, N,N-dimethylformamide, N-methyl-2-pyrrolidone, N-ethyl-2-pyrrolidone, dimethyl imidazolidinone, and combinations of two or more thereof.

10. The method of producing a varnish according to any one of items of 1 to 9, wherein A₁ in general Formula (H1) and A₂ in general Formula (H2) each represent a tetravalent aromatic group; and B₁ in general Formula (H1) and B₂ in general Formula (H2) each represent a divalent aromatic group.

11. The method of producing a varnish according to any one of items 1 to 9, wherein A₁ in general Formula (H1) and A₂ in general Formula (H2) each represent a tetravalent aromatic group; and B₁ in general Formula (H1) and B₂ in general Formula (H2) each represent a divalent aliphatic group.

12. The method of producing a varnish according to any one of items 1 to 9, wherein A₁ in general Formula (H1) and A₂ in general Formula (H2) each represent a tetravalent aliphatic group; and B₁ in general Formula (H1) and B₂ in general Formula (H2) each represent a divalent aromatic group.

13. The method of producing a varnish according to item 10 or 11, wherein A₁ in general Formula (H1) and A₂ in general Formula (H2) are selected from the group consisting of tetravalent aromatic groups represented by Formulae (H3):

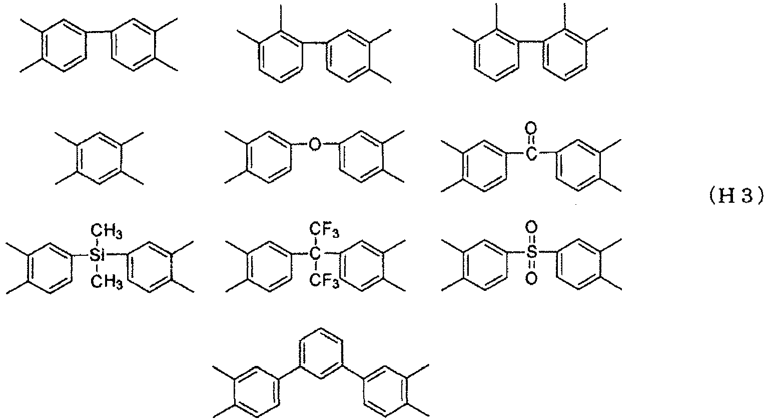

14. The method of producing a varnish according to item 12, wherein A₁ in general Formula (H1) and A₂ in general Formula (H2) are selected from the group consisting of tetravalent aliphatic groups represented by Formulae (H4):

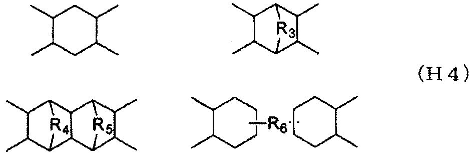

wherein, in Formulae (H4), R₃ to R₅ each independently represent a CH₂ group, a C₂H₄ group, an oxygen atom, or a sulfur atom; and R₆ represents a direct bond, a CH₂ group, a C(CH₃)₂ group, a SO₂ group, a Si(CH₃)₂ group, a C(CF₃)₂ group, an oxygen atom, or a sulfur atom.

15. The method of producing a varnish according to item 10 or 12, wherein B₁ in general Formula (H1) and B₂ in general Formula (H2) are selected from the group consisting of divalent aromatic groups represented by general Formulae (H5-1) to (H5-5):

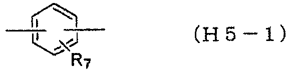

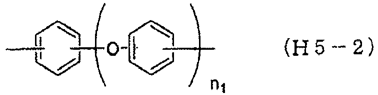

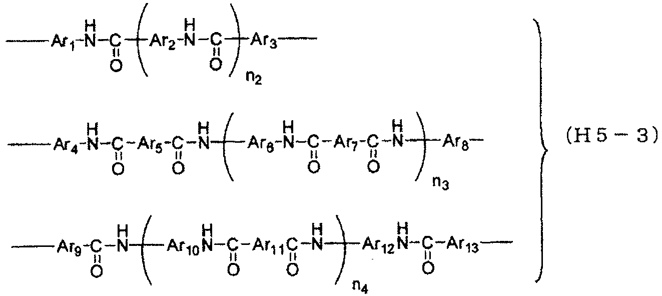

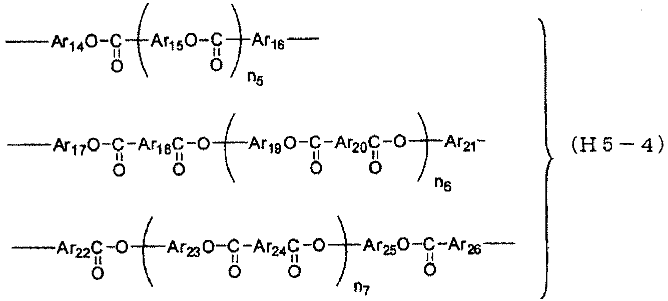

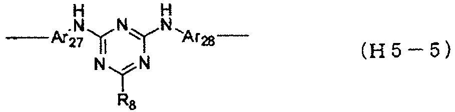

wherein, in general Formulae (H5-1) to (H5-5), R₇ represents hydrogen, a methyl group, or an ethyl group; R₈ is a monovalent organic group; Ari to Ar₂₈ each independently represent a divalent group having an aromatic ring having 6 to 18 carbon atoms; n₁ represents an integer of 1 to 5; and n₂ to n₇ each independently represent an integer of 0 to 5.

16. The method of producing a varnish according to item 11, wherein B₁ in general Formula (H1) and B₂ in general Formula (H2) are selected from the group consisting of divalent aliphatic groups represented by general Formulae (H6):

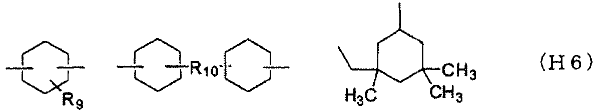

wherein, in general Formula (H6), R₉ represents hydrogen or a hydrocarbon group having 1 to 3 carbon atoms; and R₁₀ represents a direct bond, a CH₂ group, a C(CH₃)₂ group, a SO₂ group, a Si(CH₃)₂ group, a C(CF₃)₂ group, an oxygen atom, or a sulfur atom.

17. The method of producing a varnish according to any one of items 1 to 16, wherein a polyimide film with a thickness of 10 µm formed from a varnish produced by the method according to any one of items 1 to 16 has a light transmittance at 400 nm higher than that of a polyimide film formed from a polyimide produced using an organic solvent not satisfying requirements defined in each item.

18. The method of producing a varnish according to any one of items 1 to 16, wherein the varnish has transparency having a light transmittance at 400 nm of 70% or more when a polyimide film having a thickness of 10 µm is formed using the varnish produced by the method according to any one of items 1 to 16.

19. The method of producing a polyimide, comprising forming the polyimide using the varnish produced by the method according to any one of items 1 to 18.

20. A method of producing an optical material to be used for transmitting or reflecting light, using the varnish produced by any one of items 1 to 18.

The invention disclosed in Part H can provide a method of producing a polyimide precursor varnish and a polyimide varnish that can prepare polyimides having high transparency. These polyimide precursor varnish and polyimide varnish can be suitably used as transparent heat resistant base materials for a flexible display, solar cell, or touch panel.

The present inventors have diligently studied and, as a result, have found that the purity of an organic solvent highly affects the coloring of polyimide. As described above, it has been believed that the coloring of polyimide is generally based on the chemical structure thereof and also that the coloring due to deterioration of a nitrogen-containing solvent at high temperature is unavoidable. Accordingly, it has been unexpected that the purity of an organic solvent highly affects the coloring of polyimide. In particular, since the weight proportion of the organic solvent in the varnish is high, though the amount of impurities in the organic solvent is small, the impurities are believed to cause coloring of polyimide.

As described below, a polyimide of which transparency is notably increased can be prepared from a varnish containing a polyimide precursor or a polyimide produced using an organic solvent having a purity strictly controlled. The purity is controlled using, an indicator, at least one of characteristics relating to the purity, i.e., the light transmittance, the light transmittance after heating with refluxing, the purity as measured by gas chromatography, the proportion of impurity peaks in gas chromatography, the amount of non-volatile components, and the content of metal components.

The invention disclosed in Part H can prepare a polyimide having higher transparency than that of a polyimide produced by a known method. The invention disclosed in Part H is preferably used for producing the polyimide precursor described in Parts A and B.

The invention disclosed in Part H will be explained in details below.
The varnish produced by the invention disclosed in Part H comprises at least an organic solvent and a polyimide precursor represented by general Formula (H1) or a polyimide represented by general Formula (H2):

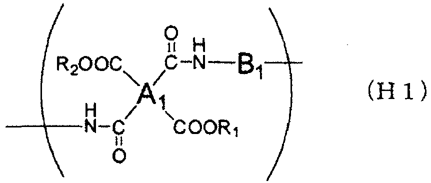

(in general Formula (H1), A₁ represents a tetravalent aliphatic or aromatic group; B₁ represents a divalent aliphatic or aromatic group; and R₁ and R₂ each independently represent a hydrogen atom, an alkyl group having 1 to 6 carbon atoms, or an alkylsilyl group having 3 to 9 carbon atoms),

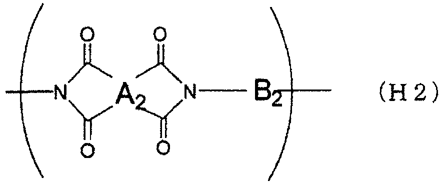

(in general Formula (H2), A₂ represents a tetravalent aliphatic or aromatic group; and B₂ represents a divalent aliphatic or aromatic group).
In the present specification, the term "varnish" means both of a varnish containing a polyimide precursor represented by general Formula (H1) and a varnish containing a polyimide represented by general Formula (H2) unless otherwise explicitly mentioned.

In some steps of the process of producing a varnish, organic solvents are used. Except that a small amount of solvents evaporates, almost the entire organic solvents used in the production process are contained in the varnish. In the present invention, the term "organic solvent used" refers to the entire organic solvents used in all steps involved in production of the varnish. More specifically, the term "organic solvent used" includes an organic solvent as a polymerization solvent used in the polymerization step and also solvents optionally used such as an organic solvent used in a step diluting a varnish to a target concentration or viscosity and an organic solvent used for preparing a dilution solution in advance for adding an additive.

The organic solvent used in the present invention satisfies the requirements described below with respect to at least one of characteristics relating to the purity defined below, i.e., (a) light transmittance, (b) light transmittance after heating with refluxing, (c) purity as measured by gas chromatography, (d) proportion of impurity peaks in gas chromatography, (e) amount of non-volatile components, and (f) content of metal components.

That is, the present invention relates to a method of producing a varnish, comprising at least an organic solvent and a polyimide precursor represented by general Formula (H1) or a polyimide represented by general Formula (H2), and the method satisfies at least one requirement selected from the following (a) to (f):
(a) producing the varnish using an organic solvent having a light transmittance of 89% or more at 400 nm and an optical path length of 1 cm;
(b) using an organic solvent having a light transmittance of 20% or more at 400 nm and an optical path length of 1 cm after heating with refluxing in nitrogen for 3 hours;
(c) using an organic solvent having a purity of 99.8% or more as measured by gas chromatography;
(d) using an organic solvent in which amount of impurities of which peak appears on the longer time side with respect to the main component peak retention time in gas chromatography is less than 0.2% in total;
(e) using an organic solvent in which amount of components non-volatile at 250°C is 0.1% or less; and
(f) using an organic solvent in which a metal content is 10 ppm or less.

Furthermore, the requirements for these characteristics are based on the entire organic solvents used. That is, the organic solvents used may be one type or two or more types. The use of two or more types of organic solvents means a case of using a solvent mixture in a specific step and a case of using different solvents in different steps such that the polymerization solvent and the solvent for diluting an additive are different from each other. In the case of using two or more types of organic solvents (hereinafter, referred to as a solvent mixture), each requirement for characteristics relating to the purity is applied to the solvent mixture as a whole. In the case where different solvents are used in different steps, each requirement for characteristics relating to the purity is applied to the mixture of all organic solvents finally contained in a varnish. The characteristics may be each measured for a mixture prepared actually mixing organic solvents. Alternatively, the characteristics may be each measured for each organic solvent, and the characteristic of a mixture as a whole may be determined by calculation. For example, when 70 parts of solvent A having a purity of 100% and 30 parts of solvent B having a purity of 90% are used, the organic solvent used has a purity of 97%.

Each requirement will be discussed in further detail.

### (a) Light transmittance

The organic solvent used preferably has a light transmittance, at 400 nm and an optical path length of 1 cm, of 89% or more, more preferably 90% or more, and most preferably 91% or more. The use of a solvent having high light transmittance reduces coloring of a polyimide film during the production of the film and is therefore preferable.

In general, when a polyimide film (referring to not only a free-standing film but also a coating film) formed into a film thickness of 10 µm has a transmittance of 70% or more at a wavelength of 400 nm, the range of use is considerably broadened in transparent application. Accordingly, "a film with a thickness of 10 µm having a transmittance at a wavelength of 400 nm of 70% or more" is one criterion. In the present invention, a varnish providing a polyimide having a transparency higher than the criterion can be prepared by controlling the purity of the organic solvent used so as to have a light transmittance of 89% or more (see Examples below).

### (b) Light transmittance after heating with refluxing

The organic solvent used preferably has a light transmittance, at 400 nm and an optical path length of 1 cm, of 20% or more, more preferably 40% or more, and most preferably 80% or more after heating the organic solvent to reflux under nitrogen atmosphere for 3 hours. The use of a solvent having high light transmittance after heating to reflex in nitrogen for 3 hours reduces coloring of a polyimide film during the production of the film and is therefore preferable. A varnish providing a polyimide for "a film with a thickness of 10 µm having a transmittance of 70% or more at a wavelength of 400 nm" can be prepared using an organic solvent controlled so as to have a purity satisfying the above-mentioned range (see Examples below).

### (c) Purity as measured by gas chromatography

The organic solvent used preferably has a purity of 99.8% or more, more preferably 99.9% or more, and most preferably 99.99% or more as measured by gas chromatography. An organic solvent having high purity can provide high light transmittance to the finally prepared polyimide film and is therefore preferable.

In general, if the purity of the organic solvent contained in a varnish is in the above-mentioned range as a result of analysis, it can be said that the organic solvent used has a purity within the above-mentioned range.

In case that a slight amount of another solvent (e.g., solvent other than the organic solvents exemplified below) is present together with the organic solvent, in the present invention, the solvent is not considered as "impurities" that affect the purity of the organic solvent as long as the solvent does not affect coloring (e.g., those having boiling points lower than that of the main component).

### (d) Proportion of impurity peak in gas chromatography

In the organic solvent used, the total amount of impurities of which peak appears on the longer time side with respect to the main component peak retention time in gas chromatography is preferably less than 0.2%, more preferably 0.1% or less, and most preferably 0.05% or less. The impurities appearing on the longer time side with respect to the main component peak retention time of the solvent have high boiling points or high intermolecular interactions. Consequently, the impurities hardly evaporate in the process of producing a polyimide film and tend to remain in a film as impurities to cause coloring. When two or more organic solvents are used, the total amount of impurities of which peak appears on the longer time side with respect to the main component peak on the longest retention time side in gas chromatography is preferably within the above-mentioned range.

### (e) Amount of non-volatile components

In the organic solvent used in the present invention, the amount of non-volatile components after heating at 250°C for 30 minutes is preferably 0.1% or less, more preferably 0.05% or less, and most preferably 0.01% or less. The non-volatile components in a solvent hardly evaporate in the production process of a polyimide film and tend to remain in the film as impurities to cause coloring of the film. Therefore, a smaller amount of non-volatile components is preferable.

### (f) Content of metal components

In the organic solvent used, the content of a metal component (e.g., Li, Na, Mg, Ca, Al, K, Ca, Ti, Cr, Mn, Fe, Co, Ni, Cu, Zn, Mo, and Cd) is preferably 10 ppm or less, more preferably 1 ppm or less, more preferably 500 ppb or less, and most preferably 300 ppb or less. A low content of metal components reduces the coloring of a solvent in high temperature treatment to reduce the coloring of a polyimide film in the production process of the film and is therefore preferable.

The requirements (a) to (f) described above may be each independently employed as a requirement for providing a polyimide having high transparency. That is, the requirements (a) to (f) described above each independently realize an embodiment of the present invention. However, it is preferable to satisfy two or more of requirements (a) to (f), and it is generally preferable to satisfy a larger number of requirements.

A solvent used is not particularly limited as long as it dissolves the above polyimide precursors or the above polyimide (in case of mixed solvent, if the mixed solvent dissolves the polyimide precursors or the polyimide, it is usable). The examples include amide solvents such as N, N-dimethylformamide, N, N-dimethylacetamide and N-methylpyrrolidone; cyclic ester solvents such as γ-butyrolactone, γ-valerolactone, δ-valerolactone, γ-caprolactone, ε-caprolactone, and α-methyl-γ-butyrolactone; carbonate solvents such as ethylene carbonate and propylene carbonate; glycol-based solvents such as triethylene glycol; phenol-based solvents such as m-cresol, p-cresol, 3-chlorophenol and 4-chlorophenol; acetophenone, 1,3-dimethyl-2-imidazolidinone, sulfolane, and dimethylsulfoxide. Due to particularly excellent solubility, aprotic solvents, such as N, N-dimethylformamide, N, N-dimethylacetamide, N-methyl-2-pyrrolidone, N-ethyl-2-pyrrolidone and dimethyl sulfoxide are preferred. In addition, other common organic solvents, for example, phenol, o-cresol, butyl acetate, ethyl acetate, isobutyl acetate, propyleneglycol methyl acetate, ethyl cellosolve, butyl cellosolve, 2-methylcellosolve acetate, ethylcellosolve acetate, butylcellosolve acetate, tetrahydrofuran, dimethoxyethane, diethoxyethane, dibutyl ether, diethylene glycol dimethyl ether, methyl isobutyl ketone, diisobutyl ketone, cyclopentanone, cyclohexanone, methyl ethyl ketone, acetone, butanol, ethanol, xylene, toluene, chlorobenzene, turpentine, mineral spirits, and petroleum naphtha-based solvents may be used. In terms of excellent dissolving ability to a polyimide precursors or a polyimide, preferred is a nitrogen-containing compound and more preferred is N, N-dimethylacetamide, N, N-dimethylformamide, N-methylpyrrolidone, N-ethylpyrrolidone and dimethylimidazolidinone. Among these, N, N-dimethylacetamide has less tendency of coloring at high temperature and reduces coloring of the film during the production of polyimide film, and is therefore preferred.

The polyimide precursors or the polyimide contained in the varnish of the present invention is as described above. The tetravalent aliphatic or aromatic group represented by A₁ in general Formula (H1) and A₂ in general Formula (H2) is a tetravalent residue in which four carboxyl groups (-COOH) are removed from a tetracarboxylic acid. Hereinafter, tetracarboxylic acid before removing the four carboxyl groups, its anhydride and the like are referred as tetracarboxylic acid component. The divalent aliphatic or aromatic group represented by B₁ in general Formula (H1) and B₂ in general Formula (H2) is a divalent residue in which two amino groups are removed from a diamine, and hereinafter the diamine before removing the two amino groups are referred as diamine component.

The combination of tetracarboxylic acid component and diamine component (tetracarboxylic acid component/diamine component) is preferably aromatic tetracarboxylic acid component/aromatic diamine component, aromatic tetracarboxylic acid component/aliphatic diamine component and aliphatic tetracarboxylic acid component/aromatic diamine component in view of excellent heat resistance. When aliphatic component is used as the individual components, those having alicyclic structure are more preferred.

The aromatic tetracarboxylic acid component is not particularly limited and may be any aromatic tetracarboxylic acid component generally employed as a tetracarboxylic acid component for a polyimides, but aromatic tetracarboxylic acid component wherein A₁ and A₂ are selected from the aromatic groups represented by Formulae (H3) are preferred because they provide a polyimide having high heat resistance.

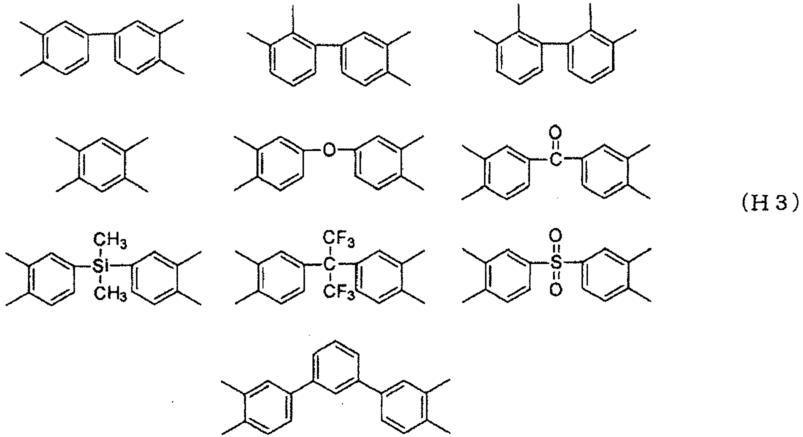

Among these, 3, 3', 4, 4'-biphenyltetracarboxylic acid, 2, 3, 3', 4'-biphenyltetracarboxylic acid, 2,2' ,3, 3' -biphenyltetracarboxylic acid, 4, 4'-oxydiphthalic acid 4,4'- (dimethyl-siladiyl)diphthalic acid and anhydride of these are more preferred because they provide polyimides having particularly high transparency. 3, 3', 4, 4'-biphenyltetracarboxylic acid, 2, 3, 3', 4'-biphenyltetracarboxylic acid, 2,2' ,3, 3' -biphenyltetracarboxylic acid and anhydride of these are particularly preferred because they further provide polyimides having low coefficient of thermal expansion.

The aliphatic tetracarboxylic acid component is not particularly limited and may be any tetracarboxylic acid component generally employed as an aliphatic tetracarboxylic acid component for polyimides, but tetracarboxylic acid component having alicyclic structure is preferred because they provide a polyimide having high heat resistance. Particularly, preference is given to tetracarboxylic acid components in which A₁ or A₂ has six-membered alicyclic structure represented by general Formula (H4):

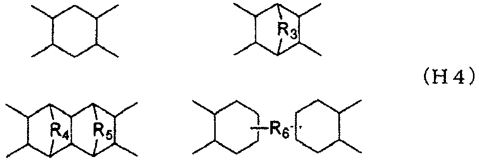

wherein, in Formulae (H4), R₃ to R₅ each independently represent a CH₂ group, a C₂H₄ group, an oxygen atom, or a sulfur atom; and R₆ represents a direct bond, a CH₂ group, a C(CH₃)₂ group, a SO₂ group, a Si(CH₃)₂ group, a C(CF₃)₂ group, an oxygen atom, or a sulfur atom. Among them, particularly preference is given to tetracarboxylic acid components of multi-alicyclic or bridge-cyclic ones because they provide polyimides having heat resistance and low coefficient of thermal expansion.

The examples of aliphatic tetracarboxylic acid components having six-membered alicyclic structure include cyclohexane-1, 2, 4, 5-tetracarboxylic acid, [1, 1'-bi(cyclohexane)]-3, 3', 4, 4'-tetracarboxylic acid, [1,1'-bi(cyclohexane)]-2, 3, 3', 4'-tetracarboxylic acid, [1, 1'-bi(cyclohexane)]-2, 2', 3, 3'-tetracarboxylic acid, 4, 4'-methylenebis(cyclohexane-1,2-dicarboxylic acid), 4, 4'-(propane-2, 2-diyl)bis(cyclohexane-1, 2-dicarboxylic acid), 4,4'-oxybis(cyclohexane-1, 2-dicarboxylic acid), 4, 4'-thiobis(cyclohexane-1,2-dicarboxylic acid), 4,4'- sulfonylbis(cyclohexane-1, 2-dicarboxylic acid, 4,4'-(dimethylsilanediyl)bis(cyclohexane-1,2-dicarboxylic acid), 4,4'-(tetrafluoropropane-2, 2-diyl)bis(cyclohexane-1, 2-dicarboxylic acid), and anhydrides of these.

Among these, preference is given to cyclohexane-1,2,4,5-tetracarboxylic acid, [1,1'-bi(cyclohexane)]-3,3',4,4'-tetracarboxylic acid, [1,1'-bi(cyclohexane)]-2,3,3',4'-tetracarboxylic acid, [1,1'-bi(cyclohexane)]-2,2',3,3'-tetracarboxylic acid and anhydrides of these.

Examples of multi-alicyclic or bridge-cyclic aliphatic tetracarboxylic acid components include octahydropentalene-1,3,4,6-tetracarboxylic acid, bicyclo[2.2.1]heptane-2,3,5,6-tetracarboxylic acid, 6-(carboxymethyl)bicyclo[2.2.1]heptane-2,3,5-tricarboxylic acid, bicyclo[2.2.2]octane-2,3,5,6-tetracarboxylic acid, bicyclo[2.2.2]octa-5-ene-2,3,7,8-tetracarboxylic acid, tricyclo[4.2.2.02,5]decane-3,4,7,8-tetracarboxylic acid, tricyclo[4.2.2.02,5]deca-7-ene-3,4,9,10-tetracarboxylic acid, 9-oxatricyclo[4.2.1.02,5]nonane-3,4,7,8-tetracarboxylic acid, decahydro-1,4:5,8-dimethanonaphthalene-2,3,6,7-tetracarboxylic acid and anhydride of these. Among them, preference is given to bicyclo[2.2.1] heptane-2,3,5,6-tetracarboxylic acid, bicyclo[2.2.2]octane-2,3,5,6-tetracarboxylic acid, decahydro-1,4:5,8-dimethanonaphthalene-2,3,6,7-tetracarboxylic acid and anhydrides of these because these are readily produced and they provide polyimides having excellent heat resistance.

The aromatic diamine component is not particularly limited and may be any aromatic diamine component generally employed as a diamine component for a polyimides, but aromatic diamine component wherein B₁ and B₂ are selected from the divalent aromatic groups represented by general Formulae (H5-1) to (H5-5) are preferred because they provide a polyimide having high heat resistance. Diamines wherein B₁ and B₂ are selected from the divalent aromatic groups represented by general Formulae (H5-3) to (H5-5) are particularly preferred because they provide a polyimide having low coefficient of thermal expansion.

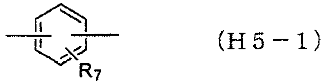

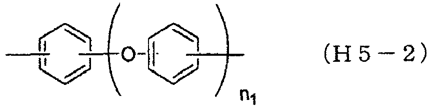

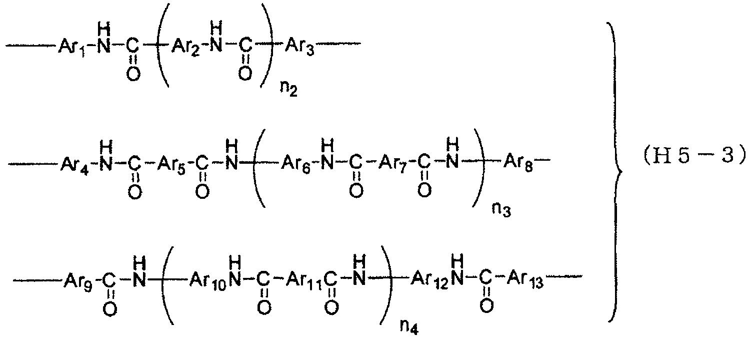

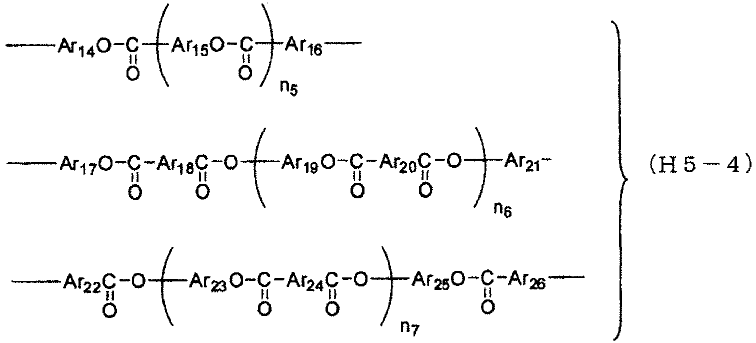

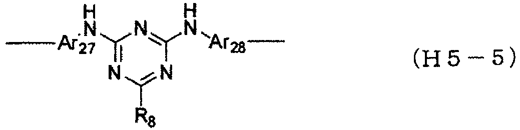

wherein, in general Formulae (H5-1) to (H5-5), R₇ represents hydrogen, a methyl group, or an ethyl group; R₈ is a monovalent organic group; Ar₁ to Ar₂₈ each independently represent a divalent group having an aromatic ring having 6 to 18 carbon atoms; n₁ represents an integer of 1 to 5; and n₂ to n₇ each independently represent an integer of 0 to 5.

The examples of the aromatic diamines represented by general Formula (H5-1) include p-phenylenediamine, m-phenylenediamine, o-phenylenediamine, 2,4-toluenediamine, 2,5-toluenediamine, 2,6-toluenediamine. Among these, p-phenylenediamine and 2,5-toluenediamine are preferred in view of particularly high heat resistance.

The examples of the aromatic diamines having ether linkage represented by general Formula (H5-2) include 4, 4'-diaminodiphenyl ether, 3, 4'-diaminodiphenyl ether, 1, 3-bis (4-aminophenoxy)benzene, 1,3-bis(3-aminophenoxy)benzene and derivatives of these. Among these, 4, 4'-diaminodiphenyl ether is preferred in view of particularly high heat resistance.

The examples of the aromatic diamines having amide linkage represented by general Formula (H5-3) include 4,4'- diaminobenzanilide, 3'-chloro-4, 4'-diaminobenzanilide, 2'-chloro-4, 4 -diaminobenzanilide, 2', 6'-dichloro-4,4'-diaminobenzanilide, 3'-methyl-4, 4'-diaminobenzanilide, 2'-methyl-4, 4'- diaminobenzanilide, 2', 6'-dimethyl-4, 4'-diaminobenzanilide, 3'-trifluoromethyl-4, 4'- diaminobenzanilide, 2'-trifluoromethyl-4, 4'-diaminobenzanilide, 3-chloro-4, 4'-diaminobenzanilide, 3-bromo-4, 4'-diaminobenzanilide, 3-methyl-4, 4'-diaminobenzanilide, 2-chloro-4, 4'-diaminobenzanilide, 2-bromo-4, 4'-diaminobenzanilide, 2-methyl-4, 4'-diaminobenzanilide, 4,3'-diaminobenzanilide, 4'-fluoro-4, 3'-diaminobenzanilide, 4'-chloro-4, 3'-diaminobenzanilide, 4'-bromo-4, 3'-diaminobenzanilide, 3, 4'-diaminobenzanilide, 4-chloro-3 ,4'-diaminobenzanilide, 4-methyl-3, 4'-diaminobenzanilide, N, N'-bis(4-aminophenyl)terephthalamide, N, N'-bis(4-aminophenyl)-2,5-dichloroterephthalamide, N, N'-bis(4-aminophenyl)-2,5-dimethylterephthalamide, N, N'-bis(4-aminophenyl)-2,3,5,6-tetrafluoroterephthalamide, N, N'-bis(4-aminophenyl)-2,3,5,6-tetrafluoroterephthalamide, N, N'-bis(4-aminophenyl)-2,3,5,6-tetrachloroterephthalamide, N, N'-bis(4-aminophenyl)-2,3,5,6-tetrabromoterephthalamide, N, N'-bis(4-aminophenyl)-4-bromoisophthalamide, N, N'-bis(4-aminophenyl)-5-tert-butylisophthalamide, N, N'-p-phenylenebis (p-aminobenzamide), N, N'-m-phenylenebis(p-aminobenzamide) and derivative of these. Among these, 4,4'-diaminobenzanilide, N, N'-bis(4-aminophenyl)terephthalamid and N, N'-p-phenylenebis (p-aminobenzamide) are preferred, and N, N'-bis(4-aminophenyl)terephthalamid and N, N'-p-phenylenebis (p-aminobenzamide) are more preferred because they provides polyimide having low coefficient of thermal expansion.

The examples of the aromatic diamines having ester linkage represented by general Formula (H5-4) include 4-aminophenyl-4-aminobenzoate, 3-aminophenyl-4-aminobenzoate, 4-aminophenyl-3-aminobenzoate, bis(4-aminophenyl)terephthalate, bis(4-aminophenyl)isophthalate, bis(4-aminophenyl)biphenyl-4,4'-dicarboxylate, 1,4-bis(4-aminobenzoyloxy)benzene, 1,3-bis(4-aminobenzoyloxy)benzene, biphenyl-4, 4'- diyl bis-(4-aminobenzoate) and derivative of these. Among these, 4-aminophenyl-4-aminobenzoate, bis(4-aminophenyl)isophthalate and 1,4-bis(4-aminobenzoyloxy)benzene are more preferred because they provide polyimides having low coefficient of thermal expansion, and 1,4-bis(4-aminobenzoyloxy)benzene is particularly preferred because it provides a polyimide having excellent light transmittance.

In general Formula (H5-5), the organic group represented by R₈ includes a hydrogen atom, an alkyl or aryl group having up to 20 carbon atoms, and an amino group optionally substituted with alkyl or aryl group having up to 20 carbon atoms. Specifically, the examples of aromatic diamines having triazine structure represented by general Formula (H5-5) include 2,4-bis(4-aminoanilino)-1,3,5-triazine, 2,4-bis(4-aminoanilino)-6-methyl-1,3,5-triazine, 2,4-bis(4-aminoanilino)-6-ethyl-1,3,5-triazine, 2,4-bis(4-aminoanilino)-6-phenyl-1,3,5-triazine, 2,4-bis(4-aminoanilino)-6-amino-1,3,5-triazine, 2,4-bis(4-aminoanilino)-6-methylamino-1,3,5-triazine, 2,4-bis(4-aminoanilino)-6-dimethylamino-1,3,5-triazine, 2,4-bis(4-aminoanilino)-6-ethylamino-1,3,5-triazine, 2,4-bis(4-aminoanilino)-6-diethylamino-1,3,5-triazine, 2,4-bis(4-aminoanilino)-6-anilino-1,3,5-triazine, and 2,4-bis(4-aminoanilino)-6-diphenylamino-1,3,5-triazine. Among these, preferred are 2,4-bis(4-aminoanilino)-6-amino-1,3,5-triazine, 2,4-bis(4-aminoanilino)-6-methylamino-1,3,5-triazine, 2,4-bis(4-aminoanilino)-6-ethylamino-1,3,5-triazine, 2,4-bis(4-aminoanilino)-6-anilino-1,3,5-triazine and more preferred are2,4-bis(4-aminoanilino)-6-anilino-1,3,5-triazine because they provide polyimides having low coefficient of thermal expansion.

The aliphatic diamine component is not particularly limited and may be any diamine component generally employed as an aliphatic diamine component for polyimides, but diamine component having divalent alicyclic structure is preferred because they provide a polyimide having high heat resistance. Particularly, preference is given to diamine components in which B₁ or B₂ have six-membered alicyclic structure represented by general Formula (H6):

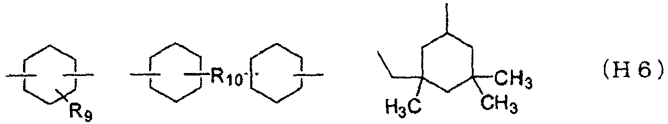

wherein, in general Formula (H6), R₉ represents hydrogen or a hydrocarbon group having 1 to 3 carbon atoms; and R₁₀ represents a direct bond, a CH₂ group, a C(CH₃)₂ group, a SO₂ group, a Si(CH₃)₂ group, a C(CF₃)₂ group, an oxygen atom, or a sulfur atom.

The preferred examples of the aromatic diamines having six-membered alicyclic structure represented by general Formula (H6) include 1,4-diaminocyclohexane, 1,4-diamino-2-methylcyclohexane, 1,4-diamine-2-ethylcyclohexane, 1,4-diamino-2-n-propylcyclohexane, 1,4-diamino-2-isopropylcyclohexane, 1,4-diamino-2-n-butylcyclohexane, 1,4-diamino-2-isobutylcyclohexane, 1,4-diamano-2-sec-butylcyclohexane, 1,4-diamino-2-tert-butylcyclohexane, 1,2-diaminocyclohexane, bi(cyclohexane)-4,4'-diamine, 4,4'-methylenedicyclohexaneamine, 4,4'-(propane-2,2-diyl)dicyclohexaneamine, 4,4'- sulfonyldicyclohexaneamine, 4,4'-(dimethylsilanediyl)dicyclohexaneamine, 4,4'-(perfluoropropane-2,2-diyl)dicyclohexaneamine, 4,4'-oxydicyclohexaneamine, 4,4'-thiodicyclohexaneamine and isophoronediamine. Particularly, more preferred is 1,4-diaminocyclohexane because it provides polyimides having low coefficient of thermal expansion. Furthermore, 1, 4-steric configuration of the diamines having 1, 4-cyclohexane structure is not particularly limited, but it is preferably trans-configuration. Cis-configuration tends lead a drawback such as coloring.

In general Formula (H1) of the polyimide precursor varnish produced in the present invention, R₁ and R₂ each independently represent a hydrogen atom, an alkyl group having 1 to 6 carbon atoms, or an alkylsilyl group having 3 to 9 carbon atoms.

In case that both of R₁ and R₂ represent hydrogen atom, it is preferable in that the production cost is low.

In case that R₁ and R₂ each independently represent methyl group, ethyl group, propyl group or isopropyl group, it is preferable in that the polyimide precursor varnish is stable in its viscosity and the obtained polyimide is excellent in heat resistance.

In case that R₁ and R₂ each independently represent trimethylsilyl group, t-butyldimethylsilyl group or triisopropylsilyl group, it is preferable in that the problem such as precipitation and the like during the production of the polyimide precursor varnish is improved and that the obtained polyimide is excellent in heat resistance.

In addition, the polyimide varnish produced in the present invention is preferable in that it enables the formation of polyimide film at lower temperature than the case of using the polyimide precursor varnish.

The varnishes produced in the present invention can be classified based on the chemical structures thereof into 1) polyamic acid varnishes, 2) polyamic acid ester varnishes, 3) polyamic acid silyl ester varnishes, and 4) polyimide varnishes. The varnishes classified into groups 1) to 3) contain polyimide precursors and are classified based on the chemical structures of R₁ and R₂ in general Formula (H1). The varnishes classified into group 4) contain polyimides represented by general Formula (H2). Each varnish classified based on the chemical structure can be readily produced by the following polymerization, but the methods of producing the polyimide precursor varnish or the polyimide varnish of the present invention are not limited to the following methods.

### 1) Method of producing polyamic acid varnish

A polyimide precursor is prepared by dissolving a diamine in an organic solvent, gradually adding a tetracarboxylic dianhydride to the resulting solution with stirring, and stirring the mixture in a temperature range of 0 to 120°C, preferably 5 to 80°C, for 1 to 72 hours. In a reaction at 80°C or more, the molecular weight varies depending on the temperature history in the polymerization, and the imidization is accelerated by the heat. Accordingly, the polyimide precursor may not be stably produced.

### 2) Method of producing polyamic acid ester varnish

A diester dicarboxylic acid chloride is prepared by reacting a tetracarboxylic dianhydride with an appropriate alcohol and reacting the resulting diester dicarboxylic acid with a chlorinating agent (e.g., thionyl chloride or oxalyl chloride). A polyimide precursor is prepared by stirring the diester dicarboxylic acid chloride and a diamine in a temperature range of -20 to 120°C, preferably -5 to 80°C, for 1 to 72 hours. In a reaction at 80°C or more, the molecular weight varies depending on the temperature history in the polymerization, and the imidization is accelerated by the heat. Accordingly, the polyimide precursor may not be stably produced. A polyimide precursor can also be readily prepared by dehydration condensation of a diester dicarboxylic acid and a diamine using, for example, a phosphorus condensing agent or a carbodiimide condensing agent. Since the polyimide precursor prepared by this process is stable, for example, even purification by reprecipitation from a solvent such as water or alcohol can be performed.

### 3) Method of producing polyamic acid silyl ester varnish

A silylated diamine is prepared by reacting a diamine and a silylating agent in advance (optionally, silylated diamine is purified by, for example, distillation). A polyimide precursor is prepared by dissolving the silylated diamine in a dehydrated solvent, gradually adding a tetracarboxylic dianhydride thereto with stirring, and stirring the mixture in a temperature range of 0 to 120°C, preferably 5 to 80°C for 1 to 72 hours. In a reaction at 80°C or more, the molecular weight varies depending on the temperature history in the polymerization, and the imidization is accelerated by the heat. Accordingly, the polyimide precursor may not be stably produced. Here, the use of a chlorine-free silylating agent does not require purification of the silylated diamine and is therefore preferable. Examples of the silylating agent not containing chlorine atoms include N,O-bis(trimethylsilyl)trifluoroacetamide, N,O-bis(trimethylsilyl)acetamide, and hexamethyldisilazane. Furthermore, N,O-bis(trimethylsilyl)acetamide and hexamethyldisilazane are preferable because they do not contain fluorine atoms and inexpensive. In order to facilitate the silylation of the diamine, an amine catalyst such as pyridine, piperidine, or triethylamine may be used. The catalyst can be also used as the polymerization catalyst of the polyimide precursor as it is.

### 4) Production of polyimide varnish

A polyimide varnish is prepared by preparing a polyimide precursor in any one of groups 1) to 3) in advance or mixing a tetracarboxylic acid component, a diamine component, and a solvent; and performing thermal imidization through heating at 150°C or more or chemical imidization with a chemical imidization agent (e.g., an acid anhydride such as acetic anhydride or an amine compound such as pyridine or isoquinoline). In the thermal imidization, the reaction is preferably performed in a nitrogen atmosphere for reducing coloring of the solvent.

All of the above-mentioned methods of production can be suitably performed in an organic solvent and can therefore readily prepare the polyimide precursor varnish or the polyimide varnish of the present invention.

In all of these methods of production, the molar ratio of the tetracarboxylic acid component to the diamine component can be appropriately determined depending on the required viscosity of the polyimide precursor and is preferably 0.90 to 1.10 and more preferably 0.95 to 1.05.

In the method of production of the present invention, when the molar ratio of the tetracarboxylic acid component to the diamine component is an excess molar ratio of the diamine component, a carboxylic acid derivative may be optionally added in an amount approximately corresponding to the number of moles of the excess diamine such that the molar proportion of the tetracarboxylic acid component is approximately equivalent to the molar proportion of the diamine component. The carboxylic acid derivative here is selected from tetracarboxylic acids that substantially do not increase the viscosity of the polyimide precursor solution (i.e., substantially do not participate in extension of molecular chain), tricarboxylic acids functioning as chain terminators, their anhydrides, dicarboxylic acids, and their anhydrides.

Examples of the usable carboxylic acid derivative include tetracarboxylic acids such as 3,3',4,4'-biphenyltetracarboxylic acid, 2,3,3',4'-biphenyltetracarboxylic acid, 2,2',3,3'-biphenyltetracarboxylic acid, 1,2,3,4-butanetetracarboxylic acid, and benzene-1,2,4,5-tetracarboxylic acid; tricarboxylic acids such as trimellitic acid and cyclohexane-1,2,4-tricarboxylic acid and acid anhydrides thereof; and dicarboxylic acids such as phthalic acid, tetrahydrophthalic acid, cis-norbornene-endo-2,3-dicarboxylic acid, cyclohexanedicarboxylic acid, succinic acid, and maleic acid and acid anhydrides thereof. The use of these carboxylic acid derivatives can prevent thermal coloring and thermal degradation during the heating. In particular, tetracarboxylic acid derivatives such as biphenyltetracarboxylic acid and carboxylic acid derivatives having reactive functional groups react in imidization to increase heat resistance and are therefore preferable.

In the varnish produced by the present invention, the total amount of the tetracarboxylic acid component and the diamine component is preferably 5% by mass or more, more preferably 10% by mass or more, and most preferably 15% by mass or more and also usually 60% by mass or less and preferably 50% by mass or less, based on the total amount of the organic solvent, the tetracarboxylic acid component and the diamine component. A too low concentration may make it difficult to control the thickness of the resulting polyimide film.

In the method of producing a polyimide precursor varnish or a polyimide varnish of the present invention, the polymerized polyimide precursor or polyimide may be diluted with an organic solvent. The organic solvent used for the dilution also preferably satisfies at least one requirement selected from the above-mentioned requirements (a) to (f).

In the method of producing a polyimide precursor varnish or a polyimide varnish of the present invention, an additive such as a chemical imidization agent (an acid anhydride such as acetic anhydride or an amine compound such as pyridine or isoquinoline), an antioxidant, a filler, a dye, a pigment, a coupling agent such as a silane coupling agent, a primer, a fire-retarding material, an antifoaming agent, a leveling agent, a rheology-controlling agent (flow assistant), a release agent, etc. can be optionally used. These additives may be added after a polyimide precursor varnish or a polyimide varnish using an organic solvent having high purity is produced. Alternatively, the additives may be dissolved or dispersed in an organic solvent having high purity (e.g., a purity of 99.8% or more) in advance and added before the production of a polyimide precursor varnish or a polyimide varnish.

The varnish produced by the method of the present invention preferably has a light transmittance at 400 nm of 70% or more, more preferably 75% or more, and most preferably 80% or more when formed into a polyimide film having a thickness of 10 µm.

The varnish produced by the method of the present invention provides a polyimide having reduced coloring and excellent light transparency and is therefore suitable for optical use, for example, as an optical material that is used for transmitting or reflecting light.

A polyimide can be produced from the varnish produced by the method of the present invention as follows. In the case of a polyimide precursor varnish, a polyimide can be suitably produced through a cyclodehydration reaction (imidization reaction) of the polyimide precursor. The process of imidization is not particularly limited, and a known thermal imidization or chemical imidization can be suitably employed. In the case of a polyimide varnish, a polyimide can be prepared by evaporating the organic solvent contained in the polyimide varnish by heating or reducing the pressure or precipitating the polyimide. The form of the resulting polyimide is not particularly limited, and preferred examples of the form include films, laminates of polyimide films and other base materials, coating films, powders, hollow beads, molded products, and foamed products.

### EXAMPLES

The present invention will now be described in more detail based on the following examples and comparative examples. However, the present invention is not limited to the following examples.

### «Examples of PART A »

In each of the following examples, evaluation was carried out based on the following methods.

### Evaluation of Polyimide Precursor

### [Varnish Solid Content]

One gram of polyimide precursor solution was weighed into an aluminum dish, and heated for 2 hours in a 200°C hot air circulating oven to remove the non-solid content. The varnish solid content (heating residue mass%) was determined from the residual matter.

### [Rotational Viscosity]

The viscosity of the polyimide precursor solution at a temperature of 25°C and a shear rate of 20 sec⁻¹ was determined using a TV-22 E-type rotary viscometer manufactured by Toki Sangyo Co., Ltd.

### [Logarithmic Viscosity]

The logarithmic viscosity was determined by measuring a 0.5 g/dL solution of the polyimide precursor in N,N-dimethylacetamide at 30°C using an Ubbelohde viscometer.

### [Solvent Purity]

The solvent purity was measured under the following conditions using a GC-2010 manufactured by Shimadzu Corporation. The purity (GC) was determined from the peak surface area fraction.
Column: DB-FFAP manufactured by J&W, 0.53 mm ID x 30 m Temperature: 40°C (5 minutes holding) + 40°C to 250°C (10 minutes/minutes) + 250°C (9 minutes holding)
Inlet temperature: 240°C
Detector temperature: 260°C
Carrier gas: Helium (10 ml/minute)
Injection amount: 1 µL

Evaluation of Polyimide Film

### [Light Transmittance]

The light transmittance at 400 nm of a polyimide film with a thickness of about 10 µm was measured using a MCPD-300 manufactured by Otsuka Electronics Co., Ltd.

### [Elastic Modulus and Elongation at break]

The initial elastic modulus and elongation at break for a chuck interval of 30 mm and a tension rate of 2 mm/min were measured using a Tensilon manufactured by Orientec Co., Ltd., on a test piece produced by punching a polyimide film with a thickness of about 10 µm into an IEC450 standard dumbbell shape.

### [Coefficient of Thermal Expansion (CTE)]

A test piece was produced by cutting a polyimide film with a thickness of about 10 µm into a strip with a width of 4 mm. Then, using a TMA-50 manufactured by Shimadzu Corporation, the temperature of the test piece was increased to 300°C at a rate of temperature increase of 20°C/min with a chuck interval of 15 mm and a load of 2 g. The average coefficient of thermal expansion from 50°C to 200°C was determined from the obtained TMA curve.

### [Dynamic Viscoelasticity Measurement]

A test piece was produced by cutting a polyimide film with a thickness of about 10 µm into a strip. Then, using a solid viscoelasticity analyzer RSAIII manufactured by TA Instruments, dynamic viscoelasticity was measured under the following conditions.
Measurement mode: Tension mode
Sweep type: Temperature step 3°C/min, Soak time 0.5 min
Frequency: 10 Hz (62.8 rad/sec)
Strain: 0.2 to 2%
Temperature range: 25°C until measurement limit
Atmosphere: In a nitrogen flow

### [Example A1]

In a reaction vessel, 10.82g (0.0947 mol) of trans-1,4-diaminocyclohexane (may be referred as t-DACH below) was charged and dissolved in 313.0g of N,N-dimethylacetamide (may be referred as t-DMAc below) that had been dehydrated using a molecular sieve. To the solution, 26.48g (0.090 mol) of 3,3',4,4'-biphenyltetracarboxylic dianhydride (may be referred as s-BPDA below) and 1.394g (0.0047 mol) of 2,3,3',4'-biphenyltetracarboxylic dianhydride (may be referred as a-BPDA below) was gradually added, and the resultant mixture was heated to 120°C. When the start of dissolution of salts in about 5 minutes was confirmed, the mixture was cooled rapidly to room temperature and held at room temperature for 8 hours with stirring to obtain a uniform and viscous co-polyimide precursor solution composition.

The obtained polyimide precursor solution composition was applied on a glass substrate, and thermally imidized by heating at 120 °C for 1 hour, at 150 °C for 30 minutes, at 200 °C for 30 minutes and finally up to 400 °C while holding it on the substrate to obtain a colorless transparent co-polyimide/glass laminate. Thus obtained co-polyimide/glass laminate was immersed in water for delamination to obtain a co-polyimide film with thickness of about 10 µm. Measurement results of properties of the film are shown in Table A1.

### [Example A2]

In a reaction vessel, 6.851g (0.06 mol) of trans-1,4-diaminocyclohexane was charged and dissolved in 220.5 g of N,N-dimethylacetamide that had been dehydrated using a molecular sieve. To the solution, 15.89g (0.054 mol) of 3,3',4,4'-biphenyltetracarboxylic dianhydride and 1.765g (0.006 mol) of 2,3,3',4'-biphenyltetracarboxylic dianhydride was gradually added, and the resultant mixture was heated to 120°C. When the start of dissolution of salts in about 5 minutes was confirmed, the mixture was cooled rapidly to room temperature and held at room temperature for 8 hours with stirring to obtain a uniform and viscous co-polyimide precursor solution composition.

The obtained polyimide precursor solution composition was applied on a glass substrate, and thermally imidized by heating at 120 °C for 1 hour, at 150 °C for 30 minutes, at 200 °C for 30 minutes and finally up to 400 °C while holding it on the substrate to obtain a colorless transparent co-polyimide/glass laminate. Thus obtained co-polyimide/glass laminate was immersed in water for delamination to obtain a co-polyimide film with thickness of about 10 µm. Measurement results of properties of the film are shown in Table A1.

### [Example A3]

In a reaction vessel, 2.28g(0.02 mol) of trans-1,4-diaminocyclohexane was charged and dissolved in 73.51 g of N,N-dimethylacetamide (hereinafter, high purity DMAc of 99.99 % purity (GC) was used unless otherwise mentioned) that had been dehydrated using a molecular sieve. To the solution, 4.71g (0.016 mol) of 3,3',4,4'-biphenyltetracarboxylic dianhydride and 1.18g (0.004 mol) of 2,3,3',4'-biphenyltetracarboxylic dianhydride was gradually added, and the resultant mixture was stirred at 25 °C for 24 hours to obtain a uniform and viscous polyimide precursor solution composition.

The obtained polyimide precursor solution composition was applied on a glass substrate, and thermally imidized by heating at 120 °C for 1 hour, at 150 °C for 30 minutes, at 200 °C for 30 minutes and finally up to 400 °C while holding it on the substrate to obtain a colorless transparent co-polyimide/glass laminate. Thus obtained co-polyimide/glass laminate was immersed in water for delamination to obtain a co-polyimide film with thickness of about 10 µm. Measurement results of properties of the film are shown in Table A1.

### [Example A4]

3.00g (0.026 mol) of trans-1,4-diaminocyclohexane was dissolved in 52.39 g of N,N-dimethylacetamide. To the solution, 6.18g (0.021 mol) of 3,3',4,4'-biphenyltetracarboxylic dianhydride and 1.55g (0.005 mol) of 2,3,3',4'-biphenyltetracarboxylic dianhydride was gradually added and stirred at 40 °C, and all solids were dissolved after 80 minutes. The mixture was further stirred for 8 hours to obtain a viscous polyimide precursor.

### [Example A5]

3.00g (0.026 mol) of trans-1,4-diaminocyclohexane was dissolved in 52.38 g of N-methylpyrrolidone (hereinafter, high purity N-methylpyrrolidone of 99.96 % purity (GC) was used unless otherwise mentioned and may be referred as NMP below). To the solution, 6.18g (0.021 mol) of 3,3',4,4'-biphenyltetracarboxylic dianhydride and 1.55g (0.005 mol) of 2,3,3',4'-biphenyltetracarboxylic dianhydride was gradually added and stirred at 40 °C and all solids were dissolved after 135 minutes. The mixture was further stirred for 8 hours to obtain a viscous polyimide precursor.

### [Example A6]

In a reaction vessel, 3.00g (0.026 mol) of trans-1,4-diaminocyclohexane was charged and dissolved in 60.35 g of N,N-dimethylacetamide. To the mixture, 5.55g (0.0273 mol) of N,O-bis(trimethylsilyl)acetamide was added and stirred at 80 °C for 2 hours to perform silylation. After cooling the solution to 40 °C, 6.77g (0.023 mol) of 3,3',4,4'-biphenyltetracarboxylic dianhydride and 0.88g(0.003 mol) of 2,3,3',4'-biphenyltetracarboxylic dianhydride was added. The mixture was stirred at 40 °C and all solids were dissolved in 1 hour. The mixture was further stirred for 8 hours to obtain a uniform and viscous co-polyimide precursor solution composition.

The obtained polyimide precursor solution was applied on a glass substrate, and thermally imidized by heating at 120 °C for 1 hour, at 150 °C for 30 minutes, at 200 °C for 30 minutes and at 350 °C for 1 hour while holding it on the substrate under nitrogen atmosphere (oxygen concentration is 200 ppm or less) to obtain a colorless transparent co-polyimide/glass laminate. Thus obtained co-polyimide/glass laminate was immersed in water for delamination to obtain a co-polyimide film with thickness of about 10 µm. Measurement results of properties of the film are shown in Table A1.

### [Comparative Example A1]

In a reaction vessel, 2.284g (0.02 mol) of trans-1,4-diaminocyclohexane was charged and dissolved in 73.51 g of N,N-dimethylacetamide (general-purpose product) that had been dehydrated using a molecular sieve. To the solution, 5.884g(0.02 mol) of 3,3',4,4'-biphenyltetracarboxylic dianhydride was gradually added, and the resultant mixture was heated to 120°C. When the start of dissolution of salts in about 5 minutes was confirmed, the mixture was cooled rapidly to room temperature and held at room temperature for 8 hours with stirring. White precipitations were observed on the wall of the reaction vessel, but a uniform and viscous polyimide precursor solution composition was obtained by pressure filtration.

The obtained polyimide precursor solution was applied on a glass substrate, and thermally imidized by heating at 120 °C for 1 hour, at 150 °C for 30 minutes, at 200 °C for 30 minutes and finally up to 400 °C while holding it on the substrate to obtain a colorless transparent polyimide film with thickness of about 10 µm. Measurement results of properties of the film are shown in Table A1.

### [Comparative Example A2]

In a reaction vessel, 2.284 g (0.02 mol) of trans-1,4-diaminocyclohexane was charged and dissolved in 73.51 g of N,N-dimethylacetamide that had been dehydrated using a molecular sieve. To the solution, 5.884 g (0.02 mol) of 3,3',4,4'-biphenyltetracarboxylic dianhydride was gradually added, and the resultant mixture was stirred at 25 °C for 48 hours. In the solution, white solid insoluble substances were observed and a uniform polyimide precursor solution was not obtained.

### [Comparative Example A3]

3.00g (0.026 mol) of trans-1,4-diaminocyclohexane was dissolved in 52.39 g of N,N-dimethylacetamide. To the solution, 7.73g (0.026 mol) of 3,3',4,4'-biphenyltetracarboxylic dianhydride was added and stirred at 40 °C and all solids were dissolved after 16 hours. The mixture was further stirred for 8 hours to obtain a viscous polyimide precursor.

### [Comparative Example A4]

3.00g (0.026 mol) of trans-1,4-diaminocyclohexane was dissolved in 52.38 g of N-methylpyrrolidone (purity (GC) was 99.62 %). To the solution, 7.73g (0.026 mol) of 3,3',4,4'-biphenyltetracarboxylic dianhydride was added and stirred at 40 °C and all solids were dissolved after 11 hours. The mixture was further stirred for 8 hours to obtain a viscous polyimide precursor.

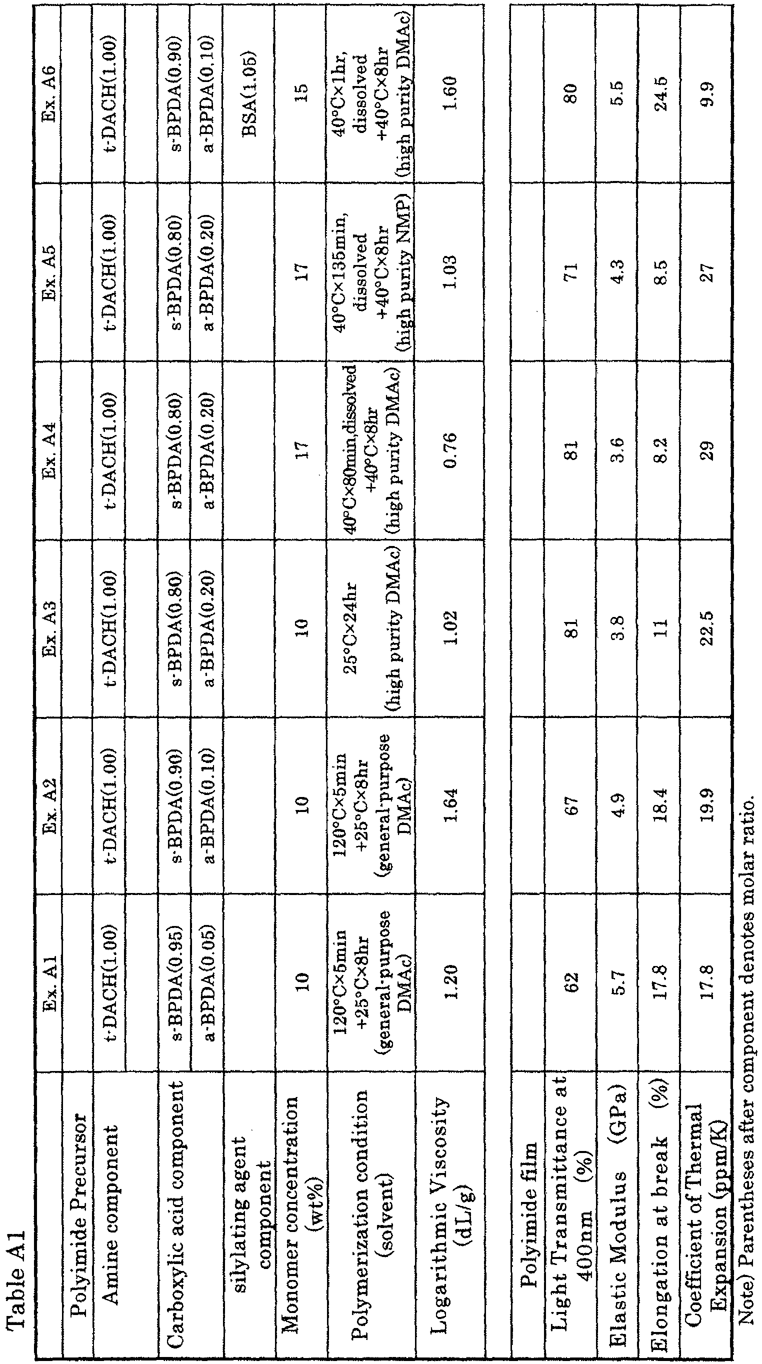

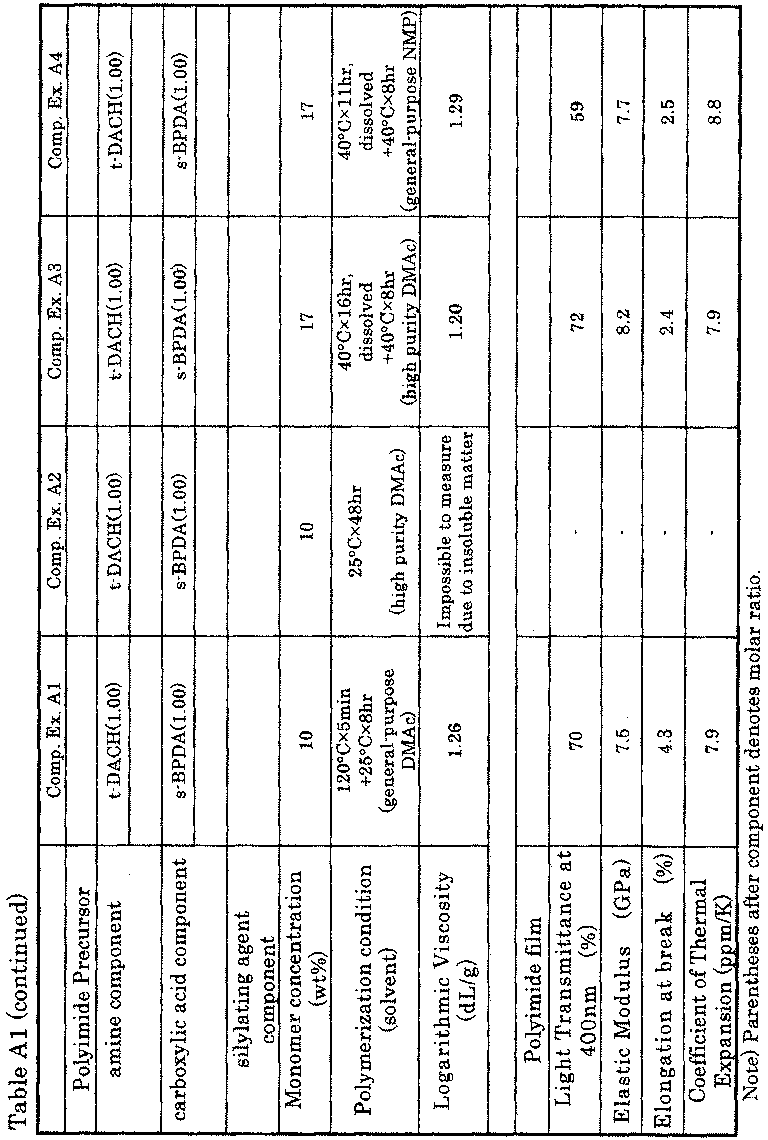

As can be seen from the results shown in Table A1, the co-polyimide precursor according to the present invention can be polymerized even under the mild conditions of 25°C by copolymerization. On the other hand, it was confirmed that a uniform solution could be obtained in a short time at a polymerization temperature of 40°C. In addition, the co-polyimide obtained from this polyimide precursor has, in addition to excellent light transmittance and a low linear coefficient of thermal expansion when formed as a film, a sufficiently large elongation at break compared with Comparative Example A1.
Moreover, it can also be seen that the co-polyimide precursor of a polyamic acid silyl ester type co-polyimide precursor (Example A6) provides a film having even lower linear coefficient of thermal expansion, compared with a co-polyimide precursor of a polyamic acid (Example A2).

Abbreviation, purity, pretreatment and the like of the raw materials used in the respective following examples are as follows (in case that pretreatment is not mentioned, materials were used without pretreatment).
t-DACH: the material used was obtained by purifying trans-1,4-diaminocyclohexane with purity 99.1% (GC), by recrystallization or sublimation.
t-1,2-DACH: trans-1,2-diaminocyclohexane with purity 99.9% (GC) was used.
s-BPDA: the material used was obtained by adding equal amount by mass of N-methyl-2-pyrrolidone to 3,3',4,4'-biphenyltetracarboxylic dianhydride {having purity 99.9% (purity determined by HPLC analysis of ring-opened 3,3',4,4'-biphenyltetracarboxylic acid), acid anhydride ratio 99.8%, Na, K, Ca, Al, Cu, Si: each < 0.1 ppm, Fe: 0.1 ppm, Cl: < 1 ppm}; stirring the mixture at room temperature for 3 hours; and recovering the undissolved powder and drying it in vacuo.
a-BPDA: the material used was obtained by adding equal amount by mass of acetone to 2,3,3',4'-biphenyltetracarboxylic dianhydride {having purity 99.6% (purity determined by HPLC analysis of ring-opened 2,3,3',4'-biphenyltetracarboxylic acid), acid anhydride ratio 99.5%, Na, K, Al, Cu, Si: each < 0.1 ppm, Ca, Fe: each 0.1 ppm, Cl: < 1 ppm}; stirring the mixture at room temperature for 3 hours; and recovering the undissolved powder and drying it in vacuo.
i-BPDA: the material used was obtained by adding equal amount by mass of N-methyl-2-pyrrolidone to 2,2',3,3'-biphenyltetracarboxylic dianhydride {having purity 99.9% (purity determined by HPLC analysis of ring-opened 2,2',3,3'-biphenyltetracarboxylic acid), acid anhydride ratio 99%}; stirring the mixture at room temperature for 3 hours; and recovering the undissolved powder and drying it in vacuo.
6FDA: 4,4'-(2,2-hexafluoroisopropylene)diphthalic dianhydride of purity 99.77 % (purity determined by H-NMR) was used.
ODPA: 4,4'-oxydiphthalic dianhydride with purity 99.9% (purity determined by HPLC analysis of ring-opened 4,4'-oxydiphthalic acid), acid anhydride ratio 99.7 % was used.
DPSDA: 4,4'- (dimethylsiladiyl)diphthalic dianhydride with purity 99.8 % (purity determined by HPLC) was used.
BTDA: 3,3',4,4'-benzophenone carboxylic dianhydride with purity 97 % or more was used.
PMDA: Pyromellitic dianhydride with purity 97 % or more was recrystallized and used.
s-BPTA: 3,3',4,4'- biphenyltetracarboxylic acid.
DMAc: N,N-dimethylacetamide used was a product purified by distillation and high purity product with purity (GC) of 99.99%.
NMP: N-methyl-2-pyrrolidone used was high purity product with purity 99.96%, and general-purpose product with purity 99.62 % (GC).

### [Example A7]

In a reaction vessel purged with nitrogen gas, 1.40 g (12.2 mmol) of t-DACH was charged and 36.6 g of N,N-dimethylacetamide that had been dehydrated using a molecular sieve was added and heated to 60°C to dissolve it. To the solution, 3.46 g (11.8 mmol) of s-BPDA and 0.09 g (0.3 mmol) of a-BPDA was gradually charged, and the resultant mixture was heated to 70°C and stirred. When the rotational viscosity exceeded 5 Pa·sec, 0.03 g (0.1 mmol) of s-BPTA was added, and the mixture was stirred for a further 2 hours to obtain a uniform and viscous polyimide precursor solution. Measurement results of properties of the polyimide precursor solution are shown in Table A2. The polyimide precursor solution was filtered using a PTFE membrane filter, and used to produce a film.

The obtained polyimide precursor solution was applied on a glass substrate, and thermally imidized by heating at 120 °C for 1 hour, at 150 °C for 30 minutes, at 200 °C for 30 minutes and at 350 °C for 3 minutes while holding it on the substrate under nitrogen atmosphere (oxygen concentration is 200 ppm) to obtain a colorless transparent co-polyimide/glass laminate. Thus obtained co-polyimide/glass laminate was immersed in water for delamination to obtain a co-polyimide film with thickness of about 10 µm. Measurement results of properties of the film are shown in Table A2.

### [Example A8]

In a reaction vessel purged with nitrogen gas, 1.40 g (12.2 mmol) of t-DACH as a diamine component was charged and dissolved in N,N-dimethylacetamide (28.4 g) that had been dehydrated using a molecular sieve of such an amount that the feeding amount of monomers (total amount of diamine component and carboxylic acid component) is 15 % by mass. The solution was heated to 50 °C, to which 3.24g (11.0 mol) of s-BPDA and 0.35 g (1.2 mmol) of a-BPDA was gradually added. The mixture was stirred at 70 °C for 8 hours to obtain a uniform and viscous polyimide precursor solution. Measurement results of properties of the polyimide precursor solution are shown in Table A2. The polyimide precursor solution was filtered using a PTFE membrane filter, and used to produce a film.

The obtained polyimide precursor solution was applied on a glass substrate, and thermally imidized by heating at 120 °C for 1 hour, at 150 °C for 30 minutes, at 200 °C for 30 minutes, then heating up and at 350 °C for 5 minutes while holding it on the substrate under nitrogen atmosphere (oxygen concentration is 200 ppm) to obtain a colorless transparent polyimide/glass laminate. Thus obtained polyimide/glass laminate was immersed in water for delamination to obtain a polyimide film with thickness of about 10 µm. Measurement results of properties of the film are shown in Table A2.

### [Examples A9 to A15]

Polyimide precursor solutions and co-polyimide films were obtained in the same manner as Example A8 except that diamine component and carboxylic acid component were used in an amount as indicated in Table A2, and N,N-dimethylacetamide is used in such an amount that the feeding amount of monomers (total amount of diamine component and carboxylic acid component) is 15 % by mass. Measurement results of properties of the polyimide precursor solutions and co-polyimide films are shown in Table A2.

### [Examples A16 to A17]

Polyimide precursor solutions and co-polyimide films were obtained in the same manner as Example A8 except that diamine component and carboxylic acid component were used in an amount as indicated in Table A2, and N-methylpyrrolidone with purity 99.96% measured by GC analysis and N-methylpyrrolidone with purity 99.62 %measured by GC analysis were used as solvents in Example A16 and Example A17, respectively, in such amounts that the feeding amount of monomers (total amount of diamine component and carboxylic acid component) is 15 % by mass. Measurement results of properties of the polyimide precursor solutions and co-polyimide films are shown in Table A2.

### [Comparative Example A5]

In a reaction vessel purged with nitrogen gas, 10 mmol (1.14g) of t-DACH as a diamine component was charged and dissolved in N,N-dimethylacetamide (22.7 g) that had been dehydrated using a molecular sieve of such an amount that the feeding amount of monomers (total amount of diamine component and carboxylic acid component) is 15 % by mass. To the solution, 9 mmol (2.65g) of s-BPDA and 1 mmol (0.218g) of PMDA was gradually added and heated to 50 °C and stirred for 12 hours. In the solution, white solid insoluble substances were observed and a uniform polyimide precursor solution was not obtained.

**Table A2**

| | | Ex. A7 | Ex. A8 | Ex. A9 | Ex. A10 | Ex. A11 | Ex. A12 | Ex. A13 | Ex. A14 | Ex. A15 | Ex. A16 | Ex. A17 | Comp. Ex. A5 |
|---|---|---|---|---|---|---|---|---|---|---|---|---|---|
| Polyimide Precursor | | | | | | | | | | | | | |
| Amine component (mmol) | 1,4-t-DACH | 12.2 | 12.2 | 12.2 | 12.2 | 12.2 | 12.2 | 12.2 | 12.2 | 11 | 12.2 | 12.2 | 10 |
| | 1,2-t-DACH | | | | | | | | | 1.2 | | | |
| Carboxylic acid component (mmol) | s-BPDA | 11.8 | 11 | 11 | 11 | 11 | 11 | 11 | 11 | 11.9 | 11.9 | 11.9 | 9 |
| | a-BPDA | 0.3 | 1.2 | | | | | | 0.6 | 0.3 | 0.3 | 0.3 | |
| | i-BPDA | | | 1.2 | | | | | | | | | |
| | 6FDA | | | | 1.2 | | | | | | | | |
| | ODPA | | | | | 1.2 | | | | | | | |
| | DPSDA | | | | | | 1.2 | | | | | | |
| | BTDA | | | | | | | 1.2 | | | | | |
| | PMDA | | | | | | | | 0.6 | | | | 1 |
| | s-BPTA | 0.1 | | | | | | | | | | | |
| Varnish solid content (wt%) | | 11 | 14 | 14 | 12 | 13 | 14 | 13 | 11 | 14 | 11 | 11 | |
| Rotational Viscosity (Pa sec) | | 8.0 | 165 | 51 | 30 | 43 | 20 | 1.8 | 1.4 | 70 | 7.3 | 3.9 | |
| Logarithmic Viscosity (dL/g) | | 1.32 | 1.60 | 1.36 | 1.54 | 1.42 | 1.24 | 0.88 | 0.98 | 1.45 | 1.24 | 1.05 | Insoluble matter |
| | | | | | | | | | | | | | |
| Polyimide film | | | | | | | | | | | | | |
| Light Transmittance at 400nm (%) | | 80 | 80 | 81 | 80 | 71 | 81 | 60 | 81 | 75 | 73 | 67 | |
| Elastic Modulus (GPa) | | 6.4 | 5.5 | 4.7 | 5.5 | 6.8 | 5.8 | 6.2 | 6.4 | 5.4 | 7.0 | 7.8 | |
| Elongation at break (%) | | 17 | 16 | 13 | 10 | 9 | 10 | 11 | 9 | 9 | 12 | 10 | |
| Coefficient of Thermal Expansion (ppm/K) | | 8 | 14 | 19 | 21 | 12 | 20 | 11 | 10 | 18 | 16 | 16 | |

As can be seen from the results shown in Table A2, the co-polyimide obtained from a polyimide precursor according to the present invention has, in addition to excellent light transmittance and a low linear coefficient of thermal expansion, a sufficiently large elongation at break compared with Comparative Example A1.
Further, in Comparative Example A5 in which s-BPDA and PMDA were used as the carboxylic acid component, a uniform polyimide precursor solution was not obtained. In contrast, in Example A14 a uniform polyimide precursor solution was obtained due to performing the copolymerization with, in addition to s-BPDA and a-BPDA, PMDA as a third carboxylic acid component.
Compared with Example A17 in which a solvent having a low purity (GC) is used, higher light transmittance was achieved for examples in which a high-purity solvent (a comparison between systems using the same raw material monomers) was used.

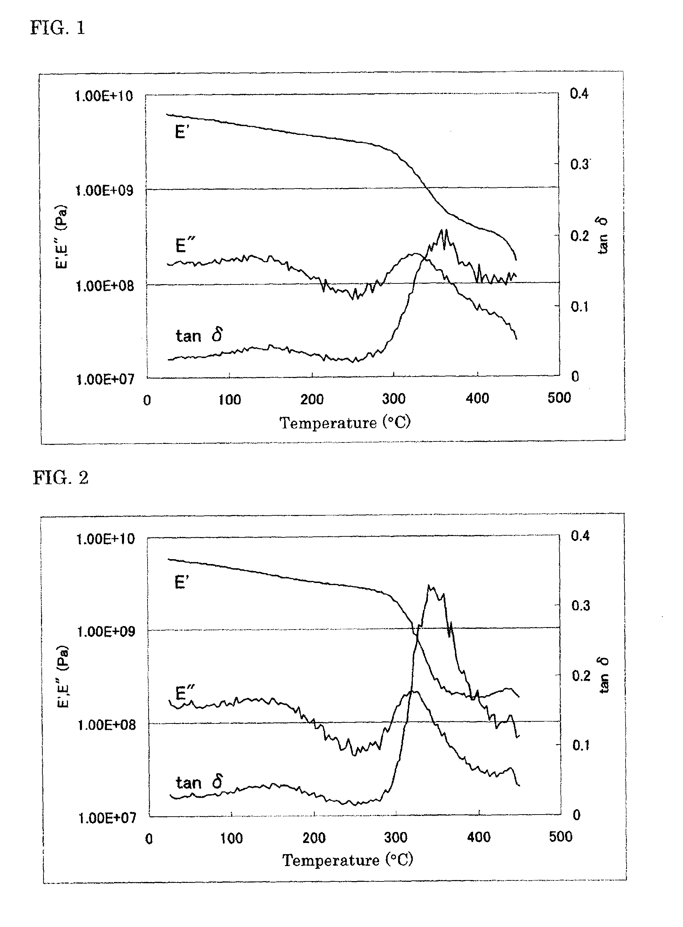

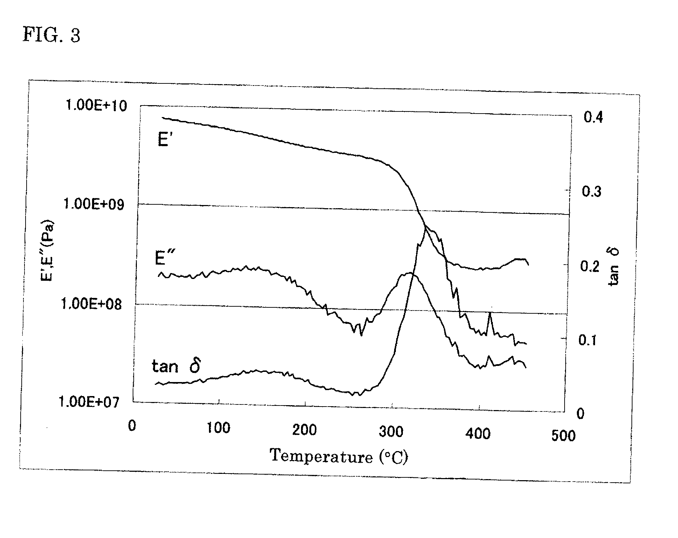

The results (storage elastic modulus E', loss elastic modulus E", and tan δ) obtained by measuring the dynamic viscoelasticity of the polyimide films obtained in Examples A8, A9, and A14 are shown in Figures 1 to 3, respectively, and based on these results, the glass transition temperature determined from the maximum point of tan δ, the minimum storage elastic modulus at a temperature of the glass transition temperature or higher, and the maximum elastic modulus at a temperature of the minimum storage elastic modulus or higher are shown in Table A3.

**Table A3**

| | | Example A8 | Example A9 | Example A14 |
|---|---|---|---|---|
| glass transition temperature | temperature (°C) | 364 | 359 | 334 |
| | Maximum tan δ | 0.21 | 0.32 | 0.25 |
| the minimum storage elastic modulus at a temperature of the glass transition temperature or higher | storage elastic modulus (GPa) | * | 0.18 | 0.26 |
| | temperature (°C) | | 392 | 395 |
| maximum elastic modulus at a temperature of the minimum storage elastic modulus or higher | storage elastic modulus (GPa) | * | 0.22 | 0.35 |
| | temperature (°C) | | 434 | 443 |

| | | | | |
|---|---|---|---|---|
| * No minimum or maximum point due to monotonic decrease | | | | |

Based on the fact that in Examples A9 and A14 a maximum of storage elastic modulus appears at a temperature of the minimum storage elastic modulus or higher, it is thought that a cross-linking structure is formed. As a result, a decrease in the elastic modulus at high temperatures is prevented. Consequently, it was confirmed that these polyimide films are suited to high-temperature process.

According to the invention disclosed in Part A, a co-polyimide precursor can be produced stably under moderate conditions, and a co-polyimide having excellent transparency, high heat resistance, high glass transition temperature, and low coefficient of linear thermal expansion and also having bending resistance (toughness, i.e., sufficiently high elongation at break) can be provided. In particular, the polyimide of the present invention can be suitably used for, for example, a transparent substrate of a display device such as a flexible display or touch panel or a solar cell substrate.

### «Examples of PART B »

In each of the following examples, evaluation was carried out based on the following methods.
[Logarithmic Viscosity], [Light Transmittance], [Elastic Modulus and Elongation at break], and [Coefficient of Thermal Expansion (CTE)] were measure as explained in Part A

### [Example B1]

In a reaction vessel, 3.220g (0.0282 mol) of trans-1,4-diaminocyclohexane (may be referred as t-DACH below) was charged and dissolved in 103.7 g of N,N-dimethylacetamide(may be referred as t-DMAc below). To the mixture, 6.281g (0.0296 mol) of N,O-bis(trimethylsilyl)acetamide was added with a syringe and stirred at 80 °C for 2 hours to perform silylation. To the solution, 8.272g (0.0281 mol) of 3,3',4,4'-biphenyltetracarboxylic dianhydride (may be referred as s-BPDA below) was gradually added. The mixture was stirred at room temperature for 8 hours to obtain a uniform and viscous polyimide precursor solution.

The obtained polyimide precursor solution was applied on a glass substrate, and thermally imidized by heating at 120 °C for 1 hour, at 150 °C for 30 minutes, at 200 °C for 30 minutes and finally up to 400 °C while holding it on the substrate to obtain a polyimide/glass laminate. Thus obtained polyimide/glass laminate was immersed in water for delamination to obtain a polyimide film with thickness of about 10 µm. Measurement of properties of the film was conducted.
The results are shown in Table B1.

### [Example B2]

In a reaction vessel, 3.00g (0.026 mol) of trans-1,4-diaminocyclohexane was charged and dissolved in 60.35 g of N,N-dimethylacetamide. To the mixture, 5.55g (0.0273 mol) of N,O-bis(trimethylsilyl)acetamide was added and stirred at 80 °C for 2 hours to perform silylation. After cooling the solution to 40 °C, 6.77g (0.023 mol) of 3,3',4,4'-biphenyltetracarboxylic dianhydride and 0.88g(0.003 mol) of 2,3,3',4'-biphenyltetracarboxylic dianhydride was added. The mixture was stirred at 40 °C and all solids were dissolved in 1 hour. The mixture was further stirred for 8 hours to obtain a uniform and viscous co-polyimide precursor solution composition.

The obtained polyimide precursor solution was applied on a glass substrate, and thermally imidized by heating at 120 °C for 1 hour, at 150 °C for 30 minutes, at 200 °C for 30 minutes and at 350 °C for 3 minutes while holding it on the substrate under nitrogen atmosphere (oxygen concentration is 200 ppm or less) to obtain a colorless transparent co-polyimide/glass laminate. Thus obtained co-polyimide/glass laminate was immersed in water for delamination to obtain a co-polyimide film with thickness of about 10 µm. Measurement results of properties of the film are shown in Table B1.

### [Comparative Example B1]

In a reaction vessel, 2.284 g (0.02 mol) of trans-1,4-diaminocyclohexane was charged and dissolved in 73.51 g of N,N-dimethylacetamide that had been dehydrated using a molecular sieve. To the solution, 5.884 g (0.02 mol) of 3,3',4,4'-biphenyltetracarboxylic dianhydride was gradually added, and the resultant mixture was stirred at room temperature for 8 hours.
However, the reaction mixture remained clouded and a uniform polyimide precursor solution was not obtained.
The results are shown in Table B1.

### [Comparative Example B2]

In a reaction vessel, 6.85g (0.06 mol) of trans-1,4-diaminocyclohexane was charged and dissolved in 98.02 g of N,N-dimethylacetamide that had been dehydrated using a molecular sieve. To the solution, 17.65g (0.06 mol) of 3,3',4,4'-biphenyltetracarboxylic dianhydride was gradually added, and the resultant mixture was heated to 120°C. When the start of dissolution of salts in about 5 minutes was confirmed, the mixture was cooled rapidly to room temperature and held at room temperature for 8 hours with stirring.
However, white precipitatios were remained in the reaction mixture and a uniform polyimide precursor solution was not obtained.
The results are shown in Table B1.

### [Comparative Example B3]

In a reaction vessel, 2.284g (0.02 mol) of trans-1,4-diaminocyclohexane was charged and dissolved in 73.51 g of N,N-dimethylacetamide that had been dehydrated using a molecular sieve. To the solution, 5.884g (0.02 mol) of 3,3',4,4'-biphenyltetracarboxylic dianhydride was gradually added, and the resultant mixture was heated to 120°C. When the start of dissolution of salts in about 5 minutes was confirmed, the mixture was cooled rapidly to room temperature and held at room temperature for 8 hours with stirring.
In the reaction mixture, white precipitations were observed on the wall of the reaction vessel, but a uniform and viscous polyimide precursor solution was obtained by pressure filtration.

This polyimide precursor varnish was applied on a glass substrate, dried in vacuo at room temperature and thermally imidized by heating at 120 °C for 1 hour, at 150 °C for 30 minutes, at 200 °C for 30 minutes and finally up to 400 °C while holding it on the substrate to obtain a polyimide/glass laminate. Thus obtained polyimide/glass laminate was immersed in water for delamination to obtain a polyimide film with thickness of about 10 µm. Measurement of properties of the film was conducted.
The results are shown in Table B1.

### [Comparative Example B4]

In a reaction vessel, 6.851g (0.06 mol) of trans-1,4-diaminocyclohexane was charged and dissolved in 220.5 g of N,N-dimethylacetamide that had been dehydrated using a molecular sieve. To the solution, 15.89g (0.054 mol) of 3,3',4,4'-biphenyltetracarboxylic dianhydride and 1.765g (0.006 mol) of 2,3,3',4'-biphenyltetracarboxylic dianhydride were gradually added, and the resultant mixture was heated to 120°C. When the start of dissolution of salts in about 5 minutes was confirmed, the mixture was cooled rapidly to room temperature and held at room temperature for 8 hours with stirring to obtain a uniform and viscous coplymerized polyimide precursor solution composition.

The obtained polyimide precursor solution composition was applied on a glass substrate, and thermally imidized by heating at 120 °C for 1 hour, at 150 °C for 30 minutes, at 200 °C for 30 minutes and finally up to 400 °C while holding it on the substrate to obtain a colorless transparent co-polyimide/glass laminate. Thus obtained co-polyimide/glass laminate was immersed in water for delamination to obtain a co-polyimide film with thickness of about 10 µm. Measurement results of properties of the film are shown in Table B1.

**Table B1**

| | | | Ex. B1 | Ex. B2 | Comp. Ex. B1 | Comp. Ex. B2 | Comp. Ex. B3 | Comp. Ex. B4 |
|---|---|---|---|---|---|---|---|---|
| Polyimide Precursor | | | | | | | | |
| Chemical Composition | Diamine component | t-DACH | 1.00 | 1.00 | 1.00 | 1.00 | 1.00 | 1.00 |
| | silylating agent component | BSA | 1.05 | 1.05 | | | | |
| | acid component | s-BPDA | 1.00 | 0.90 | 1.00 | 1.00 | 1.00 | 0.90 |
| | | a-BPDA | | 0.10 | | | | 0.10 |
| Monomer concentration (wt%) | | | 10 | 15 | 10 | 20 | 10 | 10 |
| Polymerization temperature condition (°C) | | | 25°Cx8hr | 40°Cx1hr +40°Cx8hr | 25°Cx8hr | 120°Cx5min +25°Cx8hr | 120°Cx5min +25°Cx8hr | 120°Cx5min +25°Cx8hr |
| State after polymerization (visual inspection) | | | Dissolved uniformly | Dissolved uniformly | ununiform | ununiform | ununiform (uniform after filtration) | Dissolved uniformly |
| Logarithmic Viscosity (dL/g) | | | 1.98 | 1.60 | - | - | 1.26 | 1.64 |
| | | | | | | | | |
| Evaluation of Polyimide film | | | | | | | | |
| Light Transmittance at 400nm (%) | | | 66 | 80 | - | - | 70 | 67 |
| Elastic Modulus (GPa) | | | 6.9 | 5.5 | - | - | 7.5 | 4.9 |
| Elongation at break (%) | | | 11.0 | 24.5 | - | - | 4.3 | 18.4 |
| Coefficient of Thermal Expansion (ppm/K) | | | 10.1 | 9.9 | - | - | 7.9 | 19.9 |

| | | | | | | | | |
|---|---|---|---|---|---|---|---|---|
| Note: Values in cells of Composition denote molar ratio | | | | | | | | |

As can be seen from the results shown in Table B1, since precipitation and the like does not occur, the polyimide precursor according to the present invention can be polymerized under mild conditions, and is thus suited to actual industrial production. Further, the obtained polyimide film has excellent light transmittance, a sufficient elongation at break, and a low linear coefficient of thermal expansion. In Example B2, it was confirmed that by using a plurality of types of acid component, even better light transmittance, higher elongation at break, and a lower linear coefficient of thermal expansion could be achieved.

According to the invention disclosed in Part B, a polyimide precursor using an alicyclic diamine can be produced by a method suitable for actual industrial production and a polyimide precursor having a good handling property and storage stability can be provided. The polyimide prepared from such a polyimide precursor has high transparency, high glass transition temperature, low coefficient of linear thermal expansion and also has sufficiently high toughness. Accordingly, the polyimide can be suitably used in a plastic substrate as a replacement for the glass substrate of, in particular, a display device such as a liquid crystal display, an EL display, or electronic paper.

### «Examples of PART C »

The raw materials used in the respective following examples are as follows.

2,3,3',4'-Biphenyltetracarboxylic dianhydride a-BPDA): Manufactured by Ube Industries Ltd., purity 99.6% (purity determined by HPLC analysis of ring-opened 2,3,3',4'-biphenyltetracarboxylic acid), acid anhydride ratio 99.5%.

1,3-Bis(4-aminobenzoyloxy)benzene (13P-BABB): Used was a product manufactured by Mikuni Pharmaceutical Industrial Co., Ltd., that was subjected to an activated carbon treatment, and then subjected to sublimation purification.

Solvent: Product equivalent to reagent grade or analytical grade, manufactured by Wako Pure Chemical Industries, Ltd.

2 N Aqueous solution of sodium hydroxide: Aqueous solution of sodium hydroxide, manufactured by Tokyo Chemical Industry Co., Ltd.

In each of the following examples, evaluation was carried out based on the following methods.
Evaluation of 2,3,3',4'-biphenyltetracarboxylic dianhydride (a-BPDA)

### [Solubility of a-BPDA at 25°C]

In a glass vessel, 10.0 g of a-BPDA powder having a purity of 99.6% and an acid anhydride ratio of 99.5% and 20.0 g of solvent were charged, and the resultant mixture was thoroughly stirred at 25°C for 3 hours. The undissolved a-BPDA was filtered through filter paper 5A manufactured by Advantec, Inc. to obtain a-BPDA saturated solution. 5g of the a-BPDA saturated solution was charged into an aluminum dish, heated for 1 hour at 80°C, and then heated for 1 hour at 200°C. The solubility was calculated by determining the mass of a-BPDA remaining in the saturated solution based on the residue after heating.

### [Light Transmittance]

A predetermined amount of a-BPDA powder was dissolved in a 2 N aqueous solution of sodium hydroxide to obtain a 10 mass% aqueous solution. Using a MCPD-300 manufactured by Otsuka Electronics Co., Ltd., and a standard cell having a light path length of 1 cm, the light transmittance at 400 nm of the 10 mass% a-BPDA powder / 2 N aqueous solution of sodium hydroxide was measured using the 2 N aqueous solution of sodium hydroxide as a blank.

### Evaluation of Polyimide Precursor

### [Logarithmic Viscosity]

The logarithmic viscosity was measured in the same manner as in Part A.

### [Light Transmittance]

The polyimide precursor was diluted with N,N-dimethylacetamide so as to form a 10 mass% polyimide precursor solution. Then, using a MCPD-300 manufactured by Otsuka Electronics Co., Ltd., and a standard cell having a light path length of 1 cm, the light transmittance at 400 nm of the 10 mass% polyimide precursor solution was measured using N,N-dimethylacetamide as a blank.

### Evaluation of Polyimide

[Light Transmittance], [Elastic Modulus], and [Coefficient of Thermal Expansion (CTE)] were measured in the same manner as in Part A.

### [Example C1]

In a glass vessel, 10.0 g of a-BPDA powder as a raw material and 10.0 g of dimethylsulfoxide as a solvent were charged. The resultant mixture was thoroughly stirred at 25°C for 3 hours. The solution was separated by filtration, and the obtained powder was dried in vacuo at 100°C for 2 hours to obtain a-BPDA powder having reduced color. The solubility of the used solvent, and the light transmittance and recovery ratio results of the obtained a-BPDA powder are shown in Table C1.

### [Example C2 to C5]

a-BPDA powders having reduced color were obtained in the same manner as Example C1 except that the solvent used is changed to a solvent as indicated in Table C1. Solubility of the solvents used, and results of light transmittance and recovery ratio of the obtained a-BPDA powders are shown in Table C1.

### [Comparative Example C1]

Light transmittance of the raw material a-BPDA powder that is a powder before performing purification of the present invention is shown in Table C1.

### [Comparative Example C2 to C3]

a-BPDA powders were obtained in the same manner as Example C1 except that the solvent used is changed to a solvent as indicated in Table C1. Solubility of the solvents used, and results of light transmittance and recovery ratio of the obtained a-BPDA powders are shown in Table C1.

### [Comparative Example C4]

In a glass vessel, 10.0 g of a-BPDA powder as a raw material and 90.0 g of acetic anhydride were charged. The resultant mixture was stirred at 120 °C for 3 hours, to dissolve all of a-BPDA powder. When heated for dissolving a-BPDA powder, coloring of the solution was observed. The solution was cooled to 25 °C while stirring to precipitate crystals. The solution was separated by filtration, and the obtained solid was dried in vacuo at 100°C for 2 hours. The light transmittance of the a-BPDA powder obtained by recrystallization is shown in Table C1.

**Table C1**

| | Solvent (or method of purification) | Solubility at 25°C (g/100g) | recovery ratio (%) | light transmittance at 400nm (%) |
|---|---|---|---|---|
| Example C1 | dimethylsulfoxide | 32 | 51 | 98 |
| Example C2 | N,N-dimethylformamide | 36 | 51 | 97 |
| Example C3 | Acetone | 10 | 88 | 94 |
| Example C4 | Tetrahydrofuran | 11 | 85 | 92 |
| Example C5 | Acetone/Toluene (mass ratio :2/8) | 1.9 | 92 | 89 |
| Comparative Example C1 | (unpurified) | | | 77 |
| Comparative Example C2 | Toluene | 0.2 | 97 | 81 |
| Comparative Example C3 | Acetone/Toluene (mass ratio: 1/9) | 0.8 | 95 | 84 |
| Comparative Example C4 | (recrystallization) | | 60 | 84 |

As can be seen from the results shown in Table C1, the a-BPDA according to the present invention having reduced color is improved in a light transmittance to 85% or more and preferably to 90% or more at 400 nm, and thus is preferable as a polyimide raw material for high-performance optical materials.

### [Example C6]

In a reaction vessel, 3.484 g (0.01 mol) of 1,3-bis(4-aminobenzoyloxy)benzene (13p-BABB) that had been subjected to an activated carbon treatment and then sublimation purification, and 37.31 g of N,N-dimethylacetamide (DMAc) that had been dehydrated using a molecular sieve were charged, and the resultant mixture was dissolved at 50°C under a nitrogen flow. To the solution, 3.102 g (0.01 mol) of the 2,3,3',4'-biphenyltetracarboxylic dianhydride powder (a-BPDA powder) obtained in Example C3 was gradually added, and the resultant mixture was stirred for 12 hours at 50°C to obtain a uniform and viscous polyimide precursor solution.

The obtained polyimide precursor solution was applied on a glass substrate, and thermally imidized by heating at 120 °C for 1 hour, at 150 °C for 30 minutes, at 200 °C for 30 minutes and at 350 °C for 5 minutes while holding it on the substrate under nitrogen atmosphere to obtain a colorless transparent polyimide/glass laminate. Thus obtained polyimide/glass laminate was immersed in water for delamination to obtain a co-polyimide film with thickness of about 10 µm. Measurement results of properties of the film are shown in Table C2.

### [Comparative Example C5]

Polyimide precursor solution and polyimide film were obtained in the same manner as Example C6 except that unpurified a-BPDA powder was used. Measurement results of properties are shown in Table C2.

**Table C2**

| | | Example C6 | Comparativ e Example C5 |
|---|---|---|---|
| Formulation of polyimide (molar ratio) | | | |
| Acid component | a-BPDA(Example C3, light transmittance:94%) | 1 | |
| | a-BPDA(Comparative Example C1, light transmittance:77%) | | 1 |
| Diamine component | 1,3-bis(4-aminobenzoyloxy)benzene | 1 | 1 |
| | | | |

| Evaluation results of Polyimide Precursor | | | |
|---|---|---|---|
| Logarithmic Viscosity | | 0.37 | 0.31 |
| Evaluation results of Polyimide | | | |
| Light Transmittance (%) | | 74 | 68 |
| Elastic Modulus (GPa) | | 2.7 | 2.3 |
| Coefficient of Thermal Expansion (ppm/K) | | 55 | |

As can be seen from the results shown in Table C2, the polyimide film according to the present invention, which used a-BPDA having reduced color, has improved light transmittance as a film.

The invention disclosed in Part C can provide a method of readily purifying a 2,3,3',4'-biphenyltetracarboxylic dianhydride powder having reduced color by a simple procedure, a 2,3,3',4'-biphenyltetracarboxylic dianhydride powder having reduced color, and a polyimide having an increased light transmittance that can be suitably used as a high-performance optical material.

### «Examples of PART D »

The raw materials used in the respective following examples are as follows.

3,3',4,4'-Biphenyltetracarboxylic dianhydride (s-BPDA): Manufactured by Ube Industries Ltd., purity 99.9% (purity determined by HPLC analysis of ring-opened 3,3',4,4'-biphenyltetracarboxylic acid), acid anhydride ratio 99.8%.
2,3,3',4'-Biphenyltetracarboxylic dianhydride (hereinafter may be referred to as a-BPDA): the material used was obtained by washing in acetone a product manufactured by Ube Industries Ltd., having purity 99.6% (purity determined by HPLC analysis of ring-opened 2,3,3',4'-biphenyltetracarboxylic acid) and acid anhydride ratio 99.5%.
2,2',3,3'-Biphenyltetracarboxylic dianhydride (hereinafter may be referred to as i-BPDA): the material used was obtained by washing in NMP a product manufactured by Changzhou Weijia Chemical Co., Ltd., having purity 99.9% (purity determined by HPLC analysis of ring-opened 2,2',3,3'-biphenyltetracarboxylic acid) and acid anhydride ratio 99%.
Solvent: Product equivalent to reagent grade or analytical grade, manufactured by Wako Pure Chemical Industries, Ltd.
2 N aqueous solution of sodium hydroxide: Aqueous solution of sodium hydroxide, manufactured by Tokyo Chemical Industry Co., Ltd.
Trans-1,4-diaminocyclohexane (hereinafter may be referred to as t-DACH): the material used was obtained by purifying by sublimation a product manufactured by Zhejiang Taizhou Qingquan Medical & Chemical Co., Ltd., purity 99.1% (GC analysis).

In each of the following examples, evaluation was carried out based on the following methods.

### Evaluation of 3,3',4,4'-biphenyltetracarboxylic dianhydride powder

### [Solubility of s-BPDA at 25°C]

In a glass vessel, 5.0 g of s-BPDA powder having a purity of 99.9% and an acid anhydride ratio of 99.8% and 50.0 g of solvent were charged, and the resultant mixture was thoroughly stirred at 25°C for 3 hours. The non-dissolved s-BPDA was filtered through filter paper 5A manufactured by Advantec, Inc. to obtain s-BPDA saturated solution. 5g of the s-BPDA saturated solution was charged into an aluminum dish, heated for 1 hour at 80°C, and then heated for 1 hour at 200°C. The solubility was calculated by determining the mass of s-BPDA remaining in the saturated solution based on the residue after heating.

### [Light Transmittance]

A predetermined amount of s-BPDA powder was dissolved in a 2 N aqueous solution of sodium hydroxide to obtain a 10 mass% aqueous solution. Using a MCPD-300 manufactured by Otsuka Electronics Co., Ltd., and a standard cell having a light path length of 1 cm, the light transmittance at 400 nm of the s-BPDA solution was measured using the 2 N aqueous solution of sodium hydroxide as a blank.

### Evaluation of Polyimide Precursor and Polyimide

### [Logarithmic Viscosity]

The logarithmic viscosity was measured in the same manner as in Part A.

### [Light Transmittance (Polyimide Precursor)]

Light Transmittance was measured in the same manner as in Part C. [Light Transmittance (Polyimide)], [Elastic Modulus, Elongation at Break], and [Coefficient of Thermal Expansion (CTE)] were measured in the same manner as in Part A.

### [Example D1]

In a glass vessel, 10.0 g of unpurified s-BPDA powder and 10.0 g of dimethylformamide as a solvent were charged. The resultant mixture was thoroughly stirred at 25°C for 3-hours. The solution was separated by filtration, and the obtained powder was dried in vacuo at 100°C for 2 hours to obtain s-BPDA powder. The results of evaluation of the obtained s-BPDA powder are shown in Table D1.

### [Example D2 to D7]

s-BPDA powders were obtained in the same manner as Example D1 except that the solvent used is changed to a solvent as indicated in Table D1. Solubility of the solvents used, and results of light transmittance and recovery ratio of the obtained a-BPDA powders are shown in Table C1. Herein, the yields were 9.6 g (Example D2), 9.4 g (Example D3), 9.5g (Example D4), 9.6 g (Example D5), 9.7 g (Example D6), and 9.6 g (Example D7). The results of evaluation of the obtained s-BPDA powder are shown in Table D 1.

### [Example D8]

In a glass vessel, 20.0 g of s-BPDA and 200 g of N-methyl-2-pyrrolidone as a solvent were charged, and the resultant mixture was thoroughly stirred at 25°C for 3 hours. The solution was separated by filtration, and the obtained solid was dried in vacuo for 2 hours at 100°C to obtain s-BPDA powder. The yield was 15.2 g.
5g of this s-BPDA powder was charged into a glass sublimation apparatus. The pressure was reduced to 1 Torr or less and the temperature of the bottom wall surface with which the s-BPDA was in contact was increased to 200 to 220°C, whereby the s-BPDA was sublimed. A powder of s-BPDA crystals cooled and solidified on the wall surface of an upper portion of the sublimation apparatus adjusted to a temperature of 25°C was obtained. The yield was 3.1 g. The evaluation results of the obtained s-BPDA powder are shown in Table D 1.

### [Comparative Example D1]

The evaluation results of the unpurified s-BPDA powder are shown in Table D1.

### [Comparative Examples D2 to D3]

s-BPDA powders were obtained in the same manner as Example D1 except that the solvent used is changed to a solvent as indicated in Table D1. Herein, the yields were 9.7 g (Comparative Example D2) and 9.7 g (Comparative Example D3). The results of evaluation of the obtained s-BPDA powder are shown in Table D1.

### [Comparative Example D4]

5g of unpurified s-BPDA powder was charged into a glass sublimation apparatus and sublimation was carried out in the same manner as Example D8. The yield was 4.4 g. The evaluation results of the obtained s-BPDA powder are shown in Table D1.

### [Comparative Example D5]

In a glass vessel, 5.0 g of s-BPDA and 200 g of acetic anhydride were charged. The resultant mixture was heated to reflux for 3 hours to dissolve the powder. At this time, coloring of the solution was observed. The solution was cooled to 25 °C to precipitate. The solution was separated by filtration, and the obtained solid was dried in vacuo at 100°C for 2 hours. The yield was 4.2 g. The evaluation results of the obtained s-BPDA powder are shown in Table D1.

**Table D1**

| | Solvent used/operation | Solubility of used solvent at 25°C (g/100g) | light transmittance of obtained s-BPDA powder at 400nm (%) |
|---|---|---|---|
| Example D1 | N,N-dimethylformamide | 1.3 | 81 |
| Example D2 | N,N-dimethylacetamide | 1.3 | 81 |
| Example D3 | N-methyl-2-pyrrolidone | 2.3 | 82 |
| Example D4 | acetonitrile | 0.1 | 78 |
| Example D5 | acetone | 0.1 | 79 |
| Example D6 | γ-butyrolactone | 0.4 | 79 |
| Example D7 | tetrahydrofuran | 0.3 | 79 |
| Example D8 | N-methyl-2-pyrrolidone/sublimation | - | 91 |
| Comparative Example D1 | unpurified | - | 75 |
| Comparative Example D2 | toluene | < 0.1 | 75 |
| Comparative Example D3 | chloroform | < 0.1 | 75 |
| Comparative Example D4 | sublimation | - | 72 |
| Comparative Example D5 | recrystallization | - | 3 |

As can be seen from the results shown in Table D1, the s-BPDA according to the present invention having reduced color is improved in a light transmittance of above 75%, and preferably 80% or more at 400 nm with respect to a solution obtained by dissolving the s-BPDA powder to a concentration of 10 mass% in a 2 N aqueous solution of sodium hydroxide.

The following examples will describe polyimides formed from a tetracarboxylic acid component that contains s-BPDA separated and collected based on the purification method according to the present invention, and a diamine component that is selected from the group consisting of aliphatic diamines, diamines having an alicyclic structure, and aromatic diamines having any substituent of a halogen group, a carbonyl group, and a sulfonyl group.

### [Example D9]

In a reaction vessel, 1.40 g (0.0122 mol) of trans-1,4-diaminocyclohexane (t-DACH) was charged and dissolved in 28.4 g of N,N-dimethylacetamide that had been dehydrated using a molecular sieve. The solution were heated to 50°C, and 3.50g (0.0119 mol) of s-BPDA obtained in Example D3 and 0.09g (0.0003 mol) of a-BPDA was gradually added. The resultant mixture was stirred at 50°C for 6 hours to obtain a uniform and viscous polyimide precursor solution.
The obtained polyimide precursor solution was applied on a glass substrate, and thermally imidized by heating at 120 °C for 1 hour, at 150 °C for 30 minutes, at 200 °C for 30 minutes and at 350 °C for 5 minutes under nitrogen atmosphere to obtain a colorless transparent polyimide/glass laminate. Thus obtained polyimide/glass laminate was immersed in water for delamination to obtain a polyimide film with thickness of about 10 µm.
Measurement results of properties of the film are shown in Table D2.

### [Example D10]

Polyimide precursor solution and polyimide film were obtained in the same manner as Example D9 except that an acid component indicated in Table D2 was used.
Measurement results of film properties are shown in Table D2.

### [Comparative Example D6]

Polyimide precursor solution and polyimide film were obtained in the same manner as Example D9 except that unpurified s-BPDA powder of Comparative Example D1 was used.
Measurement results of film properties are shown in Table D2.

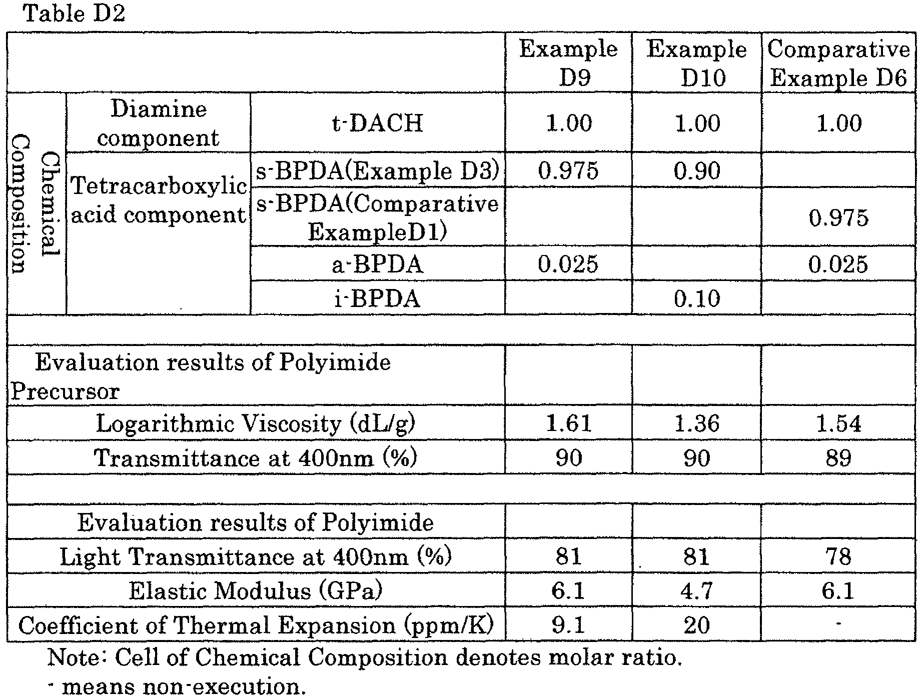

As can be seen from Table D2, when s-BPDA separated and collected by the purification method according to the present invention, the transparency of the obtained polyimide is improved, and the light transmittance of 80% or more at 400 nm when formed as a film having a 10 µm thickness can be achieved.

The invention disclosed in Part D can provide a method of readily purifying a 3,3',4,4'-biphenyltetracarboxylic dianhydride powder having reduced color by a simple operation under moderate conditions without requiring huge facilities. The use of the 3,3',4,4'-biphenyltetracarboxylic dianhydride powder having reduced color prepared by the method of purification of the present invention can provide a polyimide that can be suitably used as a high-performance optical material having excellent transparency, in particular, as a transparent base material of a display device such as a flexible display or touch panel.

### «Examples of PART E »

The raw materials used in the respective following examples are as follows.
Trans-1,4-diaminocyclohexane: Manufactured by Zhejiang Taizhou Qingquan Medical & Chemical Co., Ltd., purity 99.1% (GC analysis).
Adsorption agent: Activated carbon, Norit SX Plus, manufactured by Japan Norit Inc., specific surface area based on BET method of 1,100 m²/g.
3,3',4,4'-Biphenyltetracarboxylic dianhydride (s-BPDA): the material used was obtained by adding an equivalent mass amount of N-methyl-2-pyrrolidone to a product manufactured by Ube Industries Ltd. with purity 99.9% (purity determined by HPLC analysis of ring-opened 3,3',4,4'-biphenyltetracarboxylic acid) and acid anhydride ratio 99.8%, stirring the mixture at room temperature for 3 hours, then recovering undissolved powder and subjecting it to vacuum drying.
2,3,3',4'-Biphenyltetracarboxylic dianhydride (a-BPDA): the material used was obtained by adding an equivalent mass amount of acetone to a product manufactured by Ube Industries Ltd. with purity 99.6% (purity determined by HPLC analysis of ring-opened 2,3,3',4'-biphenyltetracarboxylic acid) and acid anhydride ratio 99.5%, stirring at room temperature for 3 hours, then recovering undissolved powder and subjecting it to vacuum drying.
2,2',3,3'-Biphenyltetracarboxylic dianhydride ("i-BPDA"): the material used was obtained by adding an equivalent mass amount of N-methyl-2-pyrrolidone to a product manufactured by Changzhou Weijia Chemical Co., Ltd. with purity 99.9% (purity determined by HPLC analysis of ring-opened 2,2',3,3'-biphenyltetracarboxylic acid) and acid anhydride ratio 99%, stirring at room temperature for 3 hours, then recovering undissolved powder and subjecting it to vacuum drying.
4,4'-(2,2-hexafluoroisopropylene)diphthalic dianhydride (6FDA): Manufactured by WeylChem Group, purity 99.77% (purity determined by H-NMR).
4,4'-(Dimethylsiladyl)diphthalic dianhydride (DPSDA): Manufactured by Toray Fine Chemicals Co., Ltd., purity 99.8% (HPLC analysis).
2 N aqueous solution of sodium hydroxide: Aqueous solution of sodium hydroxide, manufactured by Tokyo Chemical Industry Co., Ltd.
Solvent: Product equivalent to reagent grade or analytical grade, manufactured by Wako Pure Chemical Industries, Ltd.

In each of the following examples, evaluation was carried out based on the following methods.

### Evaluation of trans-1,4-diaminocyclohexane powder

### [Light Transmittance]

A predetermined amount of trans-1,4-diaminocyclohexane powder was dissolved in pure water to obtain a 10 mass% aqueous solution. Using a MCPD-300 manufactured by Otsuka Electronics Co., Ltd., and a standard cell having a light path length of 1 cm, the light transmittance at 400 nm of the trans-1,4-diaminocyclohexane solution was measured using pure water as a blank.

### Evaluation of Polyimide Precursor and Polyimide

### [Logarithmic Viscosity]

The logarithmic viscosity was measured in the same manner as in Part A.

### [Light Transmittance (polyimide precursor)]

Light transmittance was measured in the same manner as in Part C. [Light Transmittance (polyimide)], [Elastic Modulus and Elongation at break], and [Coefficient of Thermal Expansion (CTE)] were measured in the same manner as in Part A.

### [Example E1]

In a glass sublimation apparatus, 10.0 g of unpurified trans-1,4-diaminocyclohexane was charged, and the pressure was then reduced to 1 Torr or less. The temperature of the bottom wall surface with which the trans-1,4-diaminocyclohexane was in contact was increased to 50°C, whereby a sublimate was obtained on the opposite wall upper surface that was adjusted to 5°C. The yield was 8.2 g. The results of the light transmittance of the trans-1,4-diaminocyclohexane powder obtained by this method are shown in Table E1.

### [Example E2]

In a glass vessel, 5.0 g of unpurified trans-1,4-diaminocyclohexane and 25 g of n-hexane as solvent were charged, and the resultant mixture was dissolved under a nitrogen atmosphere at 60°C. To the mixture, 0.05 g of an adsorption agent (Norit SX Puls) as an adsorption agent was added, and the mixture was stirred for 1 hour at 60°C. Then, while maintaining the temperature at 60°C, the adsorption agent was removed by filtration to obtain a colorless, transparent solution. While stirring, this solution was gradually cooled to 25°C to precipitate crystals. The crystals were separated by filtration, and the obtained crystals were dried in vacuo. The results of the light transmittance of the trans-1,4-diaminocyclohexane powder obtained by this method are shown in Table E1.

### [Referential Example E1]

The evaluation result of the unpurified trans-1,4-diaminocyclohexane powder is shown in Table E1.

### [Referential Example E2]

In a glass vessel, 5.0 g of unpurified trans-1,4-diaminocyclohexane and 25 g of n-hexane as solvent were charged, and the resultant mixture was dissolved at 60°C by heating. Insoluble matters were removed by decantation and the solution was cooled to 25°C to precipitate crystals. The crystals were separated by filtration and obtained crystals were dried in vacuo. The results of the light transmittance of the trans-1,4-diaminocyclohexane powder obtained by this method are shown in Table E1.

**Table E 1**

| | Purification method | Light Transmittance at 400nm (%) |
|---|---|---|
| Example E1 | sublimation | 96 |
| Example E2 | Treatment by adsorption agent | 96 |
| Referential Example E1 | unpurified | 86 |
| Referential Example E2 | recrystallization | 89 |

As can be seen from the results shown in Table E1, the trans-1,4-diaminocyclohexane according to the present invention having reduced color has a light transmittance, at 400 nm, of 90% or more, and preferably 95% or more, and thus is preferable as a polyimide raw material for optical material applications.

### [Example E3]

In a reaction vessel purged with nitrogen gas, 1.40 g (0.0122 mol) of trans-1,4-diaminocyclohexane (t-DACH) obtained in Example E1 as a diamine component was charged and dissolved in 28.4 g of N,N-dimethylacetamide that had been dehydrated using a molecular sieve. The resultant mixture was heated to 50°C, to which 3.50g (0.0119 mol) of 3,3',4,4'-biphenyltetracarboxylic dianhydride (s-BPDA) and 0.09g (0.0003 mol) of 2,3,3',4'-biphenyltetracarboxylic dianhydride (a-BPDA) was gradually added. The concentration of monomers (total amount of diamine component and carboxylic acid component) in the solution was 15 % by mass. The solution was stirred at 50 °C for 6 hours to obtain a uniform and viscous polyimide precursor solution.
Measurement results of properties of the polyimide precursor solution are shown in Table E2.

The polyimide precursor solution was filtered using a PTFE membrane filter, and was applied on a glass substrate, and thermally imidized by heating stepwise, i.e. sequentially at 120 °C for 1 hour, at 150 °C for 30 minutes, at 200 °C for 30 minutes and at 350 °C for 5 minutes under nitrogen atmonsphere to obtain a colorless transparent polyimide/glass laminate. Thus obtained co-polyimide/glass laminate was immersed in water for delamination to obtain a polyimide film with thickness of about 10 µm. Measurement results of properties of the film are shown in Table E2.

### [Examples E4 to E7]

Polyimide precursor solutions and polyimide films were obtained in the same manner as Example E3 in which diamine component and carboxylic acid component were used as indicated in Table E2 and the concentration of monomers in the solution (total amount of diamine component and carboxylic acid component) was 15 % by mass. Measurement results of properties are shown in Table E2.

### [Comparative Example E1]

Polyimide precursor solution and polyimide film were obtained in the same manner as Example E3 except that unpurified t-DACH was used as diamine component.
Measurement results of film properties are shown in Table E2.

**Table E2**

| | | Example E3 | Example E4 | Example E5 | Example E6 | Example E7 | Comparative Example E1 |
|---|---|---|---|---|---|---|---|
| Chemical Composition | | | | | | | |
| Diamine component | t-DACH (Example E1) | 1.00 | 1.00 | 1.00 | 1.00 | 1.00 | |
| | t-DACH (Referential Example E1) | | | | | | 1.00 |
| Tetracarboxylic acid component | s-BPDA | 0.975 | 0.90 | 0.90 | | | 0.975 |
| | a-BPDA | 0.025 | | 0.10 | | | 0.025 |
| | i-BPDA | | 0.10 | | | | |
| | 6FDA | | | | 1.00 | | |
| | DPSDA | | | | | 1.00 | |
| | | | | | | | |

| Evaluation results of Polyimide Precursor | | | | | | | |
|---|---|---|---|---|---|---|---|
| Logarithmic Viscosity (dL/g) | | 1.61 | 1.36 | 1.29 | 0.79 | 1.37 | 1.42 |
| Light Transmittance at 400nm (%) | | 90 | 90 | 90 | 91 | 96 | 82 |
| | | | | | | | |

| Evaluation results of Polyimide | | | | | | | |
|---|---|---|---|---|---|---|---|
| Light Transmittance at 400nm (%) | | 81 | 81 | 82 | 89 | 89 | 78 |
| Elastic Modulus (GPa) | | 6.1 | 4.7 | 3.8 | 2.8 | 2.6 | 6.3 |
| Coefficient of Thermal Expansion (ppm/K) | | 9.1 | 20 | - | 60 | 72 | - |

| | | | | | | | |
|---|---|---|---|---|---|---|---|
| Note: Cell of Chemical Composition denotes molar ratio. - means non-execution. | | | | | | | |

As can be seen from the results shown in Table E2, the polyimide according to the present invention, in which a trans-1,4-diaminocyclohexane powder having reduced color is used, has a light transmittance at 400 nm of 80% or more, and thus is preferable as a polyimide for optical material applications.

The invention disclosed in Part E can propose a trans-1,4-diaminocyclohexane powder reduced in coloring and a polyimide reduced in coloring prepared using it as the diamine component. The polyimide prepared using the trans-1,4-diaminocyclohexane powder having reduced color of the present invention has a light transmittance of 80% or more at 400 nm and can be suitably used as an optical material.

### «Examples of PART F »

The raw materials used in the respective following examples are as follows.
2,2',3,3'-Biphenyltetracarboxylic dianhydride ("i-BPDA"): Manufactured by Changzhou Weijia Chemical Co., Ltd., purity 99.9% (purity determined by HPLC analysis of ring-opened 2,2',3,3'-biphenyltetracarboxylic acid), acid anhydride ratio 99%
3,3',4,4'-Biphenyltetracarboxylic dianhydride (s-BPDA): the material used was obtained by adding an equivalent mass amount of N-methyl-2-pyrrolidone to a product manufactured by Ube Industries Ltd. with purity 99.9% (purity determined by HPLC analysis of ring-opened 3,3',4,4'-biphenyltetracarboxylic acid) and acid anhydride ratio 99.8%, stirring the mixture at room temperature for 3 hours, then recovering undissolved powder and subjecting it to vacuum drying.
2 N Aqueous solution of sodium hydroxide: Aqueous solution of sodium hydroxide, manufactured by Tokyo Chemical Industry Co., Ltd.
Trans-1,4-diaminocyclohexane ("t-DACH"): the material used was obtained by purifying a product manufactured by Zhejiang Taizhou Qingquan Medical & Chemical Co., Ltd. with purity 99.1% (GC analysis) by sublimation.
1,4-Bis(4-aminobenzoyloxy)benzene (BABB): the material used was obtained by purifying a product manufactured by Mikuni Pharmaceutical Industrial Co., Ltd. by an activated carbon treatment and sublimation.

In each of the following examples, evaluation was carried out based on the following methods.

### [Solubility of i-BPDA at 25°C]

In a glass vessel, 5.0 g of i-BPDA powder having a purity of 99.9% and an acid anhydride ratio of 99% and 50.0 g of solvent were charged, and the resultant mixture was thoroughly stirred at 25°C for 3 hours. The non-dissolved i-BPDA was filtered through filter paper 5A manufactured by Advantec, Inc. to obtain i-BPDA saturated solution. 5g of the i-BPDA saturated solution was charged into an aluminum dish, heated for 1 hour at 80°C, and then heated for 1 hour at 200°C. The solubility was calculated by determining the mass of i-BPDA remaining in the saturated solution based on the residue after heating.

### [Light Transmittance]

A predetermined amount of i-BPDA powder was dissolved in a 2 N aqueous solution of sodium hydroxide to obtain a 10 mass% aqueous solution. Using a MCPD-300 manufactured by Otsuka Electronics Co., Ltd., and a standard cell having a light path length of 1 cm, the light transmittance at 400 nm of the i-BPDA solution was measured using the 2 N aqueous solution of sodium hydroxide as a blank.

### Evaluation of polyimide precursor and polyimide

### [Logarithmic Viscosity]

The logarithmic viscosity was measured in the same manner as in Part A.

### [Light Transmittance (polyimide precursor)]

The light transmittance was measured in the same manner as in Part C.

[Light Transmittance (polyimide)], [Elastic Modulus and Elongation at break], and [Coefficient of Thermal Expansion (CTE)] were measured in the same manner as in Part A.

### [Example F1]

In a glass vessel, 10.0 g of i-BPDA and 10.0 g of dimethylsulfoxide as a solvent were charged with. The resultant mixture was thoroughly stirred at 25°C for 3 hours. The solution was separated by filtration, and the obtained solid was dried in vacuo for 2 hours at 100°C to obtain i-BPDA powder having reduced color. The light transmittance results of the i-BPDA obtained by this method are shown in Table F1.

### [Examples F2 and F3]

i-BPDA having reduced color was obtained in the same manner as in Example F1, except that the solvent was changed to the solvent indicated in Table F1. The light transmittance results of the i-BPDA obtained by this method are shown in Table F1.

### [Example F4] Recrystallization under an inert gas atmosphere

In a glass vessel, 10.0 g of i-BPDA and 150 g of acid anhydride were charged, and the resultant mixture was heated to reflux under a nitrogen atmosphere to dissolve. After dissolving, the solution was immediately cooled to 25°C while stirring, to precipitate crystals. The solution was separated by filtration, and the obtained powder was dried in vacuo for 2 hours at 100°C. The yield was 7.9 g. The light transmittance results of the i-BPDA obtained by this method are shown in Table F1.

### [Example F5] Recrystallization under an inert gas atmosphere

In a glass vessel, 5.0 g of 1-BPDA and 75 g of acid anhydride were charged with, and the resultant mixture was dissolved by heating at 130°C under a nitrogen flow. After dissolving, 0.05 g of activated carbon (Norit SX Plus) was added, and then stirred for 30 minutes. The activated carbon was removed by filtration, and the filtrate was then cooled to 25°C while stirring. The obtained powder was dried in vacuo for 2 hours at 100°C. The yield was 4.1 g. The light transmittance results of the i-BPDA obtained by this method are shown in Table F1.

### [Example F6] Sublimation at 300°C or less

In a glass sublimation apparatus, 10.0 g of i-BPDA obtained in the same manner as Example F6 was charged, and the pressure was then reduced to 1 Torr or less. The wall surface with which the i-BPDA was in contact was heated to 230 to 250°C, whereby a sublimate was obtained on a glass surface along which 25°C cold water was flowed. The yield was 8.8 g. The light transmittance results of the i-BPDA obtained by this method are shown in Table F1.

### [Example F7] Sublimation at 300°C or more

In a glass sublimation apparatus, 10.0 g of i-BPDA obtained in the same manner as Example F6 were charged, and the pressure was then reduced to 1 Torr or less. The wall surface with which the i-BPDA was in contact was heated to 300 to 320°C, whereby a sublimate was obtained on a glass surface along which 25°C cold water was flowed. The yield was 8.4 g. The light transmittance results of the i-BPDA obtained by this method are shown in Table F1.

### [Comparative Example F1]

The light transmittance of i-BPDA with no treatment such as purification is shown in Table F1.

### [Comparative Example F2]

i-BPDA was obtained in the same manner as in Example F1, except that the solvent was changed to the solvent indicated in Table F1. The light transmittance results of the i-BPDA obtained by this method are shown in Table F1.

### [Comparative Example F3] Recrystallization under air

In a glass vessel, 5.0 g of i-BPDA and 75 g of acid anhydride were charged, and the resultant mixture was heated to reflux under an air atmosphere for 3 hours. After dissolving, the solution was cooled to 25°C to precipitate crystals. The solution was separated by filtration, and the obtained powder was dried in vacuo for 2 hours at 100°C. The yield was 3.6 g. The light transmittance results of the i-BPDA obtained by this method are shown in Table F1.

**Table F1**

| | Solvent | Solubility at 25°C (g/100g) | Light Transmittance at 400nm (%) |
|---|---|---|---|
| Example F1 | dimethylsulfoxide | 3.2 | 91 |
| Example F2 | N,N-dimethylacetamide | 4.4 | 92 |
| Example F3 | N-methyl-2-pyrrolidone | 6.8 | 93 |
| Example F4 | (recrystallization under nitrogen) | - | 93 |
| Example F5 | (activated carbon + recrystallization) | - | 91 |
| Example F6 | (sublimation at 230 - 250 °C) | - | 95 |
| Example F7 | (sublimation at 300-320 °C) | - | 89 |
| Comparative Example F1 | (unpurified) | - | 79 |
| Comparative Example F2 | Toluene | <0.1 | 79 |
| Comparative Example F3 | (recrystallization under air) | - | 66 |

| | | | |
|---|---|---|---|
| Note: notes in parenthesis means method of purification, etc. | | | |

As can be seen from the results shown in Table F1, the i-BPDA according to the present invention having reduced color has a light transmittance, at 400 nm, of 80% or more, and preferably of 90% or more, and thus is preferable as a polyimide raw material for optical material applications.

### [Example F8]

In a reaction vessel, 1.40 g (0.0122 mol) of trans-1,4-diaminocyclohexane (t-DACH) purified by sublimation was charged and dissolved in 28.4 g of N,N-dimethylacetamide that had been dehydrated using a molecular sieve. The solution was heated to 50°C, and 3.25g (0.0110 mol) of s-BPDA and 0.36g (0.0012 mol) of i-BPDA were gradually added. The resultant mixture was stirred at 50°C for 6 hours to obtain a uniform and viscous polyimide precursor solution.

The obtained polyimide precursor solution was applied on a glass substrate, and thermally imidized by heating at 120 °C for 1 hour, at 150 °C for 30 minutes, at 200 °C for 30 minutes and at 350 °C for 5 minutes under nitrogen atmosphere to obtain a colorless transparent polyimide/glass laminate. Thus obtained co-polyimide/glass laminate was immersed in water for delamination to obtain a polyimide film with thickness of about 10 µm. Measurement results of properties of the film are shown in Table F2.

### [Comparative Example F4]

Polyimide precursor solution and polyimide film were obtained in the same manner as Example F9 except that unpurified i-BPDA powder was used as i-BPDA. Measurement results of film properties are shown in Table F2.

### [Example F9]

In a reaction vessel, 3.48 g (0.01 mol) of 1,4-bis(4-aminobenzoyloxy)benzene (BABB) treated with an activated carbon and purified by sublimation, and 36.41 g of N,N-dimethylacetamide dehydrated using a molecular sieve were charged, and the resultant mixture was dissolved at 50°C under a nitrogen flow. To the solution, 2.94 g (0.01 mol) of the i-BPDA obtained in Example F3 was gradually added, and the resultant mixture was stirred for 12 hours at 50°C to obtain a uniform and viscous polyimide precursor solution.

The obtained polyimide precursor solution was applied on a glass substrate, and thermally imidized by heating at 120 °C for 1 hour, at 150 °C for 30 minutes, at 200 °C for 30 minutes and at 350 °C for 5 minutes under nitrogen atmosphere to obtain a colorless transparent polyimide/glass laminate. Thus obtained polyimide/glass laminate was immersed in water for delamination to obtain a co-polyimide film with thickness of about 10 µm. Measurement results of properties of the film are shown in Table F2.

**Table F2**

| Chemical Composition | Light Transmittance at 400nm | | Example F8 | Example F9 | Comparative Example F4 |
|---|---|---|---|---|---|
| amine component | Transmittance 90% or more | t-DACH | 1.00 | | |
| | Transmittance 80% or more | BABB | | 1.00 | 1.00 |
| acid component | Transmittance 80% or more | i-BPDA (Example F3) | 0.10 | 1.00 | |
| | | s-BPDA | 0.90 | | |
| | Transmittance 80% or less | i-BPDA(unpurified ) | | | 1.00 |
| | | | | | |

| Evaluation results of Polyimide Precursor | | | | | |
|---|---|---|---|---|---|
| Logarithmic Viscosity (dL/g) | | | 1.36 | 0.24 | 0.21 |
| Light Transmittance at 400nm (%) | | | 90 | 59 | 47 |
| | | | | | |

| Evaluation results of Polyimide | | | | | |
|---|---|---|---|---|---|
| Light Transmittance at 400nm (%) | | | 81 | 80 | 72 |
| Elastic Modulus (GPa) | | | 4.7 | 2.2 | - |
| Coefficient of Thermal Expansion (ppm/K) | | | 21 | - | - |

| | | | | | |
|---|---|---|---|---|---|
| Note: Cell of Chemical Composition denotes molar ratio. - means non-execution. | | | | | |

The invention disclosed in Part F can provide a 2,2',3,3'-biphenyltetracarboxylic dianhydride having reduced color in which a 2,2',3,3'-biphenyltetracarboxylic dianhydride is a main component. Further, since the polyimide and precursor thereof that use the 2,2',3,3'-biphenyltetracarboxylic dianhydride according to the present invention can realize high transparency, the inventive polyimide and precursor can be especially preferably used as a transparent substrate in a display device, such as a flexible display, a touch panel and the like.

### «Examples of PART G »

The raw materials used in the respective following examples are as follows.
Trans-1,4-diaminocyclohexane: Manufactured by Zhejiang Taizhou Qingquan Medical & Chemical Co., Ltd., purity 99.1% (GC analysis).
1,4-Bis(4-aminobenzoyloxy)benzene (BABB): BABB, manufactured by Mikuni Pharmaceutical Industrial Co., Ltd.
3,3',4,4'-Biphenyltetracarboxylic dianhydride (s-BPDA): Manufactured by Ube Industries Ltd., purity 99.9% (purity determined by HPLC analysis of ring-opened 3,3',4,4'-biphenyltetracarboxylic acid), acid anhydride ratio 99.8%, Na, K, Ca, Al, Cu, Si: each < 0.1 ppm, Fe: 0.1 ppm, Cl: < 1 ppm.
2,3,3',4'-Biphenyltetracarboxylic dianhydride (a-BPDA): Manufactured by Ube Industries Ltd., purity 99.6% (purity determined by HPLC analysis of ring-opened 2,3,3',4'-biphenyltetracarboxylic acid), acid anhydride ratio 99.5%, Na, K, Al, Cu, Si: each < 0.1 ppm, Ca, Fe: each 0.1 ppm, Cl: < 1 ppm.
2,2',3,3'-Biphenyltetracarboxylic dianhydride (i-BPDA): Manufactured by Changzhou Weijia Chemical Co., Ltd., purity 99.9% (purity determined by HPLC analysis of ring-opened 2,2',3,3'-biphenyltetracarboxylic acid), acid anhydride ratio 99%.
4,4'-(2,2-Hexafluoroisopropylene)diphthalic dianhydride (6FDA): Manufactured by Daikin Industries, Ltd., purity 99%.
4,4'-(Dimethylsiladyl)diphthalic dianhydride (DPSDA): Manufactured by Toray Fine Chemicals Co., Ltd., purity 99.8% (purity determined by HPLC analysis of ring-opened 3,3',4, 4'-biphenyltetracarboxylic acid), acid anhydride ratio 99%.
Solvent: Product equivalent to reagent grade, analytical grade, or purified product thereof, manufactured by Wako Pure Chemical Industries, Ltd.
2 N Aqueous solution of sodium hydroxide: Aqueous solution of sodium hydroxide, manufactured by Tokyo Chemical Industry Co., Ltd.
Adsorption agent: Activated carbon, Norit SX Plus, manufactured by Japan Norit Inc., specific surface area based on BET method of 1,100 m²/g.

In each of the following examples, evaluation was carried out based on the following methods.

Evaluation of diamine powder and tetracarboxylic anhydride powder

### [Light Transmittance]

A predetermined amount of diamine powder or tetracarboxylic anhydride powder was dissolved in a measurement solvent to obtain a 10 mass% solution. Using a MCPD-300 manufactured by Otsuka Electronics Co., Ltd., and a standard cell having a light path length of 1 cm, the light transmittance at 400 nm of the diamine powder and the tetracarboxylic anhydride powder was measured using the measurement solvent as a blank.

### Evaluation of Polyimide Precursor

### [Logarithmic Viscosity]

The logarithmic viscosity was measured in the same manner as in Part A.

### [Light Transmittance (polyimide precursor)]

The polyimide precursor was diluted with N,N-dimethylacetamide so as to form a 10 mass% polyimide precursor solution. Then, using a MCPD-300 manufactured by Otsuka Electronics Co., Ltd., and a standard cell having a light path length of 1 cm, the light transmittance at 400 nm of the 10 mass% polyimide precursor solution was measured using N,N-dimethylacetamide as a blank.

### Evaluation of Polyimide

[Light Transmittance (polyimide)], [Elastic Modulus and Elongation at break], and [Coefficient of Thermal Expansion (CTE)] were measured in the same manner as in Part A.

### [Reference Example G1] Purification of t-DACH powder

In a glass sublimation apparatus, 10.0 g of unpurified trans-1,4-diaminocyclohexane was charged, and the pressure was then reduced to 1 Torr or less. The bottom wall surface with which the trans-1,4-diaminocyclohexane was in contact was heated to 50°C, whereby a sublimate was obtained on the opposite upper wall surface that had been adjusted to a temperature of 5°C. The yield was 8.2 g. The light transmittance results of the trans-1,4-diaminocyclohexane powder obtained by this method are shown in Table G1.

### [Reference Example G2] Purification of BABB powder

In a glass vessel, 20.0 g of BABB and 140 g of N,N-dimethylacetamide was charged, and the resultant mixture was dissolved by heating to 60°C. To the solution, 0.20 g of an adsorption agent (Norit SX Plus) was added, and then stirred for 2 hours. The adsorption agent was removed by filtration. Pure water was added to the resultant product, which was then cooled to 5°C, and the precipitate was collected. The obtained precipitate (10.0 g) was charged into a glass sublimation apparatus, and the pressure was reduced to 1 Torr or less. The bottom wall surface with which the BABB was in contact was heated to 300 to 350°C, whereby a sublimate was obtained on the opposite upper wall surface that had been adjusted to a temperature of 25°C. The yield was 8.5 g. The light transmittance results of the BABB obtained by this method are shown in Table G1.

### [Reference Example G3] Purification of s-BPDA powder

In a glass vessel, 10.0 g of unpurified s-BPDA and 10.0 g of N-methyl-2-pyrrolidone as a solvent were charged, and the resultant mixture was thoroughly stirred at 25°C for 3 hours. The solution was separated by filtration, and the obtained solid was dried in vacuo for 2 hours at 100°C to obtain s-BPDA powder having reduced color. The light transmittance results are shown in Table G1.

### [Reference Example G4] Purification of a-BPDA powder

In a glass vessel, 10.0 g of unpurified a-BPDA and 10.0 g of acetone as a solvent were charged, and the resultant mixture was thoroughly stirred at 25°C for 3 hours. The solution was separated by filtration, and the obtained solid was dried in vacuo for 2 hours at 100°C to obtain 9.4 g of a-BPDA having reduced color. The light transmittance results are shown in Table G1.

### [Reference Example G5] Purification of i-BPDA powder

In a glass vessel, 10.0 g of unpurified i-BPDA and 10.0 g of N-methyl-2-pyrrolidone as a solvent were charged, and the resultant mixture was thoroughly stirred at 25°C for 3 hours. The solution was separated by filtration, and the obtained solid was dried in vacuo for 2 hours at 100°C to obtain i-BPDA having reduced color. The light transmittance results are shown in Table G1.

**Table G1**

| | Compound Name | Transmittance at 400 nm (%) | | |
|---|---|---|---|---|
| | | Measurement Solvent | Unpurified | Purified |
| Referential Example G1 | t-DACH | pure water | 86 | 96 |
| Referential Example G2 | BABB | N,N-dimethylacetamide | 69 | 82 |
| Referential Example G3 | s-BPDA | 2 N aqueous solution of NaOH | 75 | 82 |
| Referential Example G4 | a-BPDA | 2 N aqueous solution of NaOH | 79 | 95 |
| Referential Example G5 | i-BPDA | 2 N aqueous solution of NaOH | 79 | 93 |
| | 6FDA | 2 N aqueous solution of NaOH | 81 | |
| | DPSDA | 2 N aqueous solution of NaOH | 97 | |

### [Example G1]

In a reaction vessel, 1.40 g (0.0122 mol) of trans-1,4-diaminocyclohexane (t-DACH) purified in the same manner as in Reference Example G1 was charged, and dissolved in 28.4 g of N,N-dimethylacetamide that had been dehydrated using a molecular sieve. The solution was heated to 50°C, and then 3.50 g (0.0119 mol) of 3,3',4,4'-biphenyltetracarboxylic dianhydride (s-BPDA) purified in the same manner as in Reference Example G3 and 0.09 g (0.0003 mol) of a-BPDA purified in the same manner as in Reference Example G4 were gradually added. The resultant mixture was stirred for 6 hours at 50°C to obtain a uniform and viscous polyimide precursor solution.

The obtained polyimide precursor solution was applied on a glass substrate, and thermally imidized by heating at 120 °C for 1 hour, at 150 °C for 30 minutes, at 200 °C for 30 minutes and at 350 °C for 5 minutes under a nitrogen atmosphere to obtain a colorless transparent polyimide/glass laminate. Thus obtained co-polyimide/glass laminate was immersed in water for delamination to obtain a polyimide film with thickness of about 10 µm. Measurement results of properties of the film are shown in Table G2.

### [Examples G2 to G6]

A polyimide precursor solution and polyimide film were obtained in the same manner as in Example G1, except that the diamine component and acid component were used as indicated in Table G2. Measurement results of properties are shown in Table G2.

### [Comparative Examples G1 to G3]

A polyimide precursor solution and polyimide film were obtained in the same manner as in Example G1, except that the diamine component and acid component were used as indicated in Table G2. Measurement results of properties are shown in Table G2.

**Table G2**

| Chemical Composition | | Transmittance at 400 nm | | Ex. G1 | Ex. G2 | Ex. G3 | Ex. G4 | Ex. G5 | Ex. G6 | Comp. Ex. G1 | Comp. Ex. G2 | Comp. Ex. G3 |
|---|---|---|---|---|---|---|---|---|---|---|---|---|
| amine component | No Aromatic Ring | 90% or more | t-DACH purified in Reference Example G1 | 1.00 | 1.00 | 1.00 | | 1.00 | 1.00 | | 1.00 | |
| | | 90% or less | t-DACH | | | | | | | 1.00 | | |
| | with Aromatic Ring | 80% or more | BABB purified in Reference Example G2 | | | | 1.00 | | | | | |
| | | 80% or less | BABB | | | | | | | | | 1.00 |
| acid component | | 80% or more | s-BPDA purified in Reference Example G3 | 0.975 | 0.90 | 0.90 | | | | 0.975 | | |
| | | | a-BPDA purified in Reference Example G4 | 0.025 | | 0.10 | | | | 0.025 | 0.025 | |
| | | | i-BPDA purified in Reference Example G5 | | 0.10 | | 1.00 | | | | | |
| | | | 6FDA | | | | | 1.00 | | | | |
| | | | DPSDA | | | | | | 1.00 | | | |
| | | 80% or less | s-BPDA | | | | | | | | 0.975 | |
| | | | i-BPDA | | | | | | | | | 1.00 |
| | | | | | | | | | | | | |

| Evaluation results of Polyimide Precursor | | | | | | | | | | | | |
|---|---|---|---|---|---|---|---|---|---|---|---|---|
| Logarithmic Viscosity (dL/g) | | | | 1.61 | 1.36 | 1.29 | 0.24 | 0.79 | 1.37 | 1.42 | 1.54 | 0.21 |
| Light Transmittance at 400nm (%) | | | | 90 | 90 | 90 | 59 | 91 | 96 | 82 | 89 | 47 |
| | | | | | | | | | | | | |

| Evaluation results of Polyimide | | | | | | | | | | | | |
|---|---|---|---|---|---|---|---|---|---|---|---|---|
| Light Transmittance at 400nm (%) | | | | 81 | 81 | 82 | 80 | 90 | 91 | 78 | 78 | 72 |
| Elastic Modulus (GPa) | | | | 6.1 | 4.7 | 3.8 | 2.2 | 2.8 | 2.6 | 6.3 | 6.1 | - |
| Coefficient of Thermal Expansion (ppm/K) | | | | 9.1 | 20 | - | - | - | - | - | - | - |

| | | | | | | | | | | | | |
|---|---|---|---|---|---|---|---|---|---|---|---|---|
| Note: Cell of Chemical Composition denotes molar ratio. - means non-execution. | | | | | | | | | | | | |

As can be seen from the results shown in Table G2, the polyimide according to the present invention has a light transmittance at 400 nm of 80% or more, and thus is preferable as a polyimide for optical material applications.

According to the invention disclosed in Part G, provided is a polyimide having excellent transparency, high mechanical strength, and low coefficient of linear thermal expansion suitable for a transparent base material for a flexible display, solar cell, or touch panel and to provide a polyimide precursor of the polyimide.

### «Examples of PART H »

Abbreviation, purity, pretreatment and the like of the raw materials used in the respective following examples are as follows.

### [Diamine component]

1,4-t-DACH: Trans-1,4-diaminocyclohexane with purity 99.1% (GC analysis).
1,2-t-DACH: Trans-1,2-diaminocyclohexane.
ODA: 4,4'-oxydianiline with purity 99.9% (GC analysis).
DABAN: 4,4'- diaminobenzanilide with purity 99.90% (GC analysis).
4-APTP: N,N'-bis (4-aminophenyl)terephthalamide with purity 99.95% (GC analysis).
AZDA: 2,4-Bis (4 - aminoanilino) -6 - anilino-1,3,5-Triazine with purity 99.9% (GC analysis).
BABB: 1,4-Bis(4-aminobenzoyloxy)benzene with purity 99.8% (GC analysis).

### [Tetracarboxylic acid component]

s-BPDA: 3,3',4,4'-Biphenyltetracarboxylic dianhydride with purity 99.9% (purity determined by HPLC analysis of ring-opened 3,3',4,4'-biphenyltetracarboxylic acid) and acid anhydride ratio 99.8%, Na, K, Ca, Al, Cu, Si: each < 0.1 ppm, Fe: 0.1 ppm, Cl: < 1 ppm.
a-BPDA: 2,3,3',4'-Biphenyltetracarboxylic dianhydride with purity 99.6% (purity determined by HPLC analysis of ring-opened 2,3,3',4'-biphenyltetracarboxylic acid), acid anhydride ratio 99.5%, Na, K, Al, Cu, Si: each < 0.1 ppm, Ca, Fe: each 0.1 ppm, Cl: < 1 ppm.
i-BPDA: 2,2',3,3'-Biphenyltetracarboxylic dianhydride with purity 99.9% (purity determined by HPLC analysis of ring-opened 2,2',3,3'-biphenyltetracarboxylic acid) and acid anhydride ratio 99%.
6FDA: 4,4'-(2,2-Hexafluoroisopropylene)diphthalic dianhydride with purity 99%.
ODPA: 4,4'-oxydiphthalic dianhydride with purity 100% (purity determined by HPLC analysis of ring-opened 4,4'-oxydiphthalic acid) and acid anhydride ratio 99.8 %.
DPSDA: 4,4'- (dimethylsiladiyl)diphthalic dianhydride with purity 99.8 % (purity determined by HPLC) and acid anhydride ratio 99 %.
PMDA: Pyromellitic dianhydride.
s-BPTA: 3,3',4,4'- biphenyltetracarboxylic acid.
PMDA-H: 1R,2S,4S,5R-Cyclohexane tetracarboxylic dianhydride with purity 92.7% (GC analysis); purity as hydrogenated Pyromellitic dianhydride is 99.9 % (GC analysis).
BTA-H: bicyclo[2.2.2]octane-2,3:5,6-tetracarboxylic dianhydride with purity 99.9% (GC analysis).
BPDA-H: 3,3',4,4'- bicyclohexyltetracarboxylic dianhydride 99.9% (GC analysis).

### [Solvent]

NMP: N-methyl-2-pyrrolidone that was, if necessary, subjected to purification such as rectification distillation, and dehydrated using a molecular sieve.
DMAc: N,N-dimethylacetamide that was, if necessary, subjected to purification such as rectification distillation, and dehydrated using a molecular sieve.
DMI: 1,3-Dimethyl-2-imidazolidinone that was, if necessary, subjected to purification such as rectification distillation, and dehydrated using a molecular sieve.

In each of the following examples, evaluation was carried out based on the following methods.

### Evaluation of Solvent

### [GC Analysis: Solvent Purity]

The solvent purity was measured under the following conditions using a GC-2010 manufactured by Shimadzu Corporation. The purity (GC) was determined from the peak surface area fraction.
Column: DB-FFAP manufactured by J&W, 0.53 mm ID x 30 m Temperature: 40°C (5 minutes holding) + 40°C to 250°C (10 minutes/minutes) + 250°C (9 minutes holding)
Inlet temperature: 240°C
Detector temperature: 260°C
Carrier gas: Helium (10 ml/minute)
Injection amount: 1 µL

### [Non-volatile Content]

5g of solvent was weighed in a glass vessel and heated at 250°C for 30 minutes in a hot air circulating oven. The vessel was cooled and the residual matter was weighed. From the mass of the residual matter, the non-volatile content (mass %) in the solvent is determined.

### [Light Transmittance]

Using a MCPD-300 manufactured by Otsuka Electronics Co., Ltd., and a standard cell having a light path length of 1 cm, the light transmittance of a solvent at 400 nm was measured using water as a blank.

For light transmittance after heating with refluxing, measurement was carried out using a solvent that had been heated to reflux for 3 hours under nitrogen atmosphere having oxygen concentration of 200 ppm or less.

### [Quantification of Metal Component]

The metal content contained in the solvent was quantified based on inductively coupled plasma mass spectrometry (ICP-MS) using an Elan DRC II manufactured by PerkinElmer Inc.

### Evaluation of Polyimide Precursor Varnish and Polyimide Varnish

### [Varnish Solid Content]

One gram of solution (varnish) was weighed into an aluminum dish, heated for 2 hours in a 200°C hot air circulating oven to remove the non-solid content. The varnish solid content (heating residue mass%) was determined from the residual matter.

### [Rotational Viscosity]

The viscosity of the solution (varnish) at a temperature of 25°C and a shear rate of 20 sec⁻¹ was determined using a TV-22 E-type rotary viscometer manufactured by Toki Sangyo Co., Ltd.

### [Logarithmic Viscosity]

The logarithmic viscosity was determined by measuring a 0.5 g/dL solution of the varnish in N,N-dimethylacetamide at 30°C using an Ubbelohde viscometer.

### Evaluation of Polyimide Film

### [Light Transmittance]

The light transmittance at 400 nm of a polyimide film with a thickness of about 10 µm was measured using a MCPD-300 manufactured by Otsuka Electronics Co., Ltd.

### [Elastic Modulus and Elongation at break]

The initial elastic modulus and elongation at break for a chuck interval of 30 mm and a tension rate of 2 mm/min were measured using a Tensilon manufactured by Orientec Co., Ltd., on a test piece produced by punching a polyimide film into an IEC450 standard dumbbell shape.

### [Coefficient of Thermal Expansion (CTE)]

A test piece was produced by cutting a polyimide film into a strip having 4 mm width. Then, using a TMA-50 manufactured by Shimadzu Corporation, the temperature of the test piece was increased to 300°C at a rate of temperature increase of 20°C/min with a chuck interval of 15 mm and a load of 2 g. The average coefficient of thermal expansion from 50°C to 200°C was determined from the obtained TMA curve.

### [Referential Example H1]

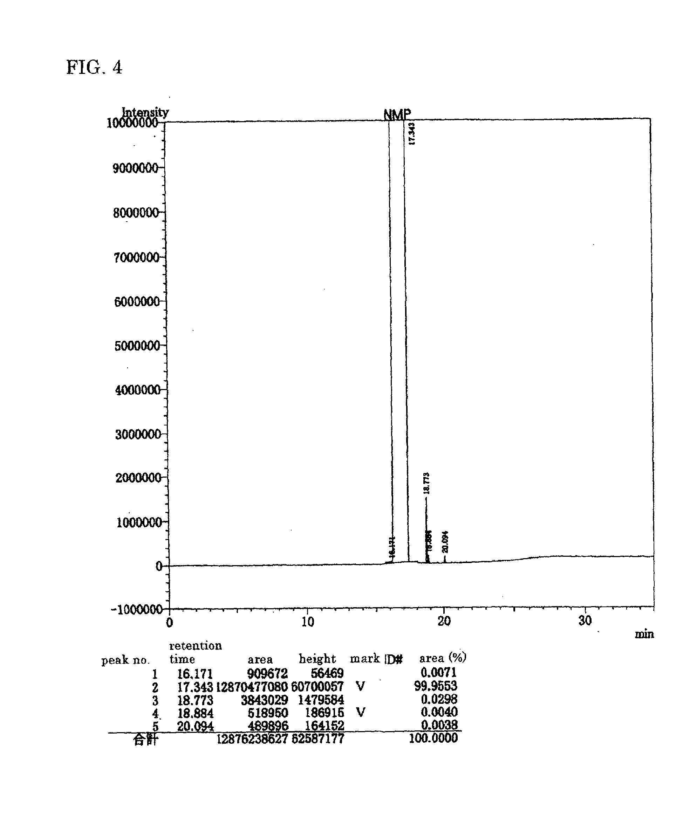

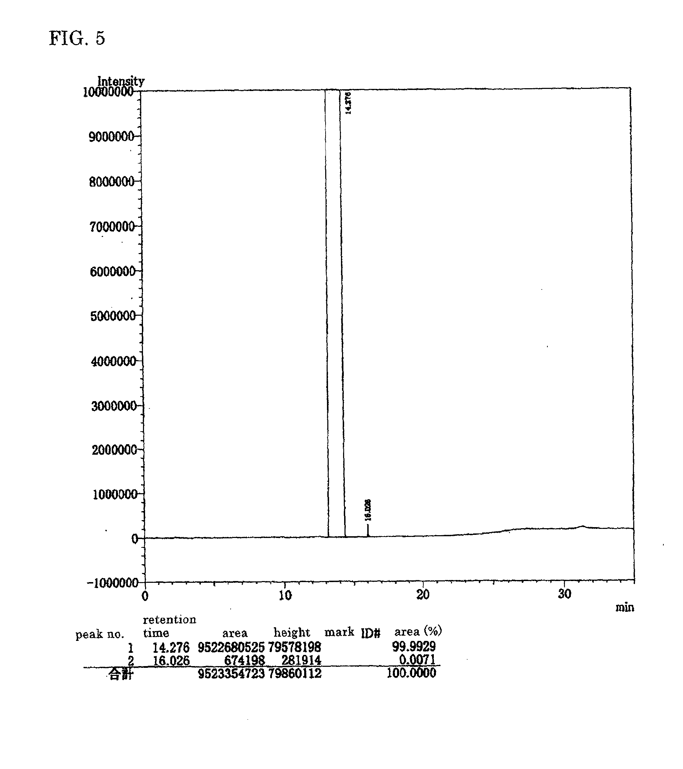

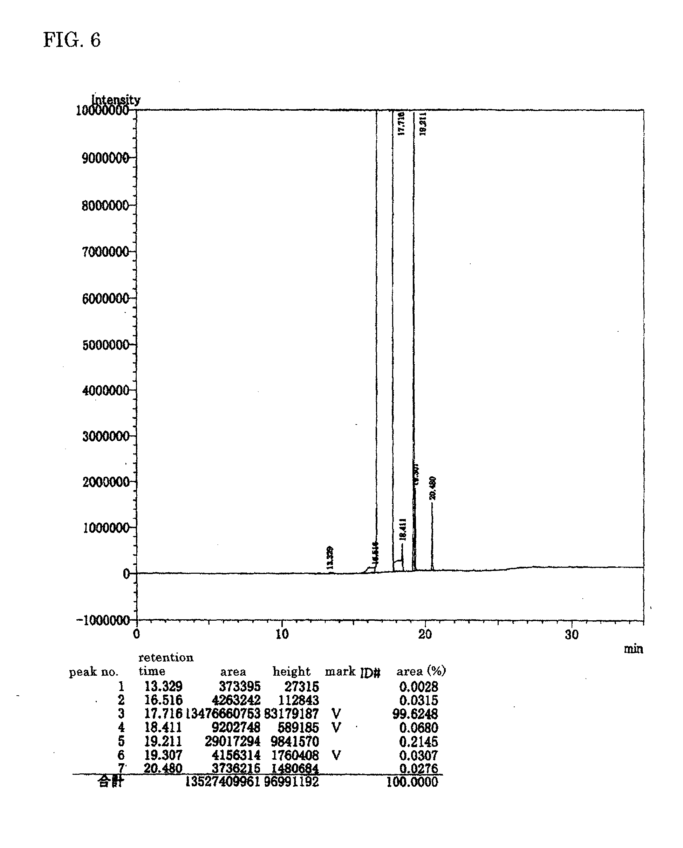

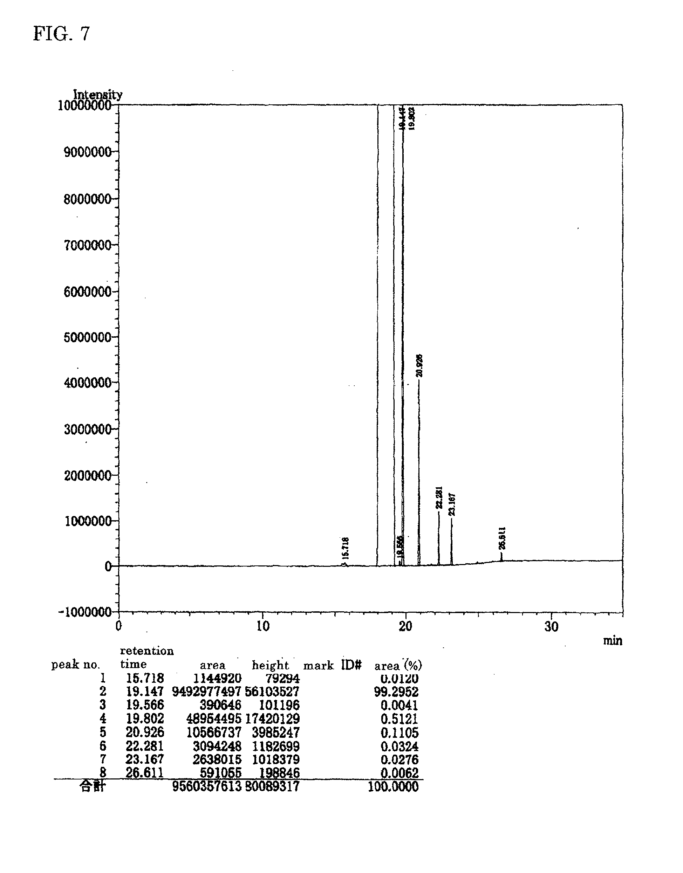

The results of evaluation of solvents used for the production of polyimide precursor varnishes and polyimide varnishes are shown in Table H1. Also, Figs 4 to 7 show the result of GC analysis for N-methyl-2-pyrrolidone(NMP) purity 99.96% (Fig. 4), N,N-dimethylacetamide (DMAc) purity 99.99% (Fig. 5), N-methyl-2-pyrrolidone (NMP) purity 99.62% (Fig. 6), and 1,3-Dimethyl-2-imidazolidinone (DMI) purity 99.30% (Fig. 7).

### [Example H1]

In a reaction vessel, 1.40 g (0.0122 mol) of trans-1,4-diaminocyclohexane (t-DACH) was charged and dissolved in 28.4 g of N-methyl-2-pyrrolidone (purity 99.96) under nitrogen atmosphere. The solution was heated to 50°C, and 3.50g (0.0119 mol) of 3,3',4,4'-biphenyltetracarboxylic dianhydride (s-BPDA) and 0.09g (0.0003 mol) of 2,3,3',4'-biphenyltetracarboxylic dianhydride (a-BPDA) were gradually added. The resultant mixture was stirred at 50°C for 6 hours to obtain a uniform and viscous polyimide precursor varnish. Measurement results of properties of the varnish are shown in Table H2.

The obtained polyimide precursor solution was applied on a glass substrate, and thermally imidized by heating at 120 °C for 1 hour, at 150 °C for 30 minutes, at 200 °C for 30 minutes and at 350 °C for 5 minutes under nitrogen atmosphere to obtain a colorless transparent polyimide/glass laminate. Thus obtained co-polyimide/glass laminate was immersed in water for delamination to obtain a polyimide film with thickness of about 10 µm. Measurement results of properties of the film are shown in Table H2.

### [Examples H2 to H18]

Polyimide precursor solution and polyimide film were obtained in the same manner as Example H1 except that diamine component, an acid component and solvent indicated in Table H2 were used. Measurement results of properties are shown in Table H2.

### [Example H19]

In a reaction vessel, 3.00g (0.026 mol) of trans-1,4-diaminocyclohexane was charged and dissolved in 60.35 g of N,N-dimethylacetamide (purity 99.99). To the mixture, 5.55g (0.0273 mol) of N,O-bis(trimethylsilyl)acetamide was added and stirred at 80 °C for 2 hours to perform silylation. After cooling the solution to 40 °C, 6.77g (0.023 mol) of 3,3',4,4'-biphenyltetracarboxylic dianhydride and 0.88g(0.003 mol) of 2,3,3',4'-biphenyltetracarboxylic dianhydride was added. The mixture was stirred at 40 °C and all solids were dissolved in 1 hour. The mixture was further stirred for 8 hours to obtain a uniform and viscous polyimide precursor varnish.

The obtained polyimide precursor varnish was applied on a glass substrate, and thermally imidized by heating at 120 °C for 1 hour, at 150 °C for 30 minutes, at 200 °C for 30 minutes and at 350 °C for 3 minutes while holding it on the substrate under nitrogen atmosphere (oxygen concentration is 200 ppm or less) to obtain a colorless transparent co-polyimide/glass laminate. Thus obtained co-polyimide/glass laminate was immersed in water for delamination to obtain a co-polyimide film with thickness of about 10 µm. Measurement results of properties of the film are shown in Table H2.

### [Example H20]

In a reaction vessel, 1.742g (0.005mol) of 1,4-bis(4-aminobenzoyloxy)benzene (BABB) and 22.44 g of N,N-dimethylacetamide (purity 99.99%) dehydrated using a molecular sieve were charged, and the resultant mixture was dissolved at room temperature (25°C) under a nitrogen flow. To the solution, 2.70g (0.0105 mol) of N,O-bis(trimethylsilyl)trifluoro acetamide (BSTFA) and 0.79g (0.01mol) of pyridine were added and stirred for 2 hours to perform silylation. To the solution, 2.223g (0.005 mol) of 4,4'-(2,2-hexafluoroisopropylidene)diphthalic dianhydride (6FDA) was gradually added and stirred for 12 hours at room temperature (25 °C) to obtain a uniform and viscous polyimide precursor varnish.

The obtained polyimide precursor varnish was applied on a glass substrate, and thermally imidized by heating at 100 °C for 15 minutes, at 200 °C 60 minutes, at 300 °C 10minutes while holding it on the substrate to obtain a colorless transparent polyimide/glass laminate. Thus obtained co-polyimide/glass laminate was immersed in water for delamination to obtain a film with thickness of about 10 µm and the properties of the film were measured. The results are shown in Table H2.

### [Example H21]

In a reaction vessel purged with nitrogen gas, 2.00g (10 mmol) of 4,4'-oxydianiline was charged and dissolved in 24.03g of N,N-dimethylacetamide that had been dehydrated using a molecular sieve of such an amount that the feeding amount of monomers (total amount of diamine component and carboxylic acid component) is 15 % by mass and stirred at 50 °C for 2 hours.

To the solution, 2.24g (10 mmol) of PMDA-H was gradually added. The solution was stirred at 50 °C for 6 hours and heated to 160 °C. The solution was stirred while removing produced water by Dean-Stark trap. After the completion of imidization reaction was confirmed by infrared spectroscopy measurement, the solution was cooled to room temperature to obtain a uniform and viscous polyimide precursor varnish.

Measurement results of properties of the varnish are shown in Table H2.

The polyimide precursor varnish that was filtered using a PTFE membrane filter was applied on a glass substrate, and thermally imidized by heating at 120 °C for 1 hour, at 150 °C for 30 minutes, at 200 °C for 30 minutes, then heating up and at 350 °C for 5 minutes while holding it on the substrate under nitrogen atmosphere (oxygen concentration is 200 ppm) to obtain a colorless transparent polyimide/glass laminate. Thus obtained polyimide/glass laminate was immersed in water for delamination to obtain a polyimide film with thickness of about 10 µm.

Measurement results of properties of the film are shown in Table H2.

### [Comparative Examples H1 and H2]

Polyimide precursor solutions and polyimide films were obtained in the same manner as Example H1 except that diamine components, acid components and solvents indicated in Table H2 were used. Measurement results of properties are shown in Table H2.

**Table H1**

| Solvent | | NMP (99.96%) | DMAc (99.99%) | NMP (99.62%) | DMI (99.30%) |
|---|---|---|---|---|---|
| GC Analysis | retention time of main component (min) | 17.34 | 14.28 | 17.72 | 15.72 |
| | Area of main component % | 99.9553 | 99.9929 | 99.6248 | 99.2952 |
| | Peak area of impurities of short retention time, % | 0.0071 | 0.0000 | 0.0343 | 0.0120 |
| | Peak area of impurities of long retention time, % | 0.0376 | 0.0071 | 0.3408 | 0.6929 |
| Non-volatile Content | Mass % | 0.002 | < 0.001 | 0.126 | 0.162 |
| Light Transmittance | Light Transmittance at 400nm (%) | 89 | 92 | 88 | |
| | Light Transmittance at 400nm after reflux (%) | 41 | 92 | 0.1 | |
| Metal Content | Na (ppb) | 120 | | | |
| | Fe (ppb) | < 2 | | | |
| | Cu (ppb) | < 2 | | | |
| | Mo (ppb) | < 1 | | | |

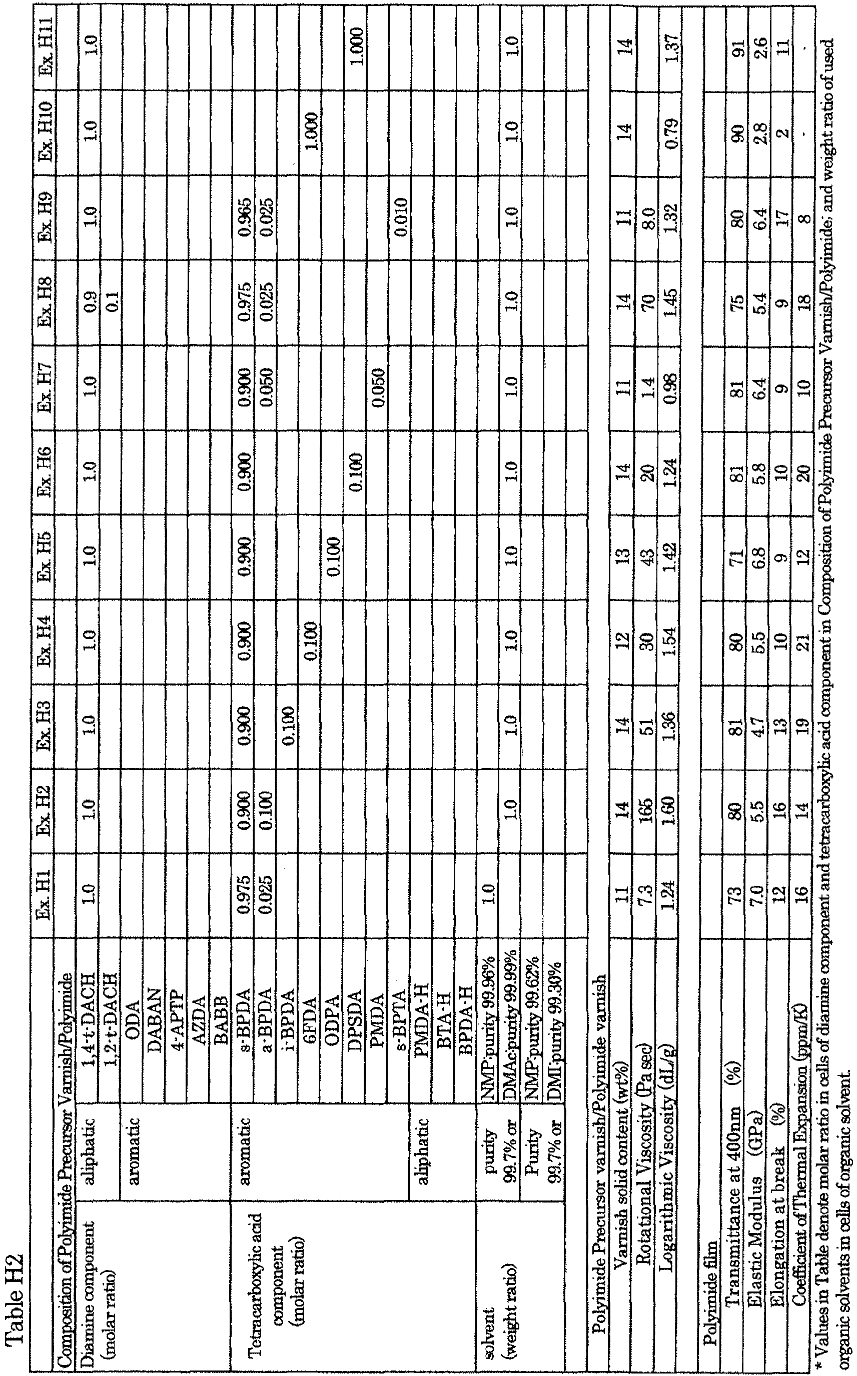

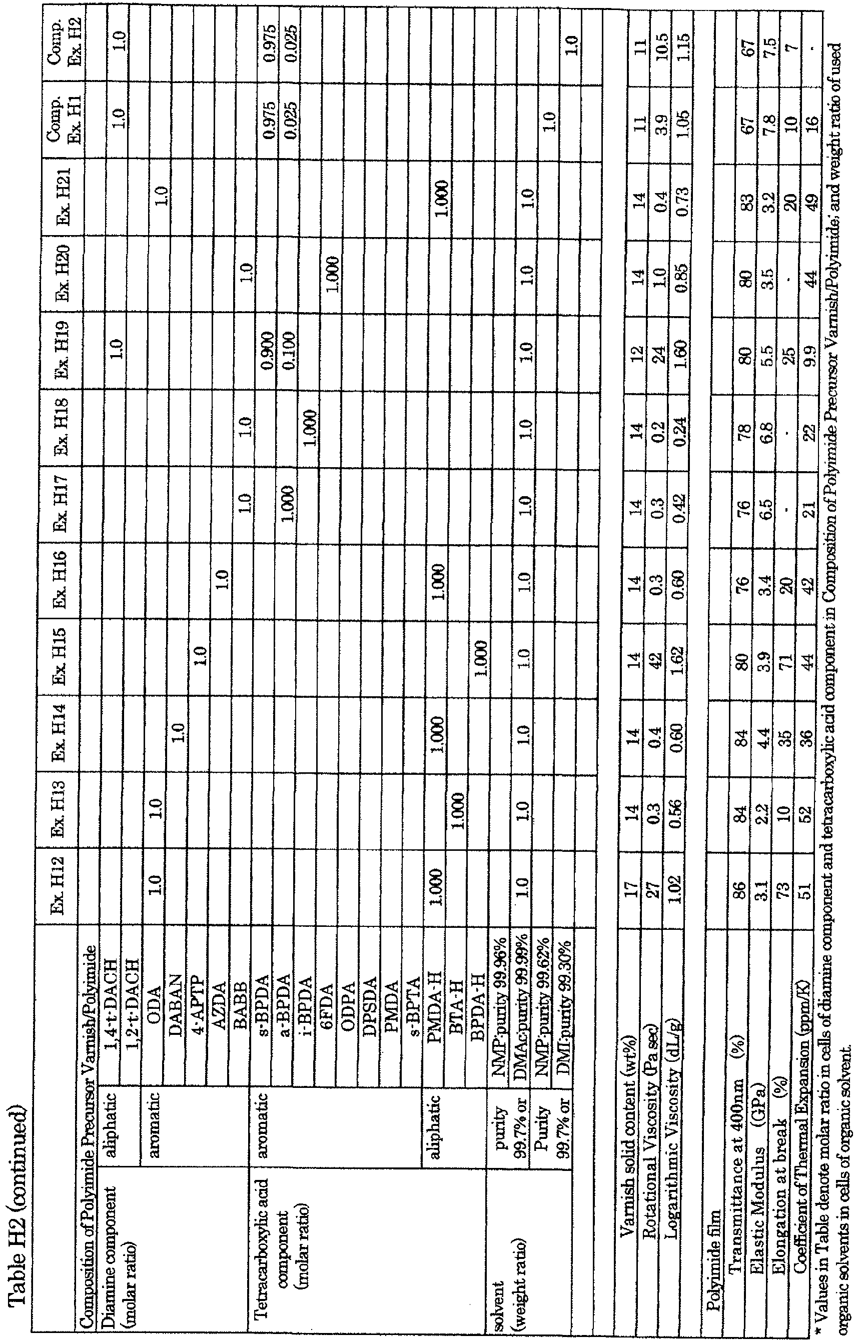

As can be seen from the results shown in Table H2, the polyimide film obtained from the polyimide precursor varnish or the polyimide varnish according to the present invention has a light transmittance of 70% or more at 400 nm and thus is preferable as a polyimide for optical material applications. In contrast, when the polyimide precursor varnish was produced by using an unsuitable solvent, the light transmittance at 400 nm decreased below 70 % and yellowish coloration was observed.

### [Examples H22 to H31 and Comparative Examples H3 and H4]

Polyimide precursor solutions and polyimide films were obtained in the same manner as Example H1 except that solvents indicated in Table H3 were used. Measurement results of properties are shown in Table H3.

**Table H3**

| | | Ex. H22 | Ex. H23 | Ex. H24 | Ex. H25 | Ex. H26 | Ex. H27 | Ex. H28 | Ex. H29 | Ex. H30 | Ex. H31 | Comp. Ex. H3 | Comp. Ex. H4 |
|---|---|---|---|---|---|---|---|---|---|---|---|---|---|
| solvent | | DMAc | DMAc | DMAc | DMAc | DMAc | DMAc | DMAc | DMAc | NMP | DMAc/NMP Mixed solvent of 3/1 molar ratio | NMP | NMP |
| Evaluation of Solvent | | | | | | | | | | | | | |
| GC Analysis | Purity (Area of main component %) | 99.99 | 99.98 | 99.95 | 99.93 | 99.90 | 99.88 | 99.85 | 99.84 | 99.96 | 99.98 | 99.77 | 99.62 |
| | Impurities of long retention time(Peak area %) | 0.00 | 0.02 | 0.03 | 0.06 | 0.09 | 0.12 | 0.08 | 0.16 | 0.04 | 0.02 | 0.21 | 0.34 |
| Non-volatile Content | Residual matter after heating (mass %) | 0.001 | | | | | | | | 0.002 | 0.001 | | 0.126 |
| Light Transmittance | Light Transmittance at 400nm (%) | 92 | 92 | 92 | 92 | 91 | 91 | 90 | 91 | 89 | 91 | 87 | 88 |
| | Light Transmittance after reflux (%) | 92 | 92 | 92 | 92 | | | | | 41 | 86 | 9 | 0.14 |
| metal content | Na (ppb) | | 120 | 110 | 93 | | | | | 66 | | | |
| | Fe (ppb) | | <2 | <2 | <2 | | | | | <2 | | | |
| | Cu (ppb) | | <2 | 6.7 | <2 | | | | | <2 | | | |
| | Mo (ppb) | | <1 | <1 | <1 | | | | | <1 | | | |
| Evaluation of Polyimide film | | | | | | | | | | | | | |
| Light Transmittance at 400nm (%) | | 80 | 78 | 77 | 81 | 79 | 79 | 77 | 75 | 73 | 76 | 65 | 59 |
| Elastic Modulus (GPa) | | 6.6 | 6.0 | 6.8 | 6.7 | 6.4 | 5.9 | 6.5 | 6.8 | 7.0 | 7.2 | 7.3 | 8.2 |
| Elongation at break (%) | | 14.2 | 24.0 | 12.8 | 11.3 | 16.0 | 23.9 | 20.6 | 9.7 | 11.7 | 18.3 | 11.8 | 4.5 |
| Breaking Strength (MPa) | | 288 | 259 | 247 | 228 | 253 | 269 | 358 | 261 | 265 | 293 | 287 | 246 |
| Coefficient of Thermal Expansion (ppm/K) | | 11.0 | 11.7 | 9.4 | 11.8 | 12.2 | 11.5 | 11.7 | 8.7 | 15.9 | 8.7 | 9.0 | 7.9 |

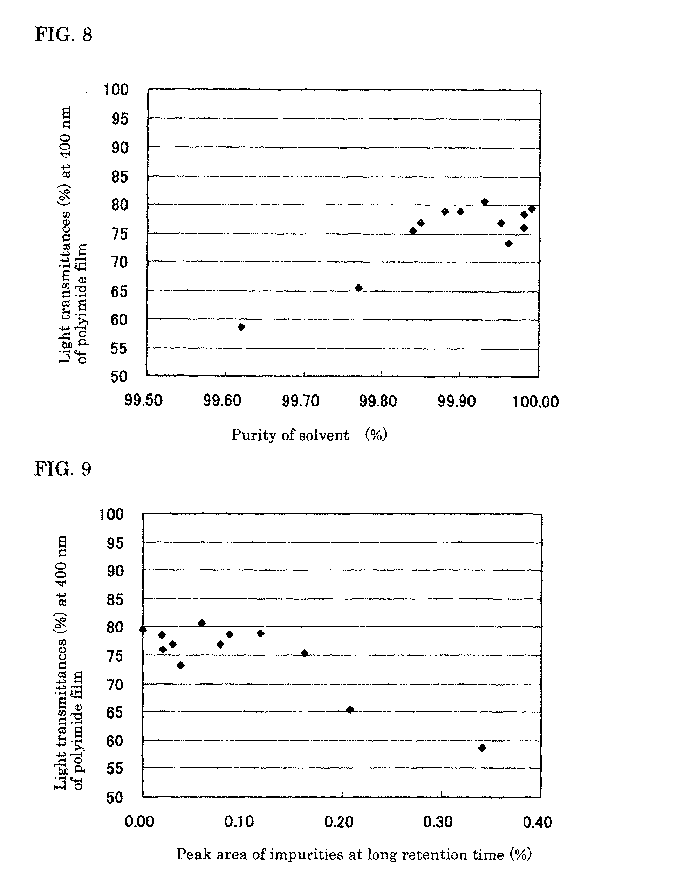

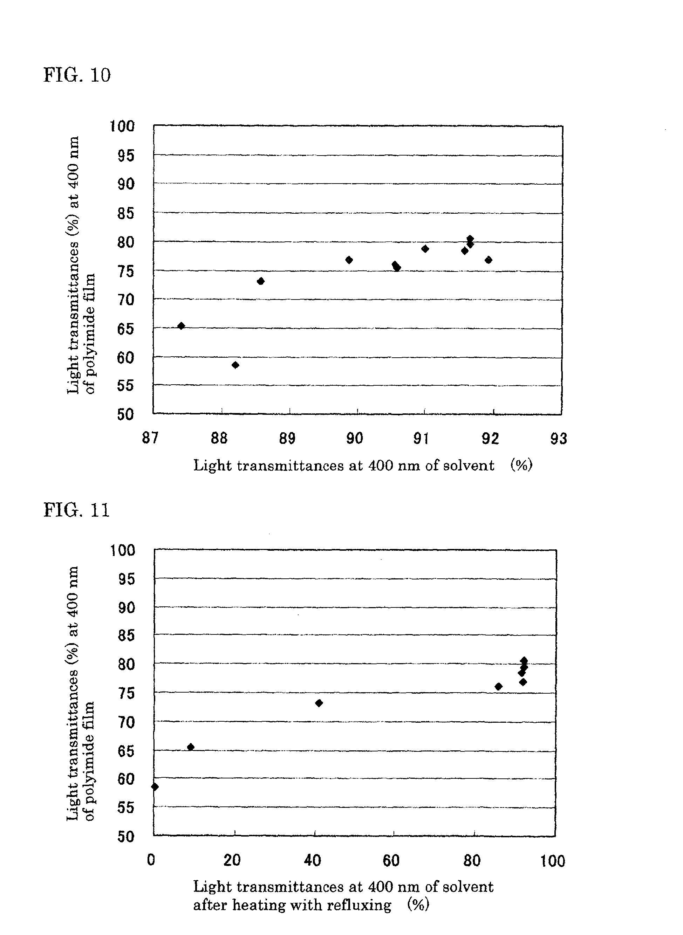

Figure 8 shows a relationship between the purity (%) of solvents and the light transmittances (%) at 400 nm of polyimide films; Figure 9 shows a relationship between the peak area (%) of impurities at long retention time and the light transmittances (%) at 400 nm of polyimide films; Figure 10 shows a relationship between the light transmittances (%) at 400 nm of solvents and the light transmittances (%) at 400 nm of polyimide films; and Figure 11 shows a relationship between the light transmittances (%) at 400 nm of solvents after heating with refluxing and the light transmittances (%) at 400 nm of polyimide films.

The invention disclosed in Part H can provide a method of producing a polyimide precursor varnish and a polyimide varnish that can prepare polyimides having high transparency. These polyimide precursor varnish and polyimide varnish can be suitably used as transparent heat resistant base materials for a flexible display, solar cell, or touch panel.

## Claims

1. A co-polyimide precursor, comprising a unit structure represented by general Formula (A1) and a unit structure represented by general Formula (A2): 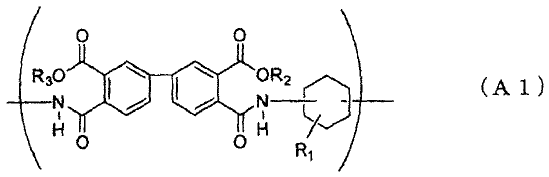 wherein, in general Formula (A1), R₁ represents a hydrogen atom or an alkyl group having 1 to 4 carbon atoms; and R₂ and R₃ each independently represent a hydrogen atom, an alkyl group having 1 to 6 carbon atoms, or an alkylsilyl group having 3 to 9 carbon atoms, 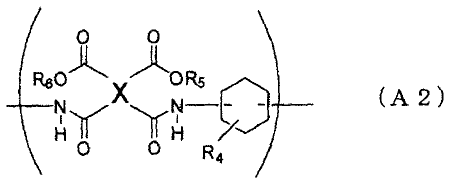 wherein, in general Formula (A2), R₄ represents a hydrogen atom or an alkyl group having 1 to 4 carbon atoms; R₅ and R₆ each independently represent a hydrogen, an alkyl group having 1 to 6 carbon atoms, or an alkylsilyl group having 3 to 9 carbon atoms; and X represents a tetravalent group other than those represented by Formulae (A3):

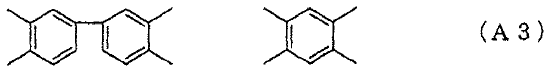

2. The co-polyimide precursor according to Claim 1, wherein the number ratio of the unit structures represented by general Formula (A1) to the unit structures represented by general Formula (A2) [the number of unit structures represented by general Formula (A1)/the number of unit structures represented by general Formula (A2)] is 50/50 to 99.5/0.5.

3. The co-polyimide precursor according to Claim 1 or 2, wherein X in general Formula (A2) is any one of tetravalent groups shown as Formulae (A4): 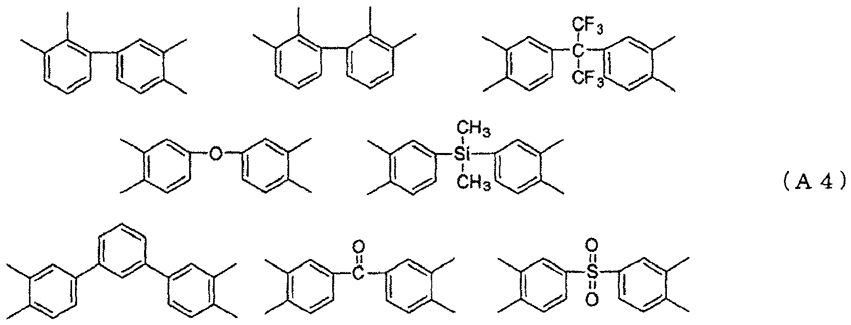 or a mixture thereof.

4. The co-polyimide precursor according to any one of Claims 1 to 3, having a logarithmic viscosity of 0.2 dL/g or more as a 0.5 g/dL solution in N,N-dimethylacetamide at 30°C.

5. A method of producing a co-polyimide precursor according to any one of Claims 1 to 4, comprising reacting a diamine component and a tetracarboxylic acid component in a solvent at temperature of 100°C or less.

6. The method of producing a co-polyimide precursor according to Claim 5, wherein the solvent used has a purity (a purity determined by GC analysis) of 99.8% or more.

7. A method of producing a solution composition of the co-polyimide precursor according to Claim 5 or 6, comprising reacting a tetracarboxylic acid component and a diamine component at a molar ratio such that the diamine component is excess to obtain a polyimide precursor; and further adding a carboxylic acid derivative in an amount approximately corresponding to the number of excess moles of the diamine to the resulting polyimide precursor such that the total molar proportion of the tetracarboxylic acid and the carboxylic acid derivative component is approximately equivalent to the molar proportion of the diamine component.

8. A co-polyimide having a unit structure represented by general Formula (A5) and a unit structure represented by general Formula (A6): 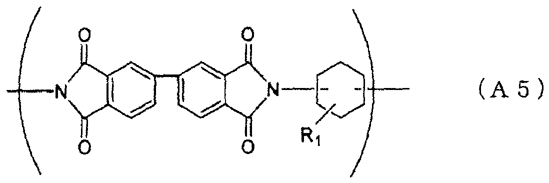 wherein, in general Formula (A5), R₁ represents a hydrogen atom or an alkyl group having 1 to 4 carbon atoms, 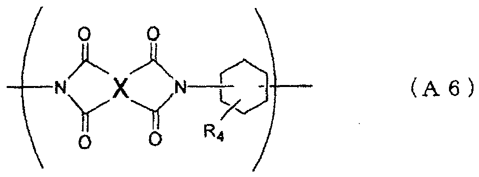 wherein, in general Formula (A6), R₄ represents a hydrogen atom or an alkyl group having 1 to 4 carbon atoms; and X represents a tetravalent group other than those represented by Formulae (A3).

9. The co-polyimide according to Claim 8, wherein the number ratio of the unit structures represented by general Formula (A5) to the unit structures represented by general Formula (A6) [the number of unit structures represented by general Formula (A5)/the number of unit structures represented by general Formula (A6)] is 50/50 to 99.5/0.5.

10. The co-polyimide according to Claim 8 or 9, wherein X in general Formula (A6) is any one of tetravalent groups shown as Formulae (A4) or a mixture thereof.

11. The co-polyimide according to any one of Claims 8 to 10, having toughness corresponding to an elongation at break at room temperature of 8% or more and transparency corresponding to a light transmittance at 400 nm of 50% or more when formed into a film having a thickness of 10 µm.

12. The co-polyimide according to any one of Claims 8 to 11, having an elastic modulus at room temperature of 3 GPa or more, toughness corresponding an elongation at break at room temperature of 10% or more, and transparency corresponding to a light transmittance at 400 nm of 75% or more when formed into a film having a thickness of 10 µm.

13. The co-polyimide according to any one of Claims 8 to 12, having an average coefficient of linear thermal expansion of 20 ppm/K or less at 50 to 200°C when formed into a film having a thickness of 10 µm.

14. The co-polyimide according to any one of Claims 8 to 13, wherein, in the dynamic viscoelastic measurement of a film having a thickness of 10 µm formed from the co-polyimide, as compared with a minimum storage elastic modulus observed at a temperature not lower than the glass transition temperature determined from the maximum point of tan δ, the co-polyimide has a maximum storage elastic modulus at a temperature not lower than the temperature at which the minimum storage elastic modulus is observed.

15. A polyimide precursor, comprising a unit structure represented by general Formula (B1): 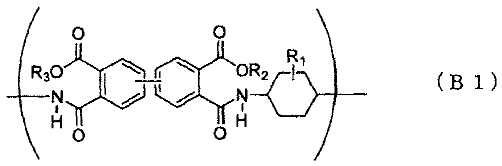 wherein, in general Formula (B1), R₁ represents a hydrogen atom or an alkyl group having 1 to 4 carbon atoms; R₂ and R₃ each represent a hydrogen atom or an alkylsilyl group having 3 to 9 carbon atoms, and at least one of R₂ and R₃ is an alkylsilyl group having 3 to 9 carbon atoms.

16. The polyimide precursor according to Claim 15, wherein general Formula (B1) is represented by general Formula (B2): 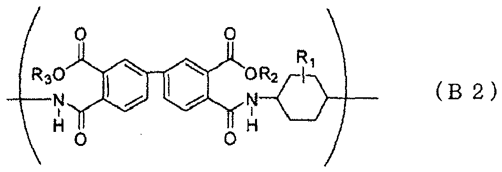 wherein, in general Formula (B2), R₁ represents a hydrogen atom or an alkyl group having 1 to 4 carbon atoms; R₂ and R₃ each represent a hydrogen atom or an alkylsilyl group having 3 to 9 carbon atoms, and at least one of R₂ and R₃ is an alkylsilyl group having 3 to 9 carbon atoms.

17. A polyimide prepared by imidization of the polyimide precursor according to Claim 15 or 16.

18. A 2,3,3',4'-biphenyltetracarboxylic dianhydride powder, having a light transmittance of 85% or more at a wavelength of 400 nm and an optical path length of 1 cm as a 10% by mass solution in a 2 N aqueous sodium hydroxide solution.

19. A method of purifying a 3,3',4,4'-biphenyltetracarboxylic dianhydride powder, comprising mixing a solvent in which the solubility of 3,3',4,4'-biphenyltetracarboxylic dianhydride at 25°C is 0.1 g/100 g or more and a 3,3',4,4'-biphenyltetracarboxylic dianhydride powder in an uneven state where at least a part of the 3,3',4,4'-biphenyltetracarboxylic dianhydride powder is not dissolved; and separating and collecting the undissolved 3,3',4,4'-biphenyltetracarboxylic dianhydride powder from the mixture.

20. A trans-1,4-diaminocyclohexane powder having a light transmittance of 90% or more at a wavelength of 400 nm and an optical path length of 1 cm as a 10% by mass solution in pure water.

21. A 2,2',3,3'-biphenyltetracarboxylic dianhydride powder having a light transmittance of 80% or more at a wavelength of 400 nm and an optical path length of 1 cm as a 10% by mass solution in a 2 N aqueous sodium hydroxide solution as a solvent.

22. A polyimide prepared by a reaction between a diamine component and a tetracarboxylic acid component, wherein
the diamine component comprises an aromatic ring-free diamine (including a derivative thereof, the same applies to the following) having a light transmittance of 90% or more or an aromatic ring-containing diamine (including a derivative thereof, the same applies to the following) having a light transmittance of 80% or more (here, the transmittance of the diamine component is that measured at a wavelength of 400 nm and an optical path length of 1 cm as a 10% by mass solution in pure water or N, N-dimethylacetamide); and
the tetracarboxylic acid component comprises a tetracarboxylic acid (including a derivative thereof, the same applies to the following) having a light transmittance of 75% or more (here, the transmittance of the tetracarboxylic acid component is that measured at a wavelength of 400 nm and an optical path length of 1 cm as a 10% by mass solution in a 2 N aqueous sodium hydroxide solution).

23. A polyimide precursor, comprising an aromatic ring-free diamine in an amount of 50% by mol or more of the total moles of the diamine component used; the polyimide precursor having a light transmittance of 90% or more at a wavelength of 400 nm and an optical path length of 1 cm as a 10% by mass solution in a polar solvent.

24. A polyimide precursor, comprising an aromatic ring-containing diamine in an amount of 50% by mol or more of the total moles of the diamine component used; the polyimide precursor having a light transmittance of 50% or more at a wavelength of 400 nm and an optical path length of 1 cm as a 10% by mass solution in a polar solvent.

25. A method of producing a varnish, comprising at least an organic solvent and a polyimide precursor represented by general Formula (H1) or a polyimide represented by general Formula (H2): 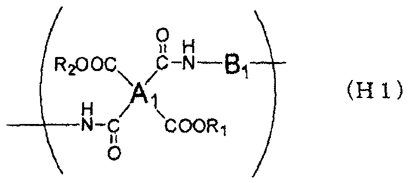 (in general Formula (H1), A₁ represents a tetravalent aliphatic or aromatic group; B₁ represents a divalent aliphatic or aromatic group; and R₁ and R₂ each independently represent a hydrogen atom, an alkyl group having 1 to 6 carbon atoms, or an alkylsilyl group having 3 to 9 carbon atoms), 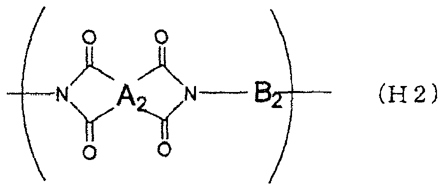 (in general Formula (H2), A₂ represents a tetravalent aliphatic or aromatic group; and B₂ represents a divalent aliphatic or aromatic group), wherein
the organic solvent to be contained in the varnish (hereinafter, referred to as the organic solvent used) has a light transmittance of 89% or more at 400 nm and an optical path length of 1 cm.
